# EUROPEAN PATENT SPECIFICATION

(11) **EP 3 347 358 B1**
(45) Date of publication and mention of the grant of the patent: **27.11.2019**
(21) Application number: 16760492.5
(22) Date of filing: 05.09.2016
(51) Int. Cl.: C07D 491/048, A61K 31/4355, A61P 3/10

(54) **[{[2,3-DIHYDRO-1H-INDEN-1-YL]AMINO}-2H,3H-FURO[3,2-B]PYRIDIN-3-YL]ACETIC ACIDS, PHARMACEUTICAL COMPOSITIONS AND USES THEREOF**
[{[2,3-DIHYDRO-1H-INDEN-1-YL]AMINO}-2H,3H-FURO[3,2-B]PYRIDIN-3-YL]ESSIGSÄUREN, PHARMAZEUTISCHE ZUSAMMENSETZUNGEN UND VERWENDUNGEN DAVON
ACIDES [{[2,3-DIHYDRO-1H-INDEN-1-YL]AMINO}-2H,3H-FURO[3,2-B]PYRIDIN-3-YL]ACÉTIQUES, COMPOSITIONS PHARMACEUTIQUES ET UTILISATIONS ASSOCIÉES

(30) Priority: 09.09.2015 EP 15184449; 08.02.2016 EP 16154638
(43) Date of publication of application: 18.07.2018
(73) Proprietor: Boehringer Ingelheim International GmbH, 55216 Ingelheim am Rhein (DE)
(72) Inventor: ECKHARDT, Matthias, 55216 Ingelheim Am Rhein (DE); WAGNER, Holger, 55216 Ingelheim Am Rhein (DE); PETERS, Stefan, 55216 Ingelheim Am Rhein (DE)
(74) Representative: Simon, Elke Anna Maria
(86) International application number: PCT/EP2016/070839
(87) International publication number: WO 2017/042121

(56) References cited:
- WO-A1-2010/143733
- WO-A1-2013/144098
- WO-A1-2014/019186
- WO-A1-2015/024526

## Description

### Field of the invention

The present invention relates to novel [{[2,3-Dihydro-1H-inden-1-yl]amino}-2H,3H-furo[3,2-b]pyridine-3-yl]acetic acids, that are agonists of the G-protein coupled receptor 40 (GPR40, also known as free fatty acid receptor FFAR 1), to processes for their preparation, to pharmaceutical compositions containing these compounds and to their medical use for the prophylaxis and/or treatment of diseases which can be influenced by the modulation of the function of GPR40. Particularly, the pharmaceutical compositions of the invention are suitable for the prophylaxis and/or therapy of metabolic diseases, such as diabetes, more specifically type 2 diabetes mellitus, and conditions associated with the disease, including insulin resistance, obesity, cardiovascular disease and dyslipidemia.

### Background of the Invention

Metabolic diseases are diseases caused by an abnormal metabolic process and may either be congenital due to an inherited enzyme abnormality or acquired due to a disease of an endocrine organ or failure of a metabolically important organ such as the liver or the pancreas.

Diabetes mellitus is a disease state or process derived from multiple causative factors and is defined as a chronic hyperglycemia associated with resulting damages to organs and dysfunctions of metabolic processes. Depending on its etiology, one differentiates between several forms of diabetes, which are either due to an absolute (lacking or decreased insulin secretion) or to a relative lack of insulin. Diabetes mellitus Type I (IDDM, insulin-dependent diabetes mellitus) generally occurs in adolescents under 20 years of age. It is assumed to be of auto-immune etiology, leading to an insulitis with the subsequent destruction of the beta cells of the islets of Langerhans which are responsible for the insulin synthesis. In addition, in latent autoimmune diabetes in adults (LADA; Diabetes Care. 8: 1460-1467, 2001) beta cells are being destroyed due to autoimmune attack. The amount of insulin produced by the remaining pancreatic islet cells is too low, resulting in elevated blood glucose levels (hyperglycemia). Diabetes mellitus Type II generally occurs at an older age. It is above all associated with a resistance to insulin in the liver and the skeletal muscles, but also with a defect of the islets of Langerhans. High blood glucose levels (and also high blood lipid levels) in turn lead to an impairment of beta cell function and to an increase in beta cell apoptosis.

Persistent or inadequately controlled hyperglycemia is associated with a wide range of pathologies. Diabetes is a very disabling disease, because today's common antidiabetic drugs do not control blood sugar levels well enough to completely prevent the occurrence of high and low blood sugar levels. Out of range blood sugar levels are toxic and cause long-term complications for example retinopathy, renopathy, neuropathy and peripheral vascular disease. There is also a host of related conditions, such as obesity, hypertension, stroke, heart disease and hyperlipidemia, for which persons with diabetes are substantially at risk.

Obesity is associated with an increased risk of follow-up diseases such as cardiovascular diseases, hypertension, diabetes, hyperlipidemia and an increased mortality. Diabetes (insulin resistance) and obesity are part of the "metabolic syndrome" which is defined as the linkage between several diseases (also referred to as syndrome X, insulin-resistance syndrome, or deadly quartet). These often occur in the same patients and are major risk factors for development of diabetes type II and cardiovascular disease. It has been suggested that the control of lipid levels and glucose levels is required to treat diabetes type II, heart disease, and other occurrences of metabolic syndrome (see e.g., Diabetes 48: 1836-1841, 1999; JAMA 288: 2209-2716, 2002).

The free fatty acid receptor GPR40 (also referred to as either FFAR, FFAR1, or FFA1) is a cell-surface receptor and a member of the gene superfamily of G-protein coupled receptors, which was first identified as a so-called orphan receptor, i.e. a receptor without a known ligand, based on the predicted presence of seven putative transmembrane regions in the corresponding protein (Sawzdargo et al. (1997) Biochem. Biophys. Res. Commun. 239: 543-547). GPR40 is found to be highly expressed in several particular cell types: the pancreatic β cells and insulin-secreting cell lines, as well as in enteroendocrine cells, taste cells, and is reported to be expressed in immune cells, splenocytes, and in the human and monkey brain. Meanwhile, fatty acids of varying chain lengths are thought to represent the endogenous ligands for GPR40, activation of which is linked primarily to the modulation of the Gq family of intra-cellular signaling G proteins and concomitant induction of elevated calcium levels, although activation of Gs- and Gi-proteins to modulate intracellular levels of cAMP have also been reported. GPR40 is activated especially by long-chain FFA, particularly oleate, as well as the PPAR-gamma agonist rosiglitazone.

It has been recognized that the fatty acids that serve as activators for GPR40 augment the elevated plasma glucose-induced secretion of insulin through GPR40 receptors that are expressed in the insulin secreting cells (Itoh et al. (2003) Nature 422: 173-176; Briscoe et al. (2003) J. Biol. Chem. 278: 11303-11311; Kotarsky et al. (2003) Biochem. Biophys. Res. Commun. 301: 406-410). Despite initial controversy, the use of GPR40 agonist appears to be the appropriate for increasing insulin release for the treatment of diabetes (see e.g. Diabetes 2008, 57, 2211; J. Med. Chem. 2007, 50, 2807). Typically, long term diabetes therapy leads to the gradual diminution of islet activity, so that after extended periods of treatment Type 2 diabetic patients need treatment with daily insulin injections instead. GPR40 agonists may have the potential to restore or preserve islet function, therefore, GPR40 agonists may be beneficial also in that that they may delay or prevent the diminution and loss of islet function in a Type 2 diabetic patient.

It is well established that the incretins GLP-1 (glucagon-like peptide- 1) and GIP (glucose-dependent insulinotropic peptide; also known as gastric inhibitory peptide) stimulate insulin secretion and are rapidly inactivated in vivo by DPP-4. These peptidyl hormones are secreted by endocrine cells that are located in the epithelium of the small intestine. When these endocrine cells sense an increase in the concentration of glucose in the lumen of the digestive tract, they act as the trigger for incretin release. Incretins are carried through the circulation to beta cells in the pancreas and cause the beta cells to secrete more insulin in anticipation of an increase of blood glucose resulting from the digesting meal. Further studies indicating that the GPR40 modulatory role on the release of incretins from the enteroendocrine cells, including CCK, GLP-1, GIP, PYY, and possibly others, suggest that GPR40 modulators may contribute to enhanced insulin release from the pancreatic beta cells also indirectly by e.g. a synergistic effect of GLP-1 and possibly GIP on the insulin release, and the other release incretins may also contribute to an overall beneficial contribution of GPR40 modulation on metabolic diseases. The indirect contributions of GPR40 modulation on insulin release through the elevation of plasma levels of incretins may be further augmented by the coadministration of inhibitors of the enzymes responsible for the incretin degradation, such as inhibitors of DPP-4.

Insulin imbalances lead to conditions such as type II diabetes mellitus, a serious metabolic disease. The modulation of the function of GPR40 in modulating insulin secretion indicates the therapeutic agents capable of modulating GPR40 function could be useful for the treatment of disorders such as diabetes and conditions associated with the disease, including insulin resistance, obesity, cardiovascular disease and dyslipidemia.

### Object of the present invention

The object of the present invention is to provide new compounds, hereinafter described as compounds of formula (I), in particular new [{[2,3-Dihydro-1H-inden-1-yl]amino}-2H,3H-furo[3,2-b]pyridine-3-yl]acetic acids, which are active with regard to the G-protein-coupled receptor GPR40, notably are agonists of the G-protein-coupled receptor GPR40.

A further object of the present invention is to provide new compounds, in particular new [{[2,3-Dihydro-1H-inden-1-yl]amino}-2H,3H-furo[3,2-b]pyridine-3-yl]acetic acids, which have an activating effect on the G-protein-coupled receptor GPR40 *in vitro* and/or *in vivo* and possess suitable pharmacological and pharmacokinetic properties to use them as medicaments.

A further object of the present invention is to provide effective GPR40 agonists, in particular for use in the treatment of metabolic disorders, for example diabetes, dyslipidemia and/or obesity.

A further object of the present invention is to provide a pharmaceutical composition comprising at least one compound according to the invention.

A further object of the present invention is to provide a combination of at least one compound according to the invention with one or more additional therapeutic agents. Further objects of the present invention become apparent to the one skilled in the art by the description hereinbefore and in the following and by the examples.

GPR40 modulators are known in the art, for example, the compounds disclosed in WO 2004041266 (EP 1559422), WO 2007033002, WO 2009157418 and WO 2014019186, WO2010143733, WO2013144098, WO2015024526. The [{[2,3-Dihydro-1H-inden-1-yl]amino}-2H,3H-furo[3,2-b]pyridine-3-yl]acetic acids of the present invention may provide several advantages, such as enhanced potency, lower plasma-protein-binding, high metabolic and/or chemical stability, high selectivity and tolerability, enhanced solubility, and the possibility to form stable salts.

### Summary of the Invention

In a first aspect the invention relates to a compound of formula I 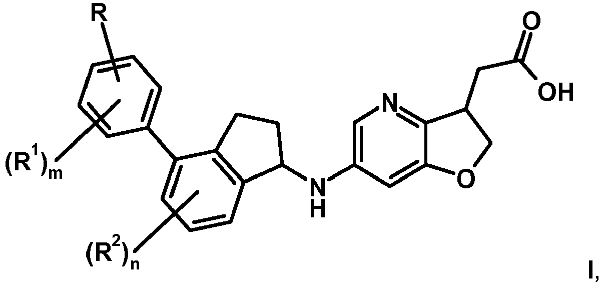 wherein
- R: is selected from the group R-G1 consisting of
H, F, Cl, Br, I, C₁₋₆-alkyl, C₂₋₆-alkenyl, C₂₋₆-alkynyl, C₃₋₆-cycloalkyl, NC-, HNR^{N}-C(=O)-, C₁₋₄-alkyl-NR^{N}-C(=O)-, C₃₋₆-cycloalkyl-NR^{N}-C(=O)-, heterocyclyl-NR^{N}-C(=O)-, heteroaryl-NR^{N}-C(=O)-, HOOC-, C₁₋₄-alkyl-O-C(=O)-, O₂N-, HR^{N}N-, C₁₋₄-alkyl-R^{N}N-, C₁₋₄-alkyl-C(=O)NR^{N}-, C₃₋₆-cycloalkyl-C(=O)NR^{N}-, heterocyclyl-C(=O)NR^{N}-, heteroaryl-C(=O)NR^{N}-, C₁₋₄-alkyl-S(=O)₂NR^{N}-, C₃₋₆-cycloalkyl-S(=O)₂NR^{N}-, heterocyclyl-S(=O)₂NR^{N}-, heteroaryl-S(=O)₂NR^{N}-, HO-, C₁₋₆-alkyl-O-, HOOC-C₁₋₃-alkyl-O-, heterocyclyl-C₁₋₃-alkyl-O-, phenyl-C₁₋₃-alkyl-O-, C₃₋₆-cycloalkyl-O-, heterocyclyl-O-, heteroaryl-O-, C₁₋₄-alkyl-S-, C₃₋₆-cycloalkyl-S-, heterocyclyl-S-, C₁₋₄-alkyl-S(=O)-, C₃₋₆-cycloalkyl-S(=O)-, heterocyclyl-S(=O)-, C₁₋₄-alkyl-S(=O)₂-, C₃₋₆-cycloalkyl-S(=O)₂-, heterocyclyl-S(=O)₂-, phenyl-S(=O)₂-, heteroaryl-S(=O)₂-, HNR^{N}-S(=O)₂-, C₁₋₄-alkyl-NR^{N}-S(=O)₂-, heterocyclyl, phenyl, and heteroaryl,
wherein each alkyl, cycloalkyl, and heterocyclyl group or sub-group within the groups forming R is optionally substituted with 1 or more F atoms and optionally substituted with 1 to 3 groups independently selected from Cl, C₁₋₃-alkyl, NC-, (R^{N})₂N-, HO-, C₁₋₃-alkyl-O-, and C₁₋₃-alkyl-S(=O)₂-; and
wherein each phenyl and heteroaryl group or sub-group within the groups forming R is optionally substituted with 1 to 5 substituents independently selected from F, Cl, C₁₋₃-alkyl, HF₂C-, F₃C-, NC-, (R^{N})₂N-, HO-, C₁₋₃-alkyl-O-, F₃C-O-, and C₁₋₃-alkyl-S(=O)₂-;
wherein each heterocyclyl group or sub-group within the groups forming R is selected from
a cyclobutyl group wherein 1 CH₂ group is replaced by -NR^{N}- or -O-,
a C₅₋₆-cycloalkyl group wherein 1 CH₂ group is replaced by -C(=O)-, -NR^{N}-,-O- or -S(=O)₂- and/or 1 CH group by N;
a C₅₋₆-cycloalkyl group wherein 1 CH₂ group is replaced by -NR^{N}- or -O-, a second CH₂ group is replaced by -NR^{N}-, -C(=O)- or -S(=O)₂- and/or 1 CH group is replaced by N; and
a C₅₋₆-cycloalkyl group wherein 2 CH₂ groups are replaced by -NR^{N}- or 1 CH₂ group by -NR^{N}- and the other by -O- and a third CH₂ group is replaced by-C(=O)- or -S(=O)₂- and/or 1 CH group by N;
wherein each heteroaryl group or sub-group within the groups forming R is selected from
tetrazolyl and a 5- or 6-membered heteroaromatic ring which contains 1, 2, or 3 heteroatoms independently of each other selected from =N-, -NR^{N}-, -O-, and -S-, wherein in heteroaromatic groups containing a -HC=N- unit this group is optionally replaced by -NR^{N}-C(=O)-;
wherein in heteroaryl and heterocyclyl rings with one or more NH groups, each of them is replaced by NR^{N};
- R¹: is selected from the group R¹-G1 consisting of H, F, Cl, C₁₋₄-alkyl, C₃₋₆-cycloalkyl-, HO-C₁₋₄-alkyl, C₁₋₄-alkyl-O-C₁₋₄-alkyl, NC-, HO-, C₁₋₄-alkyl-O-, C₃₋₆-cycloalkyl-O-, C₁₋₄-alkyl-S-, C₁₋₄-alkyl-S(O)-, and C₁₋₄-alkyl-S(O)₂-,
wherein any alkyl and cycloalkyl group or sub-group within the groups forming R¹ is optionally substituted with 1 or more F atoms, and wherein multiple R¹ may be identical or different if m is 2, 3 or 4;
- m: is an integer selected from 1, 2, 3, and 4;
- R²: is selected from the group R²-G1 consisting of H, F, Cl, C₁₋₄-alkyl, NC-, and C₁₋₄-alkyloxy,
wherein any alkyl group or sub-group within the groups forming R² is optionally substituted with 1 or more F atoms, and wherein multiple R² may be identical or different if n is 2 or 3;
- n: is an integer selected from 1, 2, and 3;
- R^{N}: is independently of each other selected from the group R^{N}-G1 consisting of H, C₁₋₄-alkyl, HO-C₁₋₄-alkyl-(H₂C)-, C₁₋₃-alkyl-O-C₁₋₄-alkyl-, C₁₋₄-alkyl-C(=O)-, C₁₋₄-alkyl-NH-C(=O)-, C₁₋₄-alkyl-N(C₁₋₄-alkyl)-C(=O)-, C₁₋₄-alkyl-O-C(=O)-, and C₁₋₄-alkyl-S(=O)₂-,
wherein each alkyl group or sub-group within the groups forming R^{N} is optionally substituted with 1 or more F atoms;
wherein in any definition mentioned hereinbefore and if not specified otherwise, any alkyl group or sub-group may be straight-chained or branched, the isoforms, tautomers, stereoisomers, metabolites, prodrugs, solvates, hydrates, and the salts thereof, particularly the physiologically acceptable salts thereof with inorganic or organic acids or bases, or the combinations thereof.

The extension -Gn used within the definitions is meant to identify genus n of the respective substituent. For example, R¹-G1 defines genus 1 of the substituent R¹. The expression "optionally substituted with 1 or more F atoms" means that none or one up to successively all H atoms bound to carbon atoms of the respective group or submoiety may be replaced by F atoms, preferably 1 to 5 H atoms or, more preferred, 1 to 3 H atoms may be replaced by F atoms.

In a further aspect this invention relates to a pharmaceutical composition, comprising one or more compounds of general formula **I** or one or more pharmaceutically acceptable salts thereof according to the invention, optionally together with one or more inert carriers and/or diluents.

According to another aspect of the invention, there is provided the use of a compound of the general formula I or a pharmaceutically acceptable salt thereof for the manufacture of a medicament for a therapeutic method as described hereinbefore and hereinafter.

According to another aspect of the invention, there is provided a compound of the general formula **I** or a pharmaceutically acceptable salt thereof for use in a therapeutic method as described hereinbefore and hereinafter.

In a further aspect this invention relates to a method for treating a disease or condition mediated by the activation of the G-protein-coupled receptor GPR40 in a patient that includes the step of administering to the patient in need of such treatment a therapeutically effective amount of a compound of the general formula **I** or a pharmaceutically acceptable salt thereof in combination with a therapeutically effective amount of one or more additional therapeutic agents.

In a further aspect this invention relates to the use of a compound of the general formula **I** or a pharmaceutically acceptable salt thereof in combination with one or more additional therapeutic agents for the treatment of diseases or conditions which are mediated by the activation of the G-protein-coupled receptor GPR40.

In a further aspect this invention relates to a pharmaceutical composition which comprises a compound according to general formula **I** or a pharmaceutically acceptable salt thereof and one or more additional therapeutic agents, optionally together with one or more inert carriers and/or diluents.

Other aspects of the invention become apparent to the one skilled in the art from the specification and the experimental part as described hereinbefore and hereinafter.

### Detailed Description

Unless otherwise stated, the groups, residues, and substituents, particularly R, R¹, R², m, and n are defined as above and hereinafter. If residues, substituents, or groups occur several times in a compound, they may have the same or different meanings. Some preferred meanings of individual groups and substituents of the compounds according to the invention will be given hereinafter. Any and each of these definitions may be combined with each other.

### R:

### R-G1:

The group R is preferably selected from the group R-G1 as defined hereinbefore.

### R-G2:

In another embodiment the group R is selected from the group R-G2 consisting of H, F, Cl, C₁₋₆-alkyl, C₃₋₆-cycloalkyl, NC-, HNR^{N}-C(=O)-, C₁₋₄-alkyl-NR^{N}-C(=O)-, C₃₋₆-cycloalkyl-NR^{N}-C(=O)-, heterocyclyl-NR^{N}-C(=O)-, HOOC-, HR^{N}N-, C₁₋₄-alkyl-R^{N}N-, C₁₋₄-alkyl-C(=O)NR^{N}-, C₃₋₆-cycloalkyl-C(=O)NR^{N}-, heterocyclyl-C(=O)NR^{N}-, C₁₋₄-alkyl-S(=O)₂NR^{N}-, HO-, C₁₋₆-alkyl-O-, HOOC-C₁₋₂-alkyl-O-, heterocyclyl-C₁₋₂-alkyl-O-, phenyl-C₁₋₂-alkyl-O-, C₃₋₆-cycloalkyl-O-, heterocyclyl-O-, heteroaryl-O-, C₁₋₄-alkyl-S(=O)₂-, C₃₋₆-cycloalkyl-S(=O)₂-, heterocyclyl-S(=O)₂-, HNR^{N}-S(=O)₂-, C₁₋₄-alkyl-NR^{N}-S(=O)₂-, heterocyclyl, and heteroaryl,
wherein each alkyl, cycloalkyl, and heterocyclyl group or sub-group within the groups forming R is optionally substituted with 1 or more F atoms and optionally substituted with 1 to 2 groups independently selected from Cl, H₃C-, NC-, R^{N}HN-, HO-, H₃C-O-, and H₃C-S(=O)₂-;
wherein each heteroaryl group or sub-group within the groups forming R is optionally substituted with 1 to 3 substituents independently selected from F, Cl, H₃C-, F₃C-, NC-, (R^{N})₂N-, HO-, H₃C-O-, F₃C-O-, and H₃C-S(=O)₂-;
wherein each heterocyclyl group or sub-group within the groups forming R is selected from
   a cyclobutyl group wherein 1 CH₂ group is replaced by -NR^{N}- or -O-,
   a C₅₋₆-cycloalkyl group wherein 1 CH₂ group is replaced by -C(=O)-, -NR^{N}-,-O- or -S(=O)₂- and/or 1 CH group by N;
   a C₅₋₆-cycloalkyl group wherein 1 CH₂ group is replaced by -NR^{N}- or -O-, a second CH₂ group is replaced by -NR^{N}-, -C(=O)- or -S(=O)₂- and/or 1 CH group is replaced by N;
wherein each heteroaryl group or sub-group within the groups forming R is selected from
   tetrazolyl, a 5-membered heteroaromatic ring which contains 1, 2 or 3 heteroatoms independently of each other selected from =N-, -NH-, O and S, and a 6-membered heteroaromatic ring which contains 1 or 2 =N- atoms, wherein a -HC=N- unit is optionally replaced by -NH-C(=O)-;
and wherein in each of the above heteroaryl and heterocyclyl group or sub-group containing one or more NH, said group(s) is replaced by NR^{N}.

### R-G3:

In another embodiment the group R is selected from the group R-G3 consisting of F, Cl, C₁₋₄-alkyl, NC-, H₂N-C(=O)-, C₁₋₃-alkyl-NR^{N}-C(=O)-, HOOC-, H₂N-, C₁₋₃-alkyl-C(=O)NR^{N}-, C₁₋₄-alkyl-S(=O)₂NR^{N}-, HO-, C₁₋₅-alkyl-O-, HOOC-CH₂-O-, heterocyclyl-CH₂-O-, phenyl-CH₂-O-, C₃₋₆-cycloalkyl-O-, heterocyclyl-O-, heteroaryl-O-, heterocyclyl-S(=O)₂-, heterocyclyl, and heteroaryl,
wherein each alkyl, cycloalkyl, and heterocyclyl group or sub-group within the groups forming R is optionally substituted with 1 or more F atoms and optionally substituted with 1 group selected from Cl, H₃C-, NC-, R^{N}HN-, HO-, H₃C-O-, and H₃C-S(=O)₂-;
wherein each heteroaryl group or sub-group within the groups forming R is optionally substituted with 1 to 2 substituents independently selected from F, Cl, H₃C-, F₃C-, NC-, (R^{N})₂N-, HO-, H₃C-O-, F₃C-O-, and H₃C-S(=O)₂-;
wherein each heterocyclyl or sub-group within the groups forming R is selected from a cyclobutyl group wherein 1 CH₂ group is replaced by -NR^{N}- or -O-, a C₅₋₆-cycloalkyl group wherein 1 CH₂ group is replaced by -C(=O)-, -NR^{N}-, -O- or -S(=O)₂- and/or 1 CH group by N;
wherein each heteroaryl group or sub-group within the groups forming R is selected from tetrazolyl, a 5-membered heteroaromatic ring which contains 1, 2 or 3 heteroatoms independently of each other selected from =N-, -NH-, O and S, and a 6-membered heteroaromatic ring which contains 1 or 2 =N- atoms, wherein a -HC=N- unit is optionally replaced by -NH-C(=O)-;
and wherein in each heteroaryl and heterocyclyl group or sub-group mentioned under R-G3 containing one or more NH, said group(s) is replaced by NR^{N}.

### R-G4:

According to another embodiment the group R is selected from the group R-G4 consisting of
F, Cl, NC-, H₂NC(=O)-, H₃CHN-C(=O)-, (H₃C)₂N-C(=O)-, HOOC-, H₂N-, HO-; C₁₋₃-alkyl optionally substituted with 1 or more F or optionally monosubstituted with HO-;
cyclopropyl optionally monosubstituted with NC-;
H₃C-O- optionally monosubstituted with C₁₋₄-alkyl, HOOC-, oxetanyl, tetrahydrofuranyl, tetrahydropyranyl, 1,1-dioxotetrahydrothiopyranyl, or phenyl,
wherein the C₁₋₄-alkyl group optionally attached to H₃C-O- is optionally monosubstituted with NC-, HO- or H₃C-S(=O)₂-, and
wherein said oxetanyl, tetrahydrofuranyl, tetrahydropyranyl and 1,1-dioxotetrahydrothiopyranyl groups are optionally monosubstituted with H₃C- or HO-;
cyclopropyl-O-, tetrahydrofuranyl-O- and tetrahydropyranyl-O-; and
a heteroaryl group selected from pyrazolyl, [1,2,4]oxadiazolyl, tetrazolyl, pyridyl, pyridin-2-onyl, pyrazinyl, pyrimidinyl, and pyrimidin-4-onyl,
wherein each of said heteroaryl groups is optionally monosubstituted with H₃C-, and
wherein each H-N group in said heteroaryl groups is optionally replaced with H₃C-N or (H₃C)₂C(OH)-H₂C-N.

### R-G5:

In another embodiment the group R is selected from the group R-G5 consisting of F, Cl, H₃C-, H₃C-H₂C-, (H₃C)₂CH-, 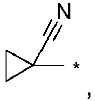 F₃C-, HOCH₂-, NC-, H₂N-C(=O)-, H₃C-NH-C(=O)-, (H₃C)₂N-C(=O)-, HOOC-, H₂N-, HO-, H₃C-O-, cyclopropyl-O-, 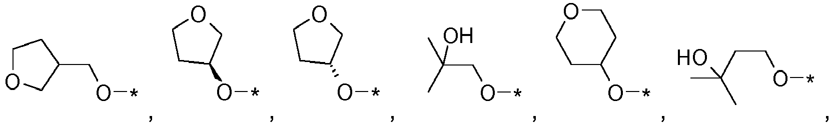 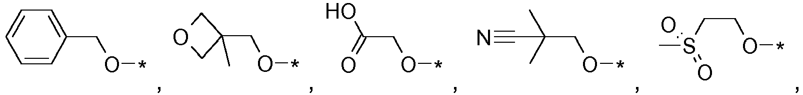 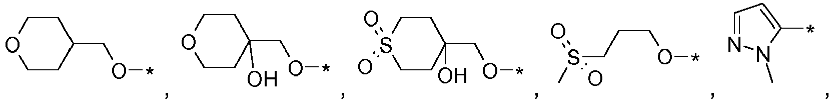 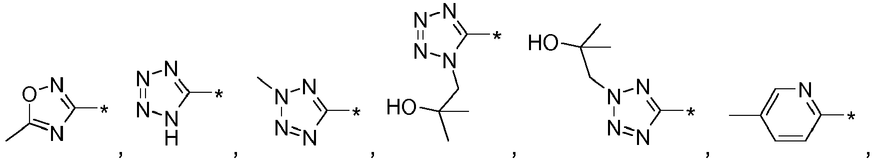 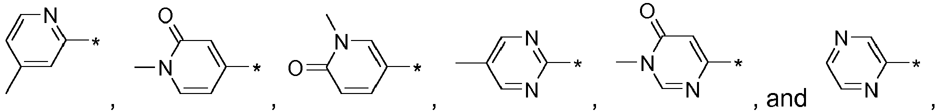 wherein the asterisk, -*, indicates the site of attachment.

### R-G6:

In another embodiment the group R is selected from the group R-G6 consisting of

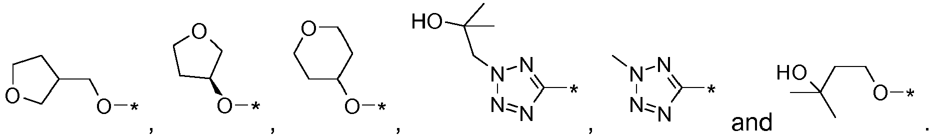

### R-G7:

In another embodiment the group R is selected from the group R-G7 consisting of

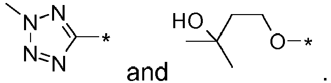

### R¹:

### R¹-G1:

The group R¹ is preferably selected from the group R¹-G1 as defined hereinbefore.

### R¹-G2:

According to one embodiment the group R¹ is selected from the group R¹-G2 consisting of H, F, Cl, C₁₋₃-alkyl, cyclopropyl, NC-, HO-, and C₁₋₃-alkyl-O-,
wherein each alkyl group or sub-group within the groups mentioned under R¹-G2 is optionally substituted with 1 or more F atoms.

### R¹-G3:

According to one embodiment the group R¹ is selected from the group R¹-G3 consisting of H, F, Cl, H₃C-, H₃C-H₂C-, F₃C-, NC-, and H₃CO-.

### R¹-G4:

According to one embodiment the group R¹ is selected from the group R¹-G4 consisting of H₃C-.

### R²:

### R²-G1:

The group R² is preferably selected from the group R²-G1 as defined hereinbefore.

### R²-G2:

In another embodiment the group R² is selected from the group R²-G2 consisting of H, F, Cl, H₃C-, F₃C-, NC-, and H₃CO-.

### R²-G3:

In another embodiment the group R² is selected from the group R²-G3 consisting of H and F.

### R²-G4:

In another embodiment the group R² is selected from the group R²-G4 consisting of H.

### R²-G5:

In another embodiment the group R² is selected from the group R²-G5 consisting of F.

### R^{N}:

### R^{N}-G1:

The group R^{N} is preferably selected from the group R^{N}-G1 as defined hereinbefore.

### R^{N}-G2:

In another embodiment the group R^{N} is selected from the group R^{N}-G2 consisting of H, C₁₋₃-alkyl, HO-C₁₋₄-alkyl-H₂C-, H₃C-O-C₁₋₄-alkyl-, C₁₋₃-alkyl-C(=O)-, and C₁₋₃-alkyl-S(=O)₂-.

### R^{N}-G3:

In another embodiment the group R^{N} is selected from the group R^{N}-G3 consisting of H, H₃C-, HO-C₃-alkyl-H₂C-, H₃C-C(=O)-, and H₃C-S(=O)₂-.

### m:

m is an integer selected from 1, 2, 3 and 4.

Preferably, m is an integer selected from 1 and 2.

More preferably, m is 2.

### n:

n is an integer selected from 1, 2 and 3.

Preferably, n is an integer selected from 1 and 2.

More preferably, n is 1.

The following preferred embodiments of compounds of the formula I are described using generic formulae I.1, I.2, and 1.3, wherein any tautomers, solvates, hydrates and salts thereof, in particular the pharmaceutically acceptable salts thereof, are encompassed.

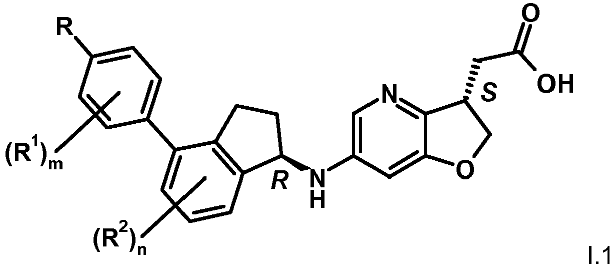

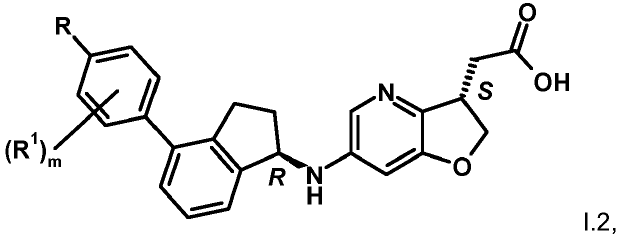

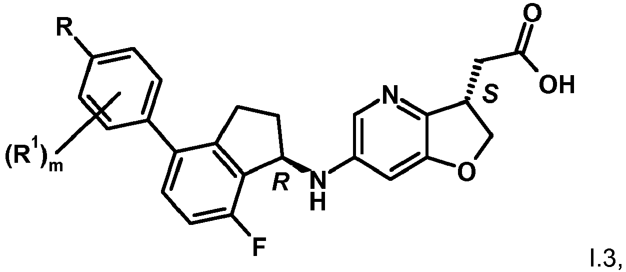

Examples of preferred subgeneric embodiments (E) according to the present invention are set forth in the following table 1, wherein each substituent group of each embodiment is defined according to the definitions set forth hereinbefore and wherein all other substituents of the formulae **I**, I.1, 1.2, and I.3 are defined according to the definitions set forth hereinbefore. For example, the entry -G1 in the column under R- and the line of E1 means that in embodiment E1 substituent R is selected from the definition designated R-G1. The same applies analogously to the other variables incorporated in the general formulae.

**Table 1:**

| **E** | **R-** | **R¹-** | **R²-** | **R^{N}-** | **m** | **n** |
|---|---|---|---|---|---|---|
| **E1** | **-G1** | **-G1** | **-G1** | **-G1** | **1,2,3,4** | **1,2,3** |
| **E2** | **-G1** | **-G1** | **-G1** | **-G2** | **1,2** | **1,2** |
| **E3** | **-G1** | **-G1** | **-G1** | **-G3** | **1,2** | **1,2** |
| **E4** | **-G1** | **-G1** | **-G2** | **-G3** | **1,2** | **1,2** |
| **E5** | **-G1** | **-G2** | **-G2** | **-G1** | **1,2** | **1,2** |
| **E6** | **-G1** | **-G2** | **-G2** | **-G2** | **1,2** | **1,2** |
| **E7** | **-G1** | **-G2** | **-G2** | **-G3** | **1,2** | **1,2** |
| **E8** | **-G2** | **-G1** | **-G1** | **-G1** | **1,2** | **1,2** |
| **E9** | **-G3** | **-G1** | **-G1** | **-G1** | **1,2** | **1,2** |
| **E10** | **-G3** | **-G1** | **-G2** | **-G2** | **1,2** | **1,2** |
| **E11** | **-G3** | **-G2** | **-G2** | **-G2** | **1,2** | **1,2** |
| **E12** | **-G2** | **-G2** | **-G2** | **-G3** | **1,2** | **1,2** |
| **E13** | **-G3** | **-G2** | **-G2** | **-G3** | **1,2** | **1,2** |
| **E14** | **-G1** | **-G3** | **-G2** | **-G3** | **1,2** | **1,2** |
| **E15** | **-G1** | **-G2** | **-G3** | **-G3** | **1,2** | **1,2** |
| **E16** | **-G1** | **-G3** | **-G3** | **-G3** | **1,2** | **1,2** |
| **E17** | **-G1** | **-G4** | **-G3** | **-G3** | **1,2** | **1,2** |
| **E18** | **-G2** | **-G3** | **-G2** | **-G3** | **1,2** | **1,2** |
| **E19** | **-G2** | **-G2** | **-G3** | **-G3** | **1,2** | **1,2** |
| **E20** | **-G2** | **-G3** | **-G3** | **-G3** | **1,2** | **1,2** |
| **E21** | **-G2** | **-G4** | **-G3** | **-G3** | **1,2** | **1,2** |
| **E22** | **-G3** | **-G3** | **-G2** | **-G3** | **1,2** | **1,2** |
| **E23** | **-G3** | **-G2** | **-G3** | **-G3** | **1,2** | **1,2** |
| **E24** | **-G3** | **-G3** | **-G3** | **-G3** | **1,2** | **1,2** |
| **E25** | **-G3** | **-G4** | **-G3** | **-G3** | **1,2** | **1,2** |
| **E26** | **-G4** | **-G3** | **-G2** | **-** | **2** | **1,2** |
| **E27** | **-G4** | **-G2** | **-G3** | **-** | **2** | **1** |
| **E28** | **-G4** | **-G3** | **-G3** | **-** | **2** | **1** |
| **E29** | **-G4** | **-G4** | **-G3** | **-** | **2** | **1** |
| **E30** | **-G5** | **-G3** | **-G2** | **-** | **2** | **1,2** |
| **E31** | **-G5** | **-G2** | **-G3** | **-** | **2** | **1** |
| **E32** | **-G5** | **-G3** | **-G3** | **-** | **2** | **1** |
| **E33** | **-G5** | **-G4** | **-G3** | **-** | **2** | **1** |

Another embodiment concerns those compounds of formula I, wherein
R is selected from the group R-G4 consisting of:
F, Cl, NC-, H₂NC(=O)-, H₃CHN-C(=O)-, (H₃C)₂N-C(=O)-, HOOC-, H₂N-, HO-;
C₁₋₃-alkyl optionally substituted with 1 or more F or optionally monosubstituted with HO-;
cyclopropyl optionally monosubstituted with NC-;
H₃C-O- optionally monosubstituted with C₁₋₄-alkyl, HOOC-, oxetanyl, tetrahydrofuranyl, tetrahydropyranyl, 1,1-dioxotetrahydrothiopyranyl, or phenyl,
   wherein the C₁₋₄-alkyl group optionally attached to H₃C-alkyl-O- is optionally monosubstituted with NC-, HO- or H₃C-S(=O)₂-, and
   wherein said oxetanyl, tetrahydrofuranyl, tetrahydropyranyl and 1,1-dioxotetrahydrothiopyranyl groups are optionally monosubstituted with H₃C- or HO-;
cyclopropyl-O-, tetrahydrofuranyl-O- and tetrahydropyranyl-O-;
a heteroaryl group selected from pyrazolyl, [1,2,4]oxadiazolyl, tetrazolyl, pyridyl, pyridin-2-onyl, pyrazinyl, pyrimidinyl, and pyrimidin-4-onyl,
   wherein each of said heteroaryl groups is optionally monosubstituted with H₃C-, and
   wherein each H-N group in said heteroaryl groups is optionally replaced with H₃C-N or (H₃C)₂C(OH)-H₂C-N;
R¹ is selected from the group R¹-G3 consisting of H, F, Cl, H₃C-, H₃C-H₂C-, F₃C-, NC-, and H₃C-O-;
m is 2;
R² is selected from the group R²-G3 consisting of H and F; and
n is 1.

Another embodiment concerns those compounds of formula I, wherein
R is selected from the group R-G5 consisting of
F, Cl, H₃C-, H₃C-H₂C-, (H₃C)₂CH-, 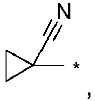 F₃C-, HOCH₂-, NC-, H₂N-C(=O)-, H₃C-NH-C(=O)-, (H₃C)₂N-C(=O)-, HOOC-, H₂N-, HO-, H₃C-O-, cyclopropyl-O-, 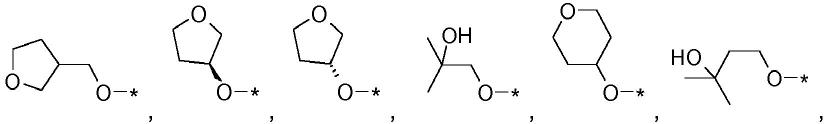 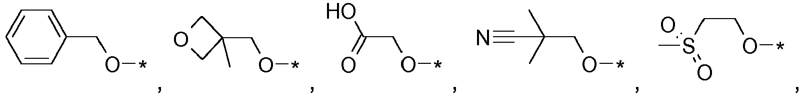 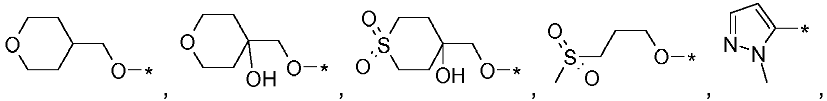 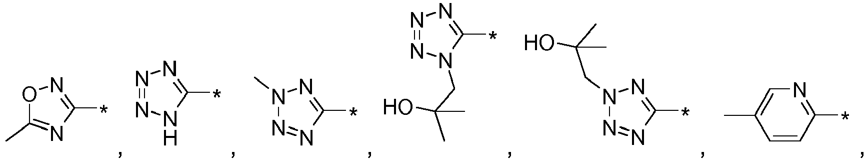 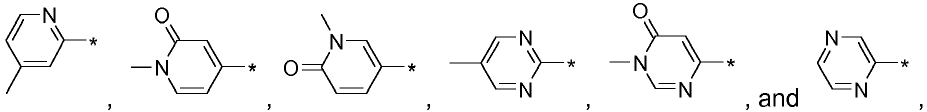 wherein the asterisk (-*) indicates the site/point of attachment;
R¹ is selected from the group R¹-G4 consisting of H₃C-;
m is 2;
R² is selected from the group R²-G5 consisting of F; and
n is 1.

Another embodiment concerns those compounds of formula I, wherein
R is: 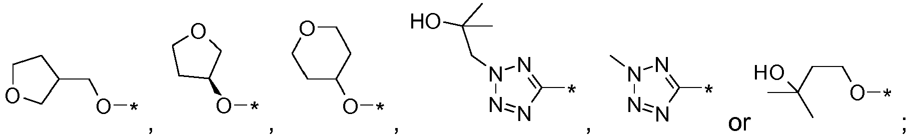 R¹ is H₃C-;
m is 2;
R² is H or F; and
n is 1.

Still another embodiment concerns those compounds of formula I, wherein
R is: 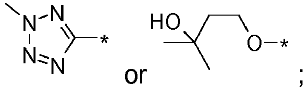 R¹ is H₃C-;
m is 2;
R² is H or F; and
n is 1.

Particularly preferred compounds, including their tautomers and stereoisomers, the salts thereof, or any solvates or hydrates thereof, are described in the experimental section hereinafter.

The compounds according to the invention and their intermediates may be obtained using methods of synthesis which are known to the one skilled in the art and described in the literature of organic synthesis for example using methods described in "Comprehensive Organic Transformations, 2nd edition", Richard C. Larock, Wiley-VCH, 2009., and "March's Advanced Organic Chemistry, 7th edition", Michael B. Smith, John Wiley & Sons, 2013. Preferably the compounds are obtained analogously to the methods of preparation explained more fully hereinafter, in particular as described in the experimental section. In some cases the sequence adopted in carrying out the reaction schemes may be varied. Variants of these reactions that are known to the skilled man but are not described in detail here may also be used. The general processes for preparing the compounds according to the invention will become apparent to the skilled man on studying the schemes that follow. Starting compounds are commercially available or may be prepared by methods that are described in the literature or herein, or may be prepared in an analogous or similar manner. Before the reaction is carried out any corresponding functional groups in the compounds may be protected using conventional protecting groups. These protecting groups may be cleaved again at a suitable stage within the reaction sequence using methods familiar to the skilled man and described in the literature for example in "Protecting Groups, 3rd Edition", Philip J. Kocienski, Theime, 2005 or "Greene's Protective Groups in Organic Synthesis, 4th Edition", Peter G. M. Wuts, Theadora W. Greene, John Wiley and Sons, 2007.

The compounds of the invention **I** are preferably accessed from a precursor **II** that bears the carboxylic acid function in a protected or masked form as sketched in Scheme 1; R, R¹, R², m and n have the meanings as defined hereinbefore and hereinafter. Suited precursor groups for the carboxylic acid may be, e.g., a carboxylic ester, a carboxylic amide, cyano, an olefin, oxazole, or a thiazole. All these groups have been transformed into the carboxylic acid function by different means which are described in the organic chemistry literature and are known to the one skilled in the art. The preferred precursor group is a C₁₋₄-alkyl or benzyl carboxylate, each of which may be additionally mono- or polysubstituted with fluorine, methyl, and/or methoxy. These ester groups may be hydrolyzed with an acid, such as hydrochloric acid or sulfuric acid, or an alkali metal hydroxide, such as lithium hydroxide, sodium hydroxide, or potassium hydroxide, to yield the carboxylic acid function; the hydrolysis is preferably conducted in aqueous solvents, such as water and tetrahydrofuran, 1,4-dioxane, alcohol, e.g., methanol, ethanol, and isopropanol, or dimethyl sulfoxide, at 0 to 120 °C. A *tert*-butyl ester is preferably cleaved under acidic conditions, e.g., trifluoroacetic acid or hydrochloric acid, in a solvent such as dichloromethane, 1,4-dioxane, isopropanol, or ethyl acetate. A benzyl ester is advantageously cleaved using hydrogen in the presence of a transition metal, preferably palladium on carbon. Benzyl esters bearing electron donating groups, such as methoxy, on the aromatic ring may also be removed under oxidative conditions; ceric ammonium nitrate (CAN) or 2,3-dichloro-5,6-dicyanoquinone (DDQ) are two commonly used reagents for this approach.

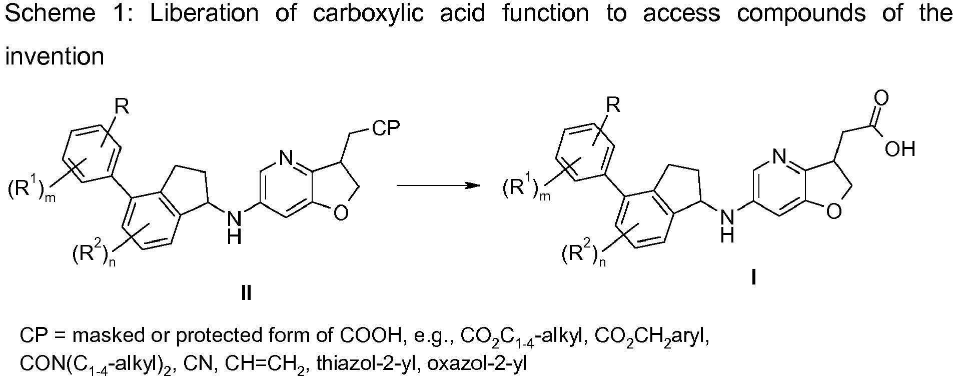

Compound **II**, in turn, may be obtained from aminoindane **III** and aza-dihydrobenzofurane**IV** which is decorated with the carboxylic acid precursor group and a leaving group (Scheme 2); R, R¹, R², m and m in Scheme 2 have the meanings as defined hereinbefore and hereinafter. The leaving group LG in **IV** is replaced with the NH group in **III** via a nucleophilic substitution reaction on the pyridine ring; suited LG may be F, Cl, Br, and I. The reaction is usually carried out in the presence of a base, such as triethylamine, ethyldiisopropylamine, 1,8-diazabicyclo[5.4.0]undecene, carbonates, e.g., Li₂CO₃, Na₂CO₃, K₂CO₃, and Cs₂CO₃, hydroxides, e.g., LiOH, NaOH, and KOH, alcoholates, e.g., NaOMe, NaOEt, and KO*t*Bu, and oxides, e.g., CaO and Ag₂O. Additives, such as silver salts, e.g., AgNO₃, AgOSO₂CF₃, and Ag₂CO, may be beneficial for the reaction to proceed. Preferred solvents are dimethylsulfoxide, N,N-dimethylformamide, N,N-dimethylacetamide, N-methylpyrrolidinone, acetonitrile, 1,4-dioxane, tetrahydrofuran, alcohol, e.g., ethanol or isopropanol, water, or mixtures thereof at temperatures of 20 to 220 °C. Preferably, coupling of aminoindane **III** and pyridine **IV** is mediated by a transition metal catalyst. Suited aza-dihydrobenzofurane**IV** for this proceeding bear Cl, Br, or I as LG and the catalyst may be derived from Cu, Ni, and Pd, preferably Pd. The catalyst or the precursor thereof may be a complex of the transition metal with ligands such as phosphines, e.g. tri-*tert*-butylphosphine, tricyclohexyl-phosphine, 2-optionally substituted biphenyl-dicyclohexyl-phosphines, 2-optionally substituted biphenyl-di-*tert*-butylphosphines, 1,1'-bis(diphenylphosphino)ferrocene, triphenylphosphine, tritolylphosphine, or trifurylphosphine, phosphites, 1,3-disubstituted imidazole carbenes, 1,3-disubstituted imidazolidine carbenes, dibenzylideneacetone, allyl, or nitriles, an elemental form of the transition metal such as palladium on carbon or nanoparticles of palladium, or a salt of the transition metal such as fluoride, chloride, bromide, acetate, triflate, or trifluoroacetate that may be combined with a separately added ligand. The reaction is commonly conducted in the presence of a base such as an alcoholate, e.g., LiO*t*Bu, NaO*t*Bu, and KO*t*Bu, lithium hexamethyldisilazide, K₃PO₄, carbonate such as Cs₂CO₃, or phenolate such as sodium 2,6-di-tert-butyl-4-methyl-phenolate. The coupling reactions are preferably conducted in benzene, toluene, tetrahydrofuran, 1,2-dimethoxyethane, 1,4-dioxane, N,N-dimethylformamide, N,N-dimethylacetamide, N-methylpyrrolidinone, alcohol such as *t*BuOH, water, or mixtures thereof, at temperatures in the range of 20 to 180 °C. For employing the chloro-pyridine **IV** (LG = Cl) particularly suited reaction conditions include chloro[2-(dicyclohexylphosphino)-3,6-dimethoxy-2',4',6'-triisopropyl-1,1'-biphenyl][2-(2-aminoethyl)phenyl]palladium(II) as catalyst precursor and sodium *tert*-butoxide as base, in 1,4-dioxane or toluene at 60 to 110 °C. In case that alkyl- or benzylester derivatives of compounds **IV** are employed, the initially formed products **II** may *in situ* hydrolyze to the carboxylic acids **I**.

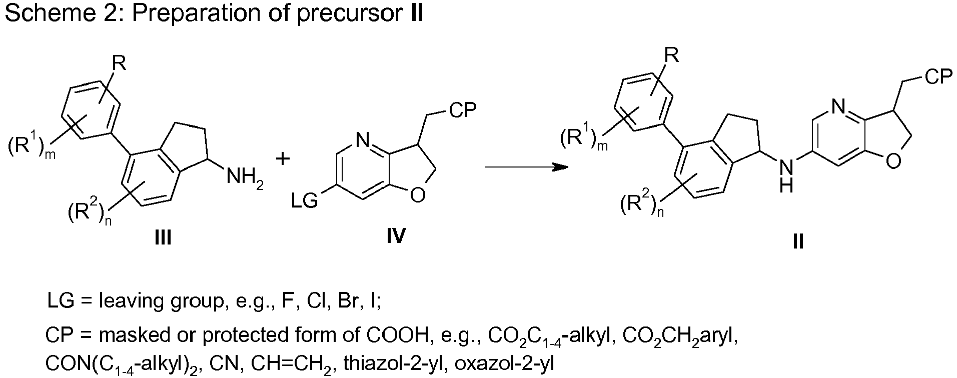

Intermediate **III** is conveniently obtained from indanol **V** which, in turn, may be prepared from indanone **VI** (Scheme 3); R, R¹, R², m and m in Scheme 3 have the meanings as defined hereinbefore and hereinafter.

The reduction of the keto group in compound **VI** is a standard transformation in organic synthesis which may be accomplished with lithium borohydride, sodium borohydride, lithium aluminum hydride, or diisobutylaluminum hydride. While sodium borohydride is employed in aqueous or alcoholic solution at 0 to 60 °C, the other reducing agents mentioned are preferably used in inert solvents, such as tetrahydrofuran, diethyl ether, dichloromethane, and toluene, at -80 to 60 °C. The reduction of the keto group may also be conducted in a stereoselective fashion providing the alcohol in enantiomerically enriched or pure form. Suited chiral reducing agents are boranes combined with an enantiomerically pure [1,3,2]oxazaborol (Corey-Bakshi-Shibata reaction or Corey-Itsuno reaction) or formic acid, formates, hydrogen, or silanes in the presence of an enantiomerically pure transition metal catalyst. Typical reaction conditions for the former approach comprise borane (complexed with, e.g., dimethyl sulfide) and (*R*)- or (*S*)-3,3-diphenyl-1-methyltetrahydro-1H,3H-pyrrolo[1,2-c][1,3,2]oxazaborol in, e.g., dichloromethane, toluene, methanol, tetrahydrofuran, or mixtures thereof, at 0 to 60 °C. Using a chiral transition metal catalyst, such as a ruthenium complex, e.g., chloro{[(1S,2S)-(-)-2-amino-1,2-diphenylethyl](4-toluenesulfonyl)-amido}-(mesitylene)ruthenium(II), may deliver the hydroxy compound with high enantiomeric excess using, e.g., formic acid in the presence of a base, e.g., triethylamine, in dichloromethane, at -20 to 60 °C. The OH group in compound **V** may be replaced with NH₂ following a two-step procedure via a protected, e.g., as phthalimide, or masked, e.g., as azide, amino derivative. Phthalimide can be introduced employing the conditions of the Mitsunobu reaction. The transformation is routinely conducted with phthalimide, a phosphine, and an azodicarboxylic ester or amide in tetrahydrofuran, 1,4-dioxane, diethyl ether, toluene, benzene, dichloromethane, or mixtures thereof, at -30 to 100 °C. Phosphines commonly used are triphenylphosphine and tributylphosphine which are regularly combined with dimethyl azodicarboxylate, diethyl azodicarboxylate, diisopropyl azodicarboxylate, di-(4-chlorobenzyl) azodicarboxylate, dibenzyl azodicarboxylate, di-tert-butyl azodicarboxylate, azodicarboxylic acid bis-(dimethylamide), azodicarboxylic acid dipiperidide, or azodicarboxylic acid dimorpholide. The amino group may be liberated from phthalimide using hydrazine in ethanol, ethylene-1,2-diamine in *n*-butanol, or 1-butylamine in *n*-butanol.

The azide group can be introduced from the hydroxy precursor **V** employing hydrazoic acid or phosphoryl azide and the conditions of the Mitsunobu reaction as described above or variants thereof. Phosphoryl azide combined with a base, such as 1,8-diazabicyclo[5.4.0]undecene, may also accomplish the transformation in tetrahydrofuran or toluene at -10 to 60°C. The azide is transformed into the amino function using, e.g., hydrogen in the presence of a transition metal such as palladium on carbon.

Both proceedings may give the aminoindane **III** in enantiomerically pure form when starting from the isomerically pure precursor **V.**

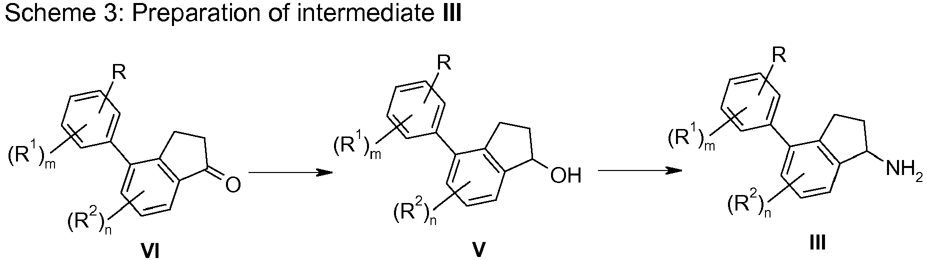

Compounds of type **IV** can be synthesized according to Scheme 4. First step is the alkylation of hydroxypyridines **VII** with bromides **VIII** in the presence of a base such as Na₂CO₃, K₂CO₃ or Cs₂CO₃, in a solvent like tetrahydrofuran, 1,2-dimethoxyethane, 1,4-dioxane, acetonitrile, N,N-dimethylformamide, or mixtures thereof, at 0 to 80 °C. Subsequently compounds **IX** can be transformed into compounds **IV** (step 2) via addition of an *in situ* generated carbon anion or radical to the double bond and subsequent trapping of the cyclic anion by a proton and the cyclic radical by a hydride source. nBuLi, iPrMgCl, iPrMgCl*LiCl, Mg, Mg*LiCl, Zn, and Zn*LiCl are preferred reagents to convert the C-l or C-Br bond into a C-M bond (M = Li, Mgl, Znl etc.) with sufficient nucleophilicity to add to the double bond. The reactions with lithium and magnesium reagents are preferably conducted in hexanes, tetrahydrofuran, diethyl ether, 1,2-dimethoxyethane, toluene, or mixtures thereof, at-100 to 60 °C, optionally in the presence of a copper-catalyst such as CuBrxSMe₂, Cul or CuCNx2LiCl. Zn is preferably used in tetrahydrofuran, dimethyl sulfoxide, N-methylpyrrolidinone, or mixtures thereof, at 0 to 100 °C. Often employed reaction conditions for the radical pathway are, e.g., tributyltin hydride or tris-(trimethylsilyl)-silane in the presence of a radical starter as for example azobisisobutyronitrile, in toluene or benzene, at 60 to 120 °C (see e.g. J. Org. Chem. 1987, 52, 4072-8). Compounds **IV** are thus typically obtained in racemic form (**rac-IV**). Column chromatography on chiral phases then gives compounds **IV** in enantiomeric enriched or enantiomeric pure form.

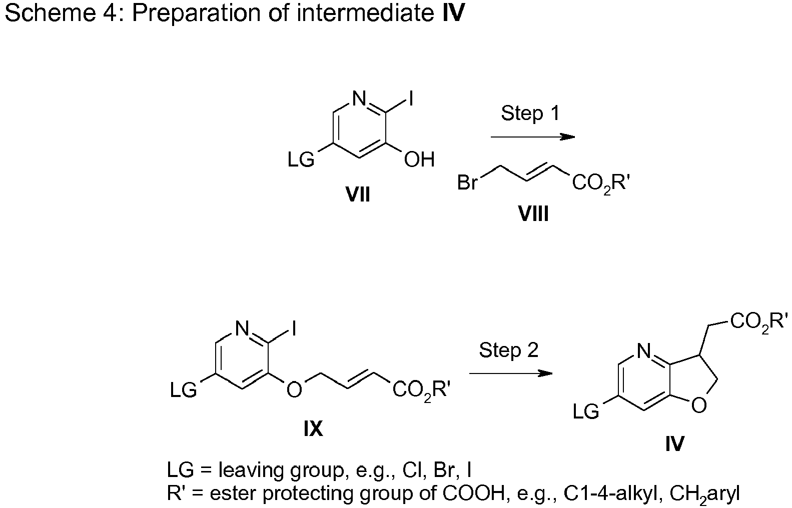

The synthetic routes presented may rely on the use of protecting groups. For example, potentially reactive groups present, such as hydroxy, carbonyl, carboxy, amino, alkylamino, or imino, may be protected during the reaction by conventional protecting groups which are cleaved again after the reaction. Suitable protecting groups for the respective functionalities and their removal are well known to the one skilled in the art and are described in the literature of organic synthesis for example in "Protecting Groups, 3rd Edition", Philip J. Kocienski, Theime, 2005 or "Greene's Protective Groups in Organic Synthesis, 4th Edition", Peter G. M. Wuts, Theadora W. Greene, John Wiley and Sons, 2007.

The compounds of general formula **I** may be resolved into their enantiomers and/or diastereomers as mentioned below. Thus, for example, *cis*/*trans* mixtures may be resolved into their *cis* and *trans* isomers and racemic compounds may be separated into their enantiomers.

The *cis*/*trans* mixtures may be resolved, for example, by chromatography into the *cis* and *trans* isomers thereof. The compounds of general formula **I** which occur as racemates may be separated by methods known *per se* into their optical antipodes and diastereomeric mixtures of compounds of general formula **I** may be resolved into their diastereomers by taking advantage of their different physico-chemical properties using methods known *per se,* e.g. chromatography and/or fractional crystallization; if the compounds obtained thereafter are racemates, they may be resolved into the enantiomers as mentioned below.

The racemates are preferably resolved by column chromatography on chiral phases or by crystallization from an optically active solvent or by reacting with an optically active substance which forms salts or derivatives such as esters or amides with the racemic compound. Salts may be formed with enantiomerically pure acids for basic compounds and with enantiomerically pure bases for acidic compounds. Diastereomeric derivatives are formed with enantiomerically pure auxiliary compounds, e.g. acids, their activated derivatives, or alcohols. Separation of the diastereomeric mixture of salts or derivatives thus obtained may be achieved by taking advantage of their different physico-chemical properties, e.g. differences in solubility; the free antipodes may be released from the pure diastereomeric salts or derivatives by the action of suitable agents. Optically active acids commonly used for such a purpose as well as optically active alcohols applicable as auxiliary residues are known to those skilled in the art.

As mentioned above, the compounds of formula **I** may be converted into salts, particularly for pharmaceutical use into the pharmaceutically acceptable salts. As used herein, "pharmaceutically acceptable salts" refer to derivatives of the disclosed compounds wherein the parent compound is modified by making acid or base salts thereof.

The compounds according to the invention are advantageously also obtainable using the methods described in the examples that follow, which may also be combined for this purpose with methods known to the skilled man from the literature.

### Terms and definitions

Terms not specifically defined herein should be given the meanings that would be given to them by one of skill in the art in light of the disclosure and the context. As used in the specification, however, unless specified to the contrary, the following terms have the meaning indicated and the following conventions are adhered to.

The terms "compound(s) according to this invention", "compound(s) of formula (I)", "compound(s) of the invention" and the like denote the compounds of the formula (I) according to the present invention including their tautomers, stereoisomers and mixtures thereof and the salts thereof, in particular the pharmaceutically acceptable salts thereof, and the solvates and hydrates of such compounds, including the solvates and hydrates of such tautomers, stereoisomers and salts thereof.

The terms "treatment" and "treating" embrace both preventative, i.e. prophylactic, or therapeutic, i.e. curative and/or palliative, treatment. Thus the terms "treatment" and "treating" comprise therapeutic treatment of patients having already developed said condition, in particular in manifest form. Therapeutic treatment may be symptomatic treatment in order to relieve the symptoms of the specific indication or causal treatment in order to reverse or partially reverse the conditions of the indication or to stop or slow down progression of the disease. Thus the compositions and methods of the present invention may be used for instance as therapeutic treatment over a period of time as well as for chronic therapy. In addition the terms "treatment" and "treating" comprise prophylactic treatment, i.e. a treatment of patients at risk to develop a condition mentioned hereinbefore, thus reducing said risk.

When this invention refers to patients requiring treatment, it relates primarily to treatment in mammals, in particular humans.

The term "therapeutically effective amount" means an amount of a compound of the present invention that (i) treats or prevents the particular disease or condition, (ii) attenuates, ameliorates, or eliminates one or more symptoms of the particular disease or condition, or (iii) prevents or delays the onset of one or more symptoms of the particular disease or condition described herein.

The terms "modulated" or "modulating", or "modulate(s)", as used herein, unless otherwise indicated, refer to the activation of the G-protein-coupled receptor GPR40 with one or more compounds of the present invention.

The terms "mediated" or "mediating" or "mediate", as used herein, unless otherwise indicated, refer to the (i) treatment, including prevention of the particular disease or condition, (ii) attenuation, amelioration, or elimination of one or more symptoms of the particular disease or condition, or (iii) prevention or delay of the onset of one or more symptoms of the particular disease or condition described herein.

The term "substituted" as used herein, means that any one or more hydrogens on the designated atom, radical or moiety is replaced with a selection from the indicated group, provided that the atom's normal valence is not exceeded, and that the substitution results in an acceptably stable compound.

In the groups, radicals, or moieties defined below, the number of carbon atoms is often specified preceding the group, for example, C₁₋₆-alkyl means an alkyl group or radical having 1 to 6 carbon atoms. In general, for groups comprising two or more subgroups, the last named subgroup is the radical attachment point, for example, the substituent "aryl-C₁₋₃-alkyl-" means an aryl group which is bound to a C₁₋₃-alkyl-group, the latter of which is bound to the core or to the group to which the substituent is attached.

In case a compound of the present invention is depicted in form of a chemical name and as a formula in case of any discrepancy the formula shall prevail.

An asterisk may be used in sub-formulas to indicate the bond which is connected to the core molecule as defined.

The numeration of the atoms of a substituent starts with the atom which is closest to the core or to the group to which the substituent is attached.

For example, the term "3-carboxypropyl-group" represents the following substituent: 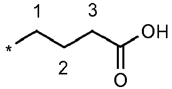 wherein the carboxy group is attached to the third carbon atom of the propyl group. The terms "1-methylpropyl-", "2,2-dimethylpropyl-" or "cyclopropylmethyl-" group represent the following groups:

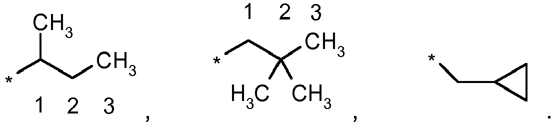

The asterisk may be used in sub-formulas to indicate the bond which is connected to the core molecule as defined.

In a definition of a group the term "wherein each X, Y and Z group is optionally substituted with" and the like denotes that each group X, each group Y and each group Z either each as a separate group or each as part of a composed group may be substituted as defined. For example a definition "R^{ex} denotes H, C₁₋₃-alkyl, C₃₋₆-cycloalkyl, C₃₋₆-cycloalkyl-C₁₋₃-alkyl or C₁₋₃-alkyl-O-, wherein each alkyl group is optionally substituted with one or more L^{ex}." or the like means that in each of the beforementioned groups which comprise the term alkyl, i.e. in each of the groups C₁₋₃-alkyl, C₃₋₆-cycloalkyl-C₁₋₃-alkyl and C₁₋₃-alkyl-O-, the alkyl moiety may be substituted with L^{ex} as defined.

Unless specifically indicated, throughout the specification and the appended claims, a given chemical formula or name shall encompass tautomers and all stereo, optical and geometrical isomers (e.g. enantiomers, diastereomers, E/Z isomers etc...) and racemates thereof as well as mixtures in different proportions of the separate enantiomers, mixtures of diastereomers, or mixtures of any of the foregoing forms where such isomers and enantiomers exist, as well as salts, including pharmaceutically acceptable salts thereof and solvates thereof such as for instance hydrates including solvates of the free compounds or solvates of a salt of the compound.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, and commensurate with a reasonable benefit/risk ratio.

As used herein, "pharmaceutically acceptable salts" refer to derivatives of the disclosed compounds wherein the parent compound is modified by making acid or base salts thereof.

Salts of other acids than those mentioned above which for example are useful for purifying or isolating the compounds of the present invention (e.g. trifluoro acetate salts) also comprise a part of the invention.

The term halogen generally denotes fluorine, chlorine, bromine and iodine.

The term "C₁₋ₙ-alkyl", wherein n is an integer from 1 to n, either alone or in combination with another radical denotes an acyclic, saturated, branched or linear hydrocarbon radical with 1 to n C atoms. For example the term C₁₋₅-alkyl embraces the radicals H₃C-, H₃C-CH₂-, H₃C-CH₂-CH₂-, H₃C-CH(CH₃)-, H₃C-CH₂-CH₂-CH₂-, H₃C-CH₂-CH(CH₃)-, H₃C-CH(CH₃)-CH₂-, H₃C-C(CH₃)₂-, H₃C-CH₂-CH₂-CH₂-CH₂-, H₃C-CH₂-CH₂-CH(CH₃)-, H₃C-CH₂-CH(CH₃)-CH₂-, H₃C-CH(CH₃)-CH₂-CH₂-, H₃C-CH₂-C(CH₃)₂-, H₃C-C(CH₃)₂-CH₂-, H₃C-CH(CH₃)-CH(CH₃)- and H₃C-CH₂-CH(CH₂CH₃)-.

The term "C₁₋ₙ-alkylene" wherein n is an integer 1 to n, either alone or in combination with another radical, denotes an acyclic, straight or branched chain divalent alkyl radical containing from 1 to n carbon atoms. For example the term C₁₋₄-alkylene includes -(CH₂)-, -(CH₂-CH₂)-, -(CH(CH₃))-, -(CH₂-CH₂-CH₂)-, -(C(CH₃)₂)-,-(CH(CH₂CH₃))-, -(CH(CH₃)-CH₂)-, -(CH₂-CH(CH₃))-, -(CH₂-CH₂-CH₂-CH₂)-, -(CH₂-CH₂-CH(CH₃))-, -(CH(CH₃)-CH₂-CH₂)-, -(CH₂-CH(CH₃)-CH₂)-, -(CH₂-C(CH₃)₂)-, -(C (CH₃)₂-CH₂)-, -(CH(CH₃)-CH(CH₃))-, -(CH₂-CH(CH₂CH₃))-, -(CH(CH₂CH₃)-CH₂)-, -(CH(CH₂CH₂CH₃))-, -(CHCH(CH₃)₂)- and -C(CH₃)(CH₂CH₃)-.

The term "C₂₋ₙ-alkenyl", is used for a group as defined in the definition for "C₁₋ₙ-alkyl" with at least two carbon atoms, if at least two of those carbon atoms of said group are bonded to each other by a double bond. For example the term C₂₋₃-alkenyl includes -CH=CH₂, -CH=CH-CH₃, -CH₂-CH=CH₂.

The term "C₂₋ₙ-alkynyl", is used for a group as defined in the definition for "C₁₋ₙ-alkyl" with at least two carbon atoms, if at least two of those carbon atoms of said group are bonded to each other by a triple bond. For example the term C₂₋₃-alkynyl includes -C≡CH, -C≡C-CH₃, -CH₂-C≡CH.

The term "C₃₋ₙ-cycloalkyl", wherein n is an integer 4 to n, either alone or in combination with another radical denotes a cyclic, saturated, unbranched hydrocarbon radical with 3 to n C atoms. The cyclic group may be mono-, bi-, tri- or spirocyclic, most preferably monocyclic. Examples of such cycloalkyl groups include cyclopropyl, cyclobutyl, cyclopentyl, cyclohexyl, cycloheptyl, cyclooctyl, cyclononyl, cyclododecyl, bicyclo[3.2.1.]octyl, spiro[4.5]decyl, norpinyl, norbonyl, norcaryl, adamantyl, etc.

Many of the terms given above may be used repeatedly in the definition of a formula or group and in each case have one of the meanings given above, independently of one another.

### Pharmacological Activity

The activity of the compounds of the invention may be demonstrated using the following assay:
IP₁ accumulation measurements using the IPOne assay system - 1321N1 cells stably expressing human GPR40 receptor (Euroscreen, Belgium) are seeded 24 h before the assay in white 384-well plates in culture medium containing 10% FCS, 1% Na-Pyruvate and 400 µg/mL G418. IP₁ is assayed according to the manufacturer's description (Cisbio Bioassays, France). In brief, the assay is started by substitution of the culture medium by stimulation buffer (Hepes 10 mM, CaCl₂ 1 mM, MgCl₂ 0.5 mM, KCI 4.2 mM, NaCl 146 mM, glucose 5.5 mM and LiCI 50 mM, pH 7.4). Cells are stimulated for 1 h at 37 °C, 5% CO₂ by addition of the compounds that are diluted in stimulation buffer containing LiCI. Assays are stopped by adding HTRF-conjugates (IP1-d2 and Anti-IP1 cryptate Tb) and lysis buffer, provided by the manufacturer. After an incubation time of 1 h at room temperature plates are measured using an EnVision™, Perkin Elmer. The obtained fluorescence ratios at 665/615 nM are then used to calculate the pEC₅₀ values using Assay Explorer 3.3 Software (Accelrys, Inc.) by interpolation using an IP₁ reference curve and subsequent sigmoidal curve fitting allowing for a variable hill slope.

The compounds according to the invention typically have EC₅₀ values in the range from about 1 nM to about 10 µM, preferably less than 1 µM, more preferably less than 100 nM.

EC₅₀ values for compounds according to the invention are shown in the following table. The number of the compound corresponds to the number of the Example in the experimental section.

**Table 2:**

| Example | EC₅₀ [nM] | Example | EC₅₀ [nM] | Example | EC₅₀ [nM] | Example | EC₅₀ [nM] |
|---|---|---|---|---|---|---|---|
| 1 | 57 | 2 | 61 | 3 | 42 | 4 | 64 |
| 5 | 26 | 118 | 158 | 119 | 90 | 120 | 52 |
| 121 | 61 | 122 | 24 | 123 | 153 | 124 | 75 |
| 125 | 693 | 126 | 1871 | 127 | 1238 | 128 | 126 |
| 129 | 184 | 130 | >10000 | 131 | 6389 | 132 | 36 |
| 133 | 33 | | | | | | |

### Chemical Stability

Degradation kinetics is used to simulate chemical stability of compounds in the acidic part of the gastro intestinal tract. The compounds of the invention show superior chemical stability in acidic aqueous media (pH value ca. 1.2) compared to the bulk of compounds explicitly disclosed in WO 2013/144097 and WO 2013/144098. Their application as medical drugs to treat human diseases is therefore less restricted and troublesome.

The chemical stability of the compounds of the invention at pH value of ca. 1.2 is determined as follows:
Compound is dissolved in an HPLC vial either in a mixture of acetonitrile/0.1 M aqueous HCI (2:3; pH ca. 1.2) or in a mixture of acetonitrile/McIIvaine buffer pH 7.4 (2:3) to get a concentration of approximately 0.25 mg/ml. The vial was then transferred into an HPLC autosampler system and maintained at a temperature of 37 °C. A first sample is taken and injected immediately into a standard HPLC system with a UV DAD detector. Further samples are injected after 2, 4, 6, 8 and 10 hours. Degradation of the compound is measured by determining the recovery rate of compound [%] for each injection using an HPLC standard gradient method. Therefore the peak area of the main peak for the first injection (AUₜ₀) is determined and set as 100%. Peak area of the main peak is determined also for the further injections (AUₜₙ, _{n= 2, 4, 6, 8, 10}) and expressed as fraction of (AUₜ₀)/ (AUₜₙ, _{n= 2, 4, 6, 8, 10}) [%].

The recovery rate of the compounds according to the invention after 2 h at pH value of ca. 1.2 determined as described above is typically above 95%, preferably above 98%.

The following table compares the recovery rate after 2 h at pH value of ca. 1.2 of compounds according to the invention and compounds of WO 2013/144097 and WO 2013/144098.

**Table 3:**

| Example in this invention | Amount of degradation after 2 h | Example (in) | Amount of degradation after 2 h |
|---|---|---|---|
| 2 | 0.2% | 4 (WO 2013/144097) | 13% |
| 3 | <0.5% | 2 (WO 2013/144098) | 94% |
| 4 | <0.5% | | |
| 5 | 0.4% | 95 (WO 2013/144097) | 4% |

Chemical structures of the examples from case WO 2013/144097 and WO 2013/144098 listed in the tables 3 to 6:

| | |
|---|---|
| Example 4 in WO 2013/144097 | Example 95 in WO 2013/144097 |
| 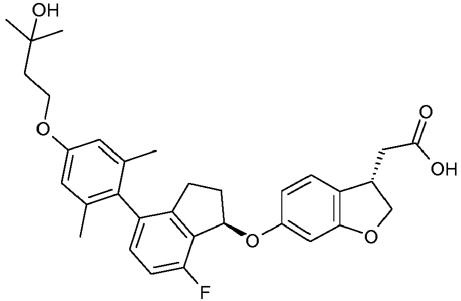 | 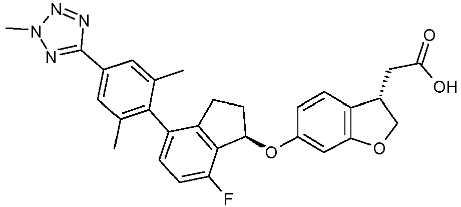 |
| Example 2 in WO 2013/144098 | Example 7 in WO 2013/144098 |
| 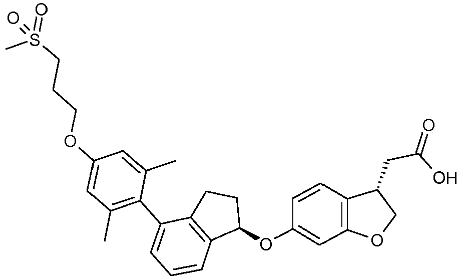 | 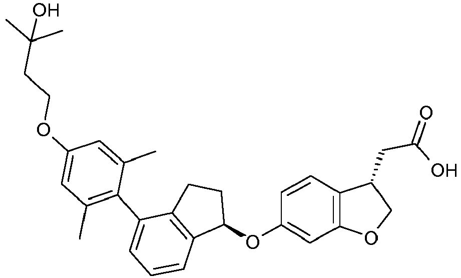 |

### Solubility

The aqueous solubility of the compounds of the invention is determined by comparing the amount dissolved in buffer to the amount in an acetonitrile/water (1/1) solution. Starting from a 10 mM DMSO stock solution aliquots are diluted with acetonitrile/water (1/1) or buffer, respectively. After 24 h of shaking, the solutions are filtrated and analyzed by LC-UV. The amount dissolved in buffer is compared to the amount in the acetonitrile solution.

Solubility is usually being measured from 0.001 to 0.125 mg/mL at a DMSO concentration of 2.5%. If more than 90% of the compound is dissolved in buffer, the value is marked with ">".

The compounds of the invention show higher solubility at low pH value (pH 2.2, emulating the acidic part of the gastro intestinal tract) compared with the bulk of compounds explicitly disclosed in WO 2013/144097. Their development and application is therefore more convenient and reliable. The following table presents the data for selected compounds of this invention and compounds from WO 2013/144097 (for structures see above below Table 3).

**Table 4:**

| Example in this invention | Solubility at pH value 2.2 [µg/mL] | Example in WO 2013/144097 | Solubility at pH value 2.2 [µg/mL] |
|---|---|---|---|
| 2 | 60 | 4 | <1 |
| 3 | 6 | | |
| 4 | 57 | | |
| 5 | 7 | 95 | <1 |
| 118 | 66 | | |
| 119 | 16 | | |
| 120 | 29 | | |
| 121 | 51 | | |
| 122 | 45 | | |
| 123 | <1 | | |
| 124 | 6 | | |
| 125 | 22 | | |
| 126 | 79 | | |
| 127 | 30 | | |
| 128 | 1 | | |
| 129 | 4 | | |
| 130 | 84 | | |
| 131 | 67 | | |
| 132 | 3 | | |
| 133 | 78 | | |

### Inhibition of CYP-2C8

The inhibition of cytochrome P450 2C8-isoenzyme catalyzed deethylation of amodiaquine by the test compound is assayed at 37 °C with human liver microsomes. All assays are carried out on a robotic system in 96 well plates. The final incubation volume contains TRIS buffer (0.1 M), MgCl₂ (5 mM), human liver microsomes (0.05 mg/mL), amodiaquine (1 µM) and the test compound at five different concentrations or no compound (high control) in duplicate (e.g. highest concentration 10-50 µM with subsequent serial 1:4 dilutions). Following a short preincubation period, reactions are started with the cofactor (NADPH, 1 mM) and stopped by cooling the incubation down to 8 °C and subsequently by addition of one volume of acetonitrile. An internal standard solution - the stable isotope d5-desethylamodiaquine - is added after quenching of incubations. Peak area analyte (= metabolite formed) and internal standard is determined by LC-MS/MS. The resulting peak area ratio analyte to internal standard in these incubations is compared to a control activity containing no test compound. Within each of the assay runs, the IC₅₀ of a positive control inhibitor (Montelukast) is determined. Experimental IC₅₀ values are calculated by least square regression according to the following equation: $% control activity = \left(100 % control activity/\left(1+\left(I/{IC}_{50}\right)S\right)\right) - B$ with I = inhibitor concentration; S = slope factor; B = background activity (lower plateau of the inhibition curve)

If the inhibition of the reaction is already >50% at the lowest concentration of the test compound, the IC₅₀ is assigned "< lowest concentration tested" (usually <0.4 µM). If the inhibition of the reaction is still <50% at the highest concentration of the test compound, the IC₅₀ is assigned "> highest concentration tested" (usually >50 µM).

The compounds of the invention show lower inhibition of the cytochrome P450 2C8-isoenzyme compared with the bulk of compounds explicitly disclosed in WO 2013/144097 and WO 2013/144098. Their potential for causing unwanted side-effects is therefore decreased. The following table displays the data for selected compounds of this invention and compounds from WO 2013/144097 and WO 2013/144098 (for structures see above below Table 3).

**Table 5:**

| Example in this invention | IC₅₀ [µM] | Example (in) | IC₅₀ [µM] |
|---|---|---|---|
| 1 | 17 | | |
| 2 | 32 | 4 (WO 2013/144097) | 6 |
| 3 | 29 | | |
| 4 | 43 | 7 (WO 2013/144098) | 5 |
| 5 | 22 | 95 (WO 2013/144097) | 9 |
| | | 2 (WO 2013/144098) | 19 |
| 118 | >50 | | |
| 119 | 41 | | |
| 120 | 12 | | |
| 121 | 29 | | |
| 122 | 32 | | |
| 123 | 9 | | |
| 124 | 9 | | |
| 125 | 21 | | |
| 126 | 37 | | |
| 127 | 27 | | |
| 128 | 14 | | |
| 129 | 12 | | |
| 130 | >50 | | |
| 131 | 37 | | |
| 132 | 23 | | |
| 133 | 21 | | |

### Inhibition of CYP-2C9

The inhibition of cytochrome P450 2C9-isoenzyme catalysed hydroxylation of diclofenac by the test compound is assayed at 37°C with human liver microsomes. All assays are carried out on a robotic system in 96 well plates. The final incubation volume contains TRIS buffer (0.1 M), MgCl₂ (5 mM), human liver microsomes (0.1 mg/mL), diclofenac (10 µM) and the test compound at five different concentrations or no compound (high control) in duplicate (e.g. highest concentration 10-50 µM with subsequent serial 1:4 dilutions). Following a short preincubation period, reactions are started with the cofactor (NADPH, 1 mM) and stopped by cooling the incubation down to 8°C and subsequently by addition of one volume of acetonitrile. An internal standard solution - the stable isotope 13C6-hydroxydiclofenac - is added after quenching of incubations. Peak area analyte (=metabolite formed) and internal standard is determined by LC-MS/MS. The resulting peak area ratio analyte to internal standard in these incubations is compared to a control activity containing no test compound. Within each of the assay runs, the IC50 of a positive control inhibitor (sulfaphenazole) is determined. Experimental IC50 values are calculated by least square regression according to the following equation: $% control activity = \left(100 % control activity/\left(1+\left(I/IC50\right)S\right)\right) ) - B$ with
I= inhibitor concentration
S= slope factor
$B = background activity \left(lower plateau of the inhibition curve\right)$

If the inhibition of the reaction is already >50% at the lowest concentration of the test compound, the IC50 is assigned "< lowest concentration tested" (usually <0.4 µM). If the inhibition of the reaction is still <50% at the highest concentration of the test compound, the IC50 is assigned "> highest concentration tested" (usually >50 µM).

The compounds of the invention show lower inhibition of the cytochrome P450 2C9-isoenzyme compared with the bulk of compounds explicitly disclosed in WO 2013/144097 and WO 144098. Their potential for causing unwanted side-effects is therefore decreased. The following table displays the data for selected compounds of this invention and compounds from WO 2013/144097 and WO 144098.

**Table 6:**

| Example in this invention | IC₅₀ [µM] | Example (in) | IC₅₀ [µM] |
|---|---|---|---|
| 1 | >50 | | |
| 2 | >50 | 4 (WO 2013/144097) | 21 |
| 3 | >50 | | |
| 4 | >50 | 7 (WO 2013/144098) | >10 |
| 5 | 48 | 95 (WO 2013/144097) | 19 |
| 118 | >50 | | |
| 119 | >50 | | |
| 120 | >50 | | |
| 121 | >50 | | |
| 122 | >50 | | |
| 123 | 28 | | |
| 124 | 49 | | |
| 125 | 47 | | |
| 126 | >50 | | |
| 127 | >50 | | |
| 128 | >50 | | |
| 129 | 50 | | |
| 130 | >50 | | |
| 131 | >50 | | |
| 132 | >50 | | |
| 133 | >50 | | |

In view of their ability to modulate the activity of the G-protein-coupled receptor GPR40, in particular an agonistic activity, the compounds of general formula I according to the invention, including the corresponding salts thereof, are theoretically suitable for the treatment of all those diseases or conditions which may be affected or which are mediated by the activation of the G-protein-coupled receptor GPR40.

Accordingly, the present invention relates to a compound of general formula I as a medicament.

Furthermore, the present invention relates to the use of a compound of general formula **I** or a pharmaceutical composition according to this invention for the treatment and/or prevention of diseases or conditions which are mediated by the activation of the G-protein-coupled receptor GPR40 in a patient, preferably in a human.

In yet another aspect the present invention relates to a method for treating a disease or condition mediated by the activation of the G-protein-coupled receptor GPR40 in a mammal that includes the step of administering to a patient, preferably a human, in need of such treatment a therapeutically effective amount of a compound or a pharmaceutical composition of the present invention.

Diseases and conditions mediated by agonists of the G-protein-coupled receptor GPR40 embrace metabolic diseases or conditions. According to one aspect the compounds and pharmaceutical compositions of the present invention are particularly suitable for treating diabetes mellitus, in particular Type 2 diabetes, Type 1 diabetes, complications of diabetes (such as e.g. retinopathy, nephropathy or neuropathies, diabetic foot, ulcers or macroangiopathies), metabolic acidosis or ketosis, reactive hypoglycaemia, hyperinsulinaemia, glucose metabolic disorder, insulin resistance, metabolic syndrome, dyslipidaemias of different origins, atherosclerosis and related diseases, obesity, high blood pressure, chronic heart failure, oedema and hyperuricaemia.

The compounds and pharmaceutical compositions of the present invention are also suitable for preventing beta-cell degeneration such as e.g. apoptosis or necrosis of pancreatic beta cells. The compounds and pharmaceutical compositions of the present invention are also suitable for improving or restoring the functionality of pancreatic cells, and also for increasing the number and size of pancreatic beta cells.

Therefore according to another aspect the invention relates to compounds of formula I and pharmaceutical compositions according to the invention for use in preventing, delaying, slowing the progression of and/or treating metabolic diseases, particularly in improving the glycaemic control and/or beta cell function in the patient.

In another aspect the invention relates to compounds of formula I and pharmaceutical compositions according to the invention for use in preventing, delaying, slowing the progression of and/or treating type 2 diabetes, overweight, obesity, complications of diabetes and associated pathological conditions.

In addition the compounds and pharmaceutical compositions according to the invention are suitable for use in one or more of the following therapeutic processes:
- for preventing, delaying, slowing the progression of or treating metabolic diseases, such as for example type 1 diabetes, type 2 diabetes, insufficient glucose tolerance, insulin resistance, hyperglycaemia, hyperlipidaemia, hypercholesterolemia, dyslipidaemia, syndrome X, metabolic syndrome, obesity, high blood pressure, chronic systemic inflammation, retinopathy, neuropathy, nephropathy, atherosclerosis, endothelial dysfunction or bone-related diseases (such as osteoporosis, rheumatoid arthritis or osteoarthritis);
- for improving glycaemic control and/or reducing fasting plasma glucose, postprandial plasma glucose and/or the glycosylated haemoglobin HbA1c;
- for preventing, delaying, slowing or reversing the progression of disrupted glucose tolerance, insulin resistance and/or metabolic syndrome to type 2 diabetes;
- for preventing, delaying, slowing the progression of or treating a condition or a disease selected from among the complications of diabetes, such as for example retinopathy, nephropathy or neuropathies, diabetic foot, ulcers or macroangiopathies;
- for reducing weight or preventing weight gain or assisting weight loss;
- for preventing or treating the degradation of pancreatic beta cells and/or improving and/or restoring the functionality of pancreatic beta cells and/or restoring the functionality of pancreatic insulin secretion;
- for maintaining and/or improving insulin sensitivity and/or preventing or treating hyperinsulinaemia and/or insulin resistance.

In particular, the compounds and pharmaceutical compositions according to the invention are suitable for the treatment of obesity, diabetes (comprising type 1 and type 2 diabetes, preferably type 2 diabetes mellitus) and/or complications of diabetes (such as for example retinopathy, nephropathy or neuropathies, diabetic foot, ulcers or macroangiopathies).

The compounds according to the invention are most particularly suitable for treating type 2 diabetes mellitus.

The dose range of the compounds of general formula I applicable per day is usually from 0.001 to 10 mg per kg body weight, for example from 0.01 to 8 mg per kg body weight of the patient. Each dosage unit may conveniently contain from 0.1 to 1000 mg, for example 0.5 to 500 mg.

The actual therapeutically effective amount or therapeutic dosage will of course depend on factors known by those skilled in the art such as age and weight of the patient, route of administration and severity of disease. In any case the compound or composition will be administered at dosages and in a manner which allows a therapeutically effective amount to be delivered based upon patient's unique condition.

The compounds, compositions, including any combinations with one or more additional therapeutic agents, according to the invention may be administered by oral, transdermal, inhalative, parenteral or sublingual route. Of the possible methods of administration, oral or intravenous administration is preferred.

### Pharmaceutical Compositions

Suitable preparations for administering the compounds of formula **I**, optionally in combination with one or more further therapeutic agents, will be apparent to those with ordinary skill in the art and include for example tablets, pills, capsules, suppositories, lozenges, troches, solutions, syrups, elixirs, sachets, injectables, inhalatives and powders etc. Oral formulations, particularly solid forms such as e.g. tablets or capsules are preferred. The content of the pharmaceutically active compound(s) is advantageously in the range from 0.1 to 90 wt.-%, for example from 1 to 70 wt.-% of the composition as a whole.

Suitable tablets may be obtained, for example, by mixing one or more compounds according to formula **I** with known excipients, for example inert diluents, carriers, disintegrants, adjuvants, surfactants, binders and/or lubricants. The tablets may also consist of several layers. The particular excipients, carriers and/or diluents that are suitable for the desired preparations will be familiar to the skilled man on the basis of his specialist knowledge. The preferred ones are those that are suitable for the particular formulation and method of administration that are desired. The preparations or formulations according to the invention may be prepared using methods known *per se* that are familiar to the skilled man, such as for example by mixing or combining at least one compound of formula **I** according to the invention, or a pharmaceutically acceptable salt of such a compound, and one or more excipients, carriers and/or diluents.

### Combination Therapy

The compounds of the invention may further be combined with one or more, preferably one additional therapeutic agent. According to one embodiment the additional therapeutic agent is selected from the group of therapeutic agents useful in the treatment of diseases or conditions described hereinbefore, in particular associated with metabolic diseases or conditions such as for example diabetes mellitus, obesity, diabetic complications, hypertension, hyperlipidemia. Additional therapeutic agents which are suitable for such combinations include in particular those which for example potentiate the therapeutic effect of one or more active substances with respect to one of the indications mentioned and/or which allow the dosage of one or more active substances to be reduced.

Therefore a compound of the invention may be combined with one or more additional therapeutic agents selected from the group consisting of antidiabetic agents, agents for the treatment of overweight and/or obesity and agents for the treatment of high blood pressure, heart failure and/or atherosclerosis.

Antidiabetic agents are for example metformin, sulphonylureas, nateglinide, repaglinide, thiazolidinediones, PPAR-(alpha, gamma or alpha/gamma) agonists or modulators, alpha-glucosidase inhibitors, DPPIV inhibitors, SGLT2-inhibitors, insulin and insulin analogues, GLP-1 and GLP-1 analogues or amylin and amylin analogues, cycloset, 11β-HSD inhibitors. Other suitable combination partners are inhibitors of protein tyrosinephosphatase 1, substances that affect deregulated glucose production in the liver, such as e.g. inhibitors of glucose-6-phosphatase, or fructose-1,6-bisphosphatase, glycogen phosphorylase, glucagon receptor antagonists and inhibitors of phosphoenol pyruvate carboxykinase, glycogen synthase kinase or pyruvate dehydrokinase, alpha2-antagonists, CCR-2 antagonists or glucokinase activators. One or more lipid lowering agents are also suitable as combination partners, such as for example HMG-CoA-reductase inhibitors, fibrates, nicotinic acid and the derivatives thereof, PPAR-(alpha, gamma or alpha/gamma) agonists or modulators, PPAR-delta agonists, ACAT inhibitors or cholesterol absorption inhibitors such as, bile acid-binding substances such as, inhibitors of ileac bile acid transport, MTP inhibitors, or HDL-raising compounds such as CETP inhibitors or ABC1 regulators.

Therapeutic agents for the treatment of overweight and/or obesity are for example antagonists of the cannabinoid1 receptor, MCH-1 receptor antagonists, MC4 receptor agonists, NPY5 or NPY2 antagonists, β3-agonists, leptin or leptin mimetics, agonists of the 5HT2c receptor.

Therapeutic agents for the treatment of high blood pressure, chronic heart failure and/or atherosclerosis are for example A-II antagonists or ACE inhibitors, ECE inhibitors, diuretics, β-blockers, Ca-antagonists, centrally acting antihypertensives, antagonists of the alpha-2-adrenergic receptor, inhibitors of neutral endopeptidase, thrombocyte aggregation inhibitors and others or combinations thereof are suitable. Angiotensin II receptor antagonists are preferably used for the treatment or prevention of high blood pressure and complications of diabetes, often combined with a diuretic such as hydrochlorothiazide.

The dosage for the combination partners mentioned above is usually 1/5 of the lowest dose normally recommended up to 1/1 of the normally recommended dose.

Preferably, compounds of the present invention and/or pharmaceutical compositions comprising a compound of the present invention optionally in combination with one or more additional therapeutic agents are administered in conjunction with exercise and/or a diet.

Therefore, in another aspect, this invention relates to the use of a compound according to the invention in combination with one or more additional therapeutic agents described hereinbefore and hereinafter for the treatment of diseases or conditions which may be affected or which are mediated by the activation of the G-protein-coupled receptor GPR40, in particular diseases or conditions as described hereinbefore and hereinafter.

In yet another aspect the present invention relates a method for treating a disease or condition mediated by the activation of the G-protein-coupled receptor GPR40 in a patient that includes the step of administering to the patient, preferably a human, in need of such treatment a therapeutically effective amount of a compound of the present invention in combination with a therapeutically effective amount of one or more additional therapeutic agents described in hereinbefore and hereinafter.

The use of the compound according to the invention in combination with the additional therapeutic agent may take place simultaneously or at staggered times. The compound according to the invention and the one or more additional therapeutic agents may both be present together in one formulation, for example a tablet or capsule, or separately in two identical or different formulations, for example as a so-called kit-of-parts.

Consequently, in another aspect, this invention relates to a pharmaceutical composition which comprises a compound according to the invention and one or more additional therapeutic agents described hereinbefore and hereinafter, optionally together with one or more inert carriers and/or diluents.

Other features and advantages of the present invention will become apparent from the following more detailed Examples which illustrate, by way of example, the principles of the invention.

### Examples / Preliminary remarks:

The terms "ambient temperature" and "room temperature" are used interchangeably and designate a temperature of about 20 °C.

As a rule, ¹H-NMR and/or mass spectra have been obtained for the compounds prepared.

Analytical HPLC parameters employed for characterization of products (TFA denotes trifluoroacetic acid and FA denotes formic acid):

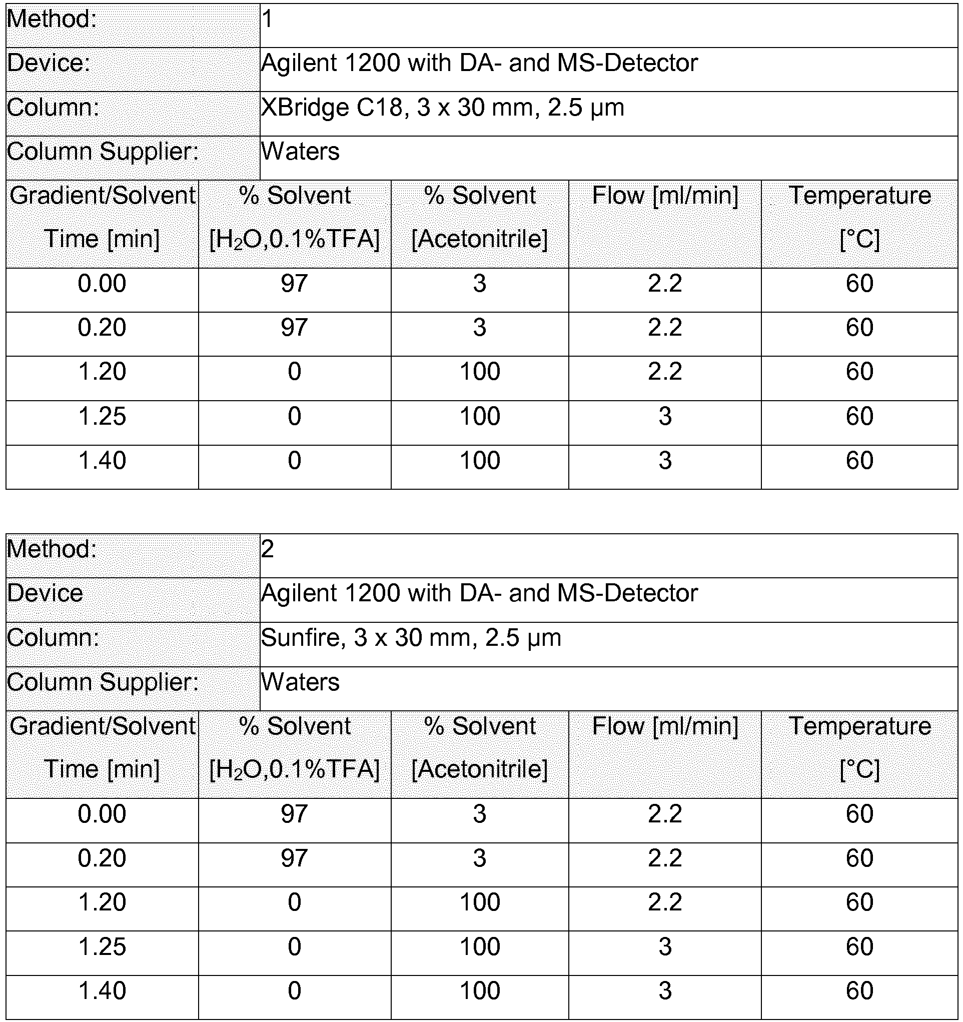

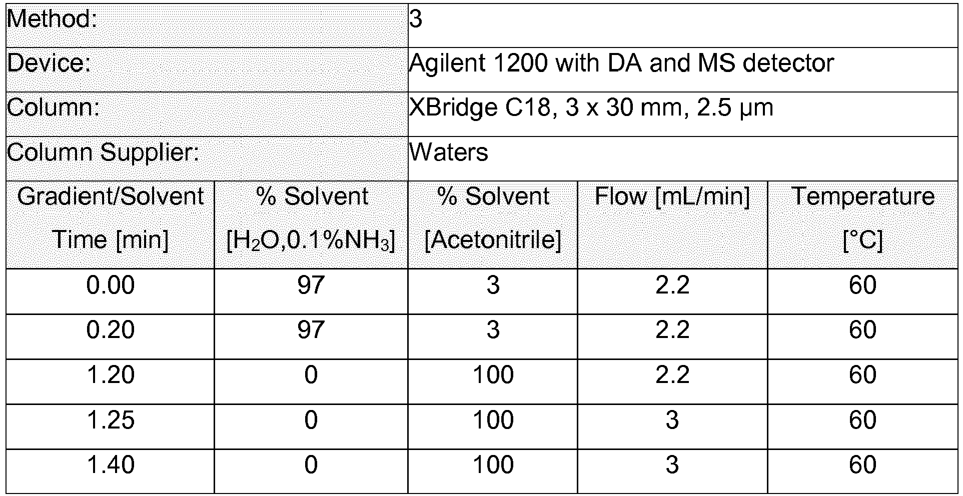

Analytical chiral HPLC parameters employed for characterization of products

| | | | | | |
|---|---|---|---|---|---|
| Method: | | 1a | | | |
| Device: | | Agilent 1260 SFC with DAD and MS | | | |
| Column: | | Daicel Chiralpak® IC, 4.6 x 250 mm, 5 µm | | | |

| Gradient/Solvent Time [min] | % Solvent [csCO₂] | % Solvent [Isopropyl acetate 20 mM NH₃] | Flow [mL/min] | Temperature [°C] | Back pressure [psi] |
|---|---|---|---|---|---|
| 0.0 | 90 | 10 | 4.0 | 40.0 | 2175.0 |
| 10.0 | 90 | 10 | 4.0 | 40.0 | 2175.0 |

| | | | | | |
|---|---|---|---|---|---|
| Method: | | 1b | | | |
| Device: | | Agilent 1260 SFC with DAD and ELSD | | | |
| Column: | | Daicel Chiralcel® OZ-H, 4.6 x 250 mm, 5 µm | | | |

| Gradient/Solvent Time [min] | % Solvent [csCO₂] | % Solvent [Methanol 20 mM NH₃] | Flow [mL/min] | Temperature [°C] | Back pressure [psi] |
|---|---|---|---|---|---|
| 0.0 | 65 | 35 | 4.0 | 40.0 | 2175.0 |
| 10.0 | 65 | 35 | 4.0 | 40.0 | 2175.0 |

The Examples that follow are intended to illustrate the present invention without restricting it:

### Intermediate 1

### Methyl 2-{6-chloro-2H,3H-furo[3,2-b]pyridin-3-yl}acetate

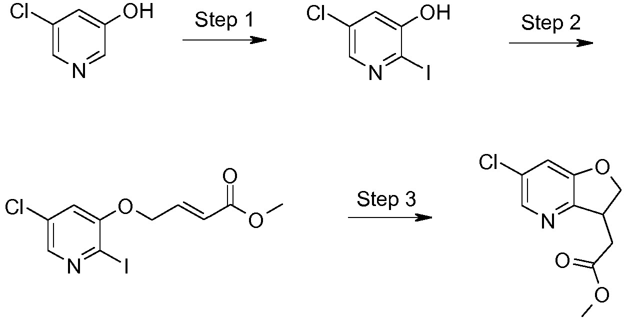

### Step 1: 5-chloro-2-iodopyridin-3-ol

To a mixture of 5-chloro-pyridin-3-ol (2.6 g) in water (20 mL) are added Na₂CO₃ (20.5 mL of a 2 M solution in water and portionwise I₂ (5.2 g). The mixture is stirred for 12 hours at room temperature and treated with hydrochloric acid (4 M) until a pH-value of 3 is reached. The mixture is stirred for 10 minutes, the precipitate is filtered off, washed with water and dried in vacuo to give the title compound. Mass spectrum (ESI⁺): m/z = 256 [M+H]+.

### Step 2: Methyl (2E)-4-[(5-chloro-2-iodopyridin-3-yl)oxy]but-2-enoate

To a mixture of 5-chloro-2-iodopyridin-3-ol (3.34 g) and K₂CO₃ (4.5 g) in N,N-dimethylformamide (20 mL) is added dropwise methyl (2E)-4-bromobut-2-enoate (2.3 mL). The mixture is stirred for 15 minutes, diluted with ethylacetate and washed successively with H₃PO₄ (1 M aqueous solution) and brine. After drying (MgSO₄) the solvents are evaporated in vacuo and the residue is chromatographed on silica gel (petroleum ether/ethyl acetate 90:10→70:30) to give the title compound. Mass spectrum (ESI⁺): m/z = 354 [M+H]+.

### Step 3: Methyl 2-{6-chloro-2H,3H-furo[3,2-b]pyridin-3-yl}acetate

Methyl (2E)-4-[(5-chloro-2-iodopyridin-3-yl)oxy]but-2-enoate (3.92 g), tris-(trimethylsilyl)-silane (5.52 g) and azo-bis-(isobutyronitrile) (AIBN, 180 mg) are dissolved in toluene (52 mL) and heated to 90°C for 1 hour. The solvent is evaporated in vacuo and the residue is chromatographed on silica gel (petroleum ether/ethyl acetate 90:10→60:40) to give the title compound. Mass spectrum (ESI⁺): m/z = 228 [M+H]+.

### Intermediate 2 and 3

### Methyl 2-[(3S)-6-chloro-2H,3H-furo[3,2-b]pyridin-3-yl]acetate and Methyl 2-[(3R)-6-chloro-2H,3H-furo[3,2-b]pyridin-3-yl]acetate

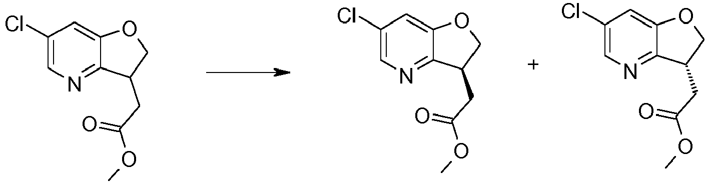

Methyl 2-{6-chloro-2H,3H-furo[3,2-b]pyridin-3-yl}acetate is separated into the enantiomers by SFC on chiral phase (column: Daicel Chirakpak IC, 5 µm, 250 mm x 20 mm; eluent: scCO₂/isopropanol 90:10, 60 mL/min, 40°C, backpressure 150 bar). Methyl 2-[(3S)-6-chloro-2H,3H-furo[3,2-b]pyridin-3-yl]acetate: LC (method 1a): t_{R} = 1.33 min.

Methyl 2-[(3R)-6-chloro-2H,3H-furo[3,2-b]pyridin-3-yl]acetate: LC (method 1a): t_{R} = 1.77 min.

### Intermediate 4

### 4-{4-[(1R)-1-Amino-7-fluoro-2,3-dihydro-1H-inden-4-yl]-3,5-dimethylphenoxy}-2-methylbutan-2-ol

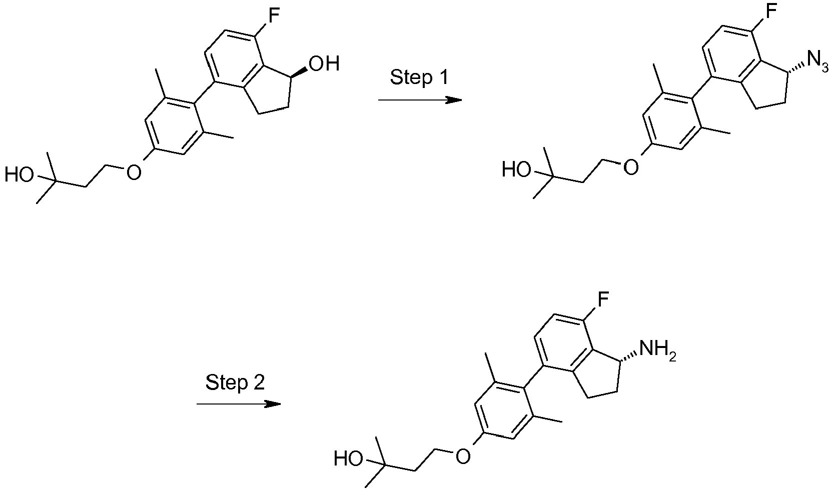

### Step 1: 4-{4-[(1R)-1-Azido-7-fluoro-2,3-dihydro-1H-inden-4-yl]-3,5-dimethylphenoxy}-2-methylbutan-2-ol

(1S)-7-Fluoro-4-[4-(3-hydroxy-3-methylbutoxy)-2,6-dimethylphenyl]-2,3-dihydro-1H-inden-1-ol (1.5 g) and 1,8-diazabicyclo[5.4.0]undec-7-en (DBU, 1.0 mL) are dissolved in toluene (15 mL) and cooled to 0°C. Diphenylphosphorylazide (1.0 mL) is added dropwise and the mixture is stirred for 12 hours while warming to room temperature. The mixture is washed with water, dried (MgSO₄) and the solvent is evaporated in vacuo. The residue is chromatographed on silica gel (petroleum ether/ethyl acetate 80:20→50:50) to give the title compound. Mass spectrum (ESI⁺): m/z = 384 [M+H]+.

### Step 2: 4-{4-[(1R)-1-Amino-7-fluoro-2,3-dihydro-1H-inden-4-yl]-3,5-dimethylphenoxy}-2-methylbutan-2-ol

A mixture of 4-{4-[(1R)-1-Azido-7-fluoro-2,3-dihydro-1H-inden-4-yl]-3,5-dimethylphenoxy}-2-methylbutan-2-ol (1.55 g) and 10% palladium on carbon (0.50 g) in ethanol (100 mL) is shaken under hydrogen atmosphere (3 bar) at room temperature for 6 hours. The catalyst is separated by filtration and the filtrate is concentrated. The residue is purified by HPLC (acetonitrile/water/ammonia) to give the title compound. Mass spectrum (ESI⁺): m/z = 358 [M+H]⁺.

The following intermediates may be obtained analogously to the procedure described for intermediate 4, starting from the corresponding indanol-derivative:

| Intermediate | Structure / Name | Mass spectrum (MS) |
|---|---|---|
| 5 | 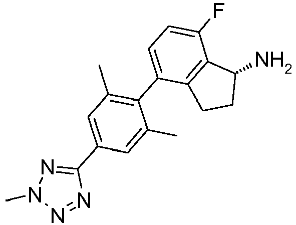 | MS (ESI⁺): m/z = 338 [M+H]⁺ |
| | (1R)-4-[2,6-Dimethyl-4-(2-methyl-2H-1,2,3,4-tetrazol-5-yl)phenyl]-7-fluoro-2,3-dihydro-1H-inden-1-amine | |
| 6 | 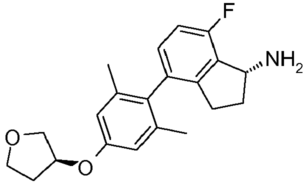 | |
| | (1R)-4-{2,6-Dimethyl-4-[(3S)-oxolan-3-yloxy]phenyl}-7-fluoro-2,3-dihydro-1H-inden-1-amine | |
| 7 | 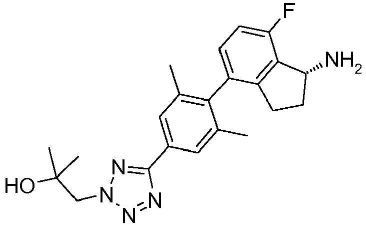 | |
| | 1-(5-{4-[(1 R)-1-Amino-7-fluoro-2,3-dihydro-1H-inden-4-yl]-3,5-dimethylphenyl}-2H-1,2,3,4-tetrazol-2-yl)-2-methylpropan-2-ol | |
| 8 | 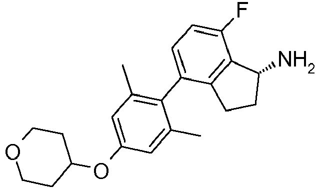 | |
| | (1R)-4-[2,6-Dimethyl-4-(oxan-4-yloxy)phenyl]-7-fluoro-2,3-dihydro-1 H-inden-1-amine | |
| 9 | 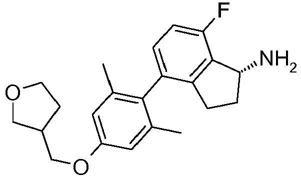 | |
| | (1R)-4-{2,6-Dimethyl-4-[(oxolan-3-yl)methoxy]phenyl}-7-fluoro-2,3-dihydro-1 H-inden-1-amine | |
| 10 | 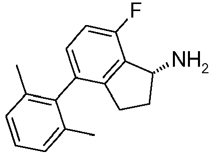 | |
| | (1R)-4-(2,6-Dimethylphenyl)-7-fluoro-2,3-dihydro-1H-inden-1-amine | |
| 11 | 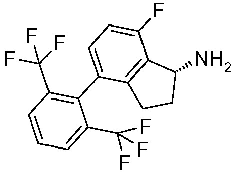 | |
| | (1R)-4-[2,6-Bis(trifluoromethyl)phenyl]-7-fluoro-2,3-dihydro-1 H-inden-1-amine | |
| 12 | 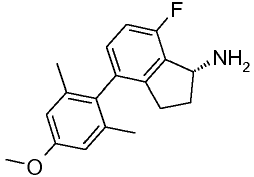 | |
| | (1R)-7-Fluoro-4-(4-methoxy-2,6-dimethylphenyl)-2,3-dihydro-1 H-inden-1-amine | |
| 13 | 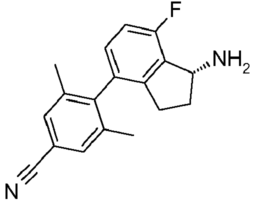 | |
| | 4-[(1R)-1-Amino-7-fluoro-2,3-dihydro-1H-inden-4-yl]-3,5-dimethylbenzonitrile | |
| 14 | 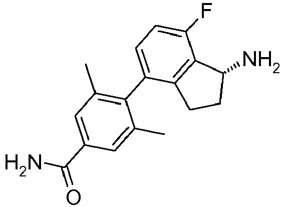 | |
| | 4-[(1R)-1-Amino-7-fluoro-2,3-dihydro-1H-inden-4-yl]-3,5-dimethylbenzamide | |
| 15 | 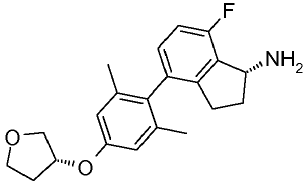 | |
| | (1R)-4-{2,6-Dimethyl-4-[(3R)-oxolan-3-yloxy]phenyl}-7-fluoro-2,3-dihydro-1 H-inden-1 -amine | |
| 16 | 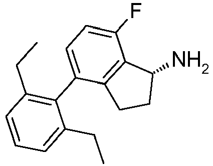 | |
| | (1R)-4-(2,6-Diethylphenyl)-7-fluoro-2,3-dihydro-1H-inden-1-amine | |
| 17 | 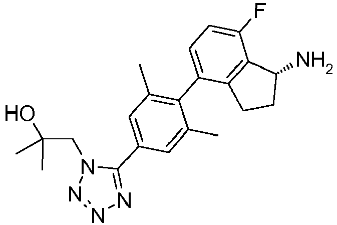 | |
| | 1-(5-{4-[(1R)-1-Amino-7-fluoro-2,3-dihydro-1H-inden-4-yl]-3,5-dimethylphenyl}-1H-1,2,3,4-tetrazol-1-yl)-2-methylpropan-2-ol | |
| 18 | 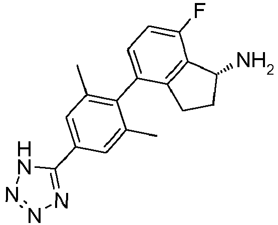 | |
| | (1R)-4-[2,6-Dimethyl-4-(1H-1,2,3,4-tetrazol-5-yl)phenyl]-7-fluoro-2,3-dihydro-1H-inden-1-amine | |
| 19 | 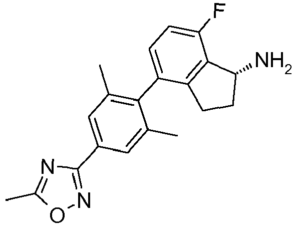 | |
| | (1R)-4-[2,6-Dimethyl-4-(5-methyl-1,2,4-oxadiazol-3-yl)phenyl]-7-fluoro-2,3-dihydro-1H-inden-1-amine | |
| 20 | 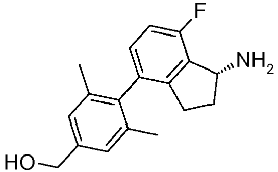 | |
| | {4-[(1R)-1-Amino-7-fluoro-2,3-dihydro-1H-inden-4-yl]-3,5-dimethylphenyl}methanol | |
| 21 | 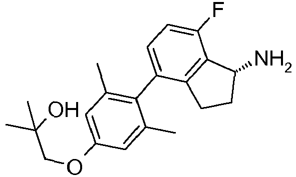 | |
| | 1-{4-[(1R)-1-Amino-7-fluoro-2,3-dihydro-1H-inden-4-yl]-3,5-dimethylphenoxy}-2-methylpropan-2-ol | |
| 22 | 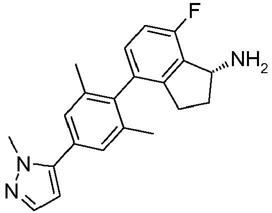 | |
| | (1R)-4-[2,6-Dimethyl-4-(1-methyl-1H-pyrazol-5-yl)phenyl]-7-fluoro-2,3-dihydro-1H-inden-1-amine | |
| 23 | 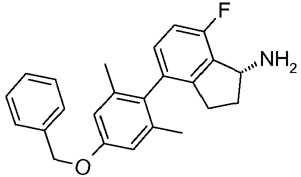 | |
| | (1R)-4-[4-(Benzyloxy)-2,6-dimethylphenyl]-7-fluoro-2,3-dihydro-1H-inden-1 -amine | |
| 24 | 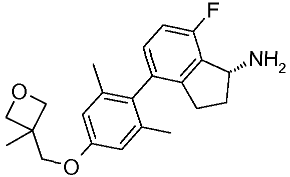 | |
| | (1R)-4-{2,6-Dimethyl-4-[(3-methyloxetan-3-yl)methoxy]phenyl}-7-fluoro-2,3-dihydro-1H-inden-1-amine | |
| 25 | 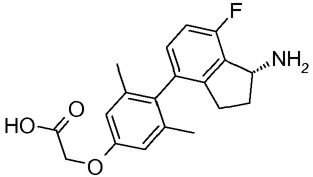 | |
| | 2-{4-[(1R)-1-Amino-7-fluoro-2,3-dihydro-1H-inden-4-yl]-3,5-dimethylphenoxy}acetic acid | |
| 26 | 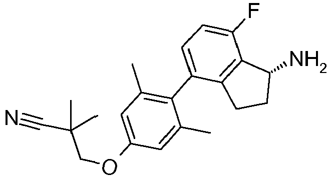 | |
| | 3-{4-[(1R)-1-Amino-7-fluoro-2,3-dihydro-1H-inden-4-yl]-3,5-dimethylphenoxy}-2,2-dimethylpropanenitrile | |
| 27 | 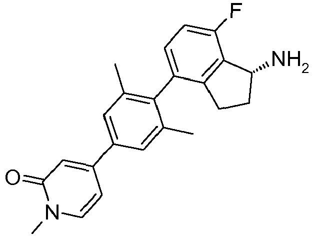 | |
| | 4-{4-[(1R)-1-Amino-7-fluoro-2,3-dihydro-1H-inden-4-yl]-3,5-dimethylphenyl}-1-methyl-1,2-dihydropyridin-2-one | |
| 28 | 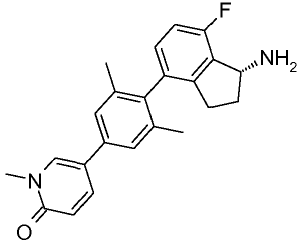 | |
| | 5-{4-[(1R)-1-Amino-7-fluoro-2,3-dihydro-1H-inden-4-yl]-3,5-dimethylphenyl}-1-methyl-1,2-dihydropyridin-2-one | |
| 29 | 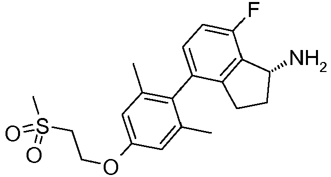 | |
| | (1R)-7-Fluoro-4-[4-(2-methanesulfonylethoxy)-2,6-dimethylphenyl]-2,3-dihydro-1H-inden-1-amine | |
| 30 | 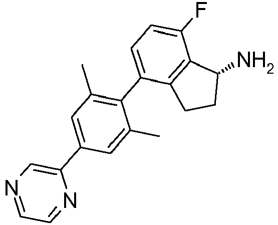 | |
| | (1R)-4-[2,6-Dimethyl-4-(pyrazin-2-yl)phenyl]-7-fluoro-2,3-dihydro-1H-inden-1-amine | |
| 31 | 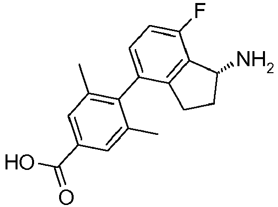 | |
| | 4-[(1R)-1-Amino-7-fluoro-2,3-dihydro-1H-inden-4-yl]-3,5-dimethylbenzoic acid | |
| 32 | 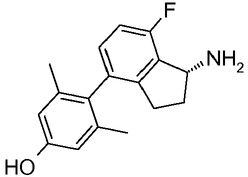 | |
| | 4-[(1R)-1-Amino-7-fluoro-2,3-dihydro-1H-inden-4-yl]-3,5-dimethylphenol | |
| 33 | 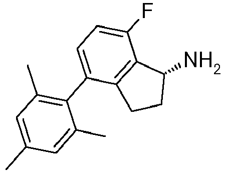 | |
| | (1R)-7-Fluoro-4-(2,4,6-trimethylphenyl)-2,3-dihydro-1H-inden-1-amine | |
| 34 | 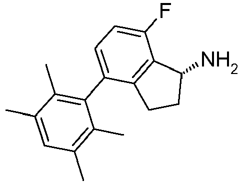 | |
| | (1R)-7-Fluoro-4-(2,3,5,6-tetramethylphenyl)-2,3-dihydro-1H-inden-1-amine | |
| 35 | 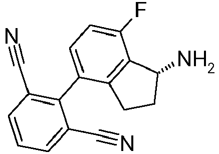 | |
| | 2-[(1R)-1-Amino-7-fluoro-2,3-dihydro-1H-inden-4-yl]benzene-1,3-dicarbonitrile | |
| 36 | 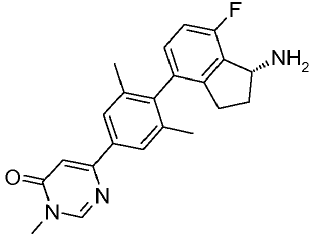 | |
| | 6-{4-[(1R)-1-Amino-7-fluoro-2,3-dihydro-1H-inden-4-yl]-3,5-dimethylphenyl}-3-methyl-3,4-dihydropyrimidin-4-one | |
| 37 | 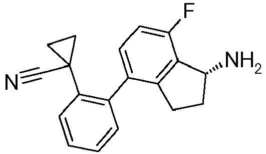 | |
| | 1-{2-[(1R)-1-Amino-7-fluoro-2,3-dihydro-1H-inden-4-yl]phenyl}cyclopropane-1-carbonitrile | |
| 38 | 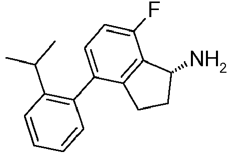 | |
| | (1R)-7-Fluoro-4-[2-(propan-2-yl)phenyl]-2,3-dihydro-1H-inden-1-amine | |
| 39 | 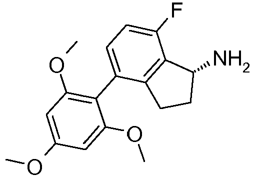 | |
| | (1R)-7-Fluoro-4-(2,4,6-trimethoxyphenyl)-2,3-dihydro-1H-inden-1-amine | |
| 40 | 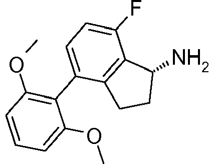 | |
| | (1R)-4-(2,6-Dimethoxyphenyl)-7-fluoro-2,3-dihydro-1H-inden-1-amine | |
| 41 | 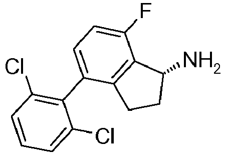 | |
| | (1R)-4-(2,6-Dichlorophenyl)-7-fluoro-2,3-dihydro-1H-inden-1-amine | |
| 42 | 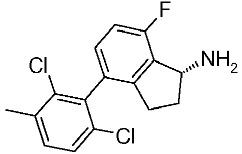 | |
| | (1R)-4-(2,6-Dichloro-3-methylphenyl)-7-fluoro-2,3-dihydro-1H-inden-1 -amine | |
| 43 | 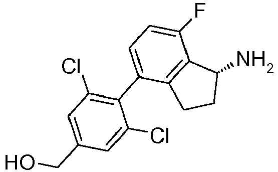 | |
| | {4-[(1R)-1-Amino-7-fluoro-2,3-dihydro-1H-inden-4-yl]-3,5-dichlorophenyl}methanol | |
| 44 | 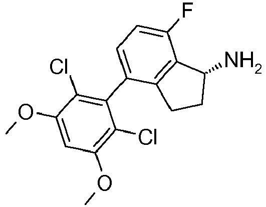 | |
| | (1R)-4-(2,6-Dichloro-3,5-dimethoxyphenyl)-7-fluoro-2,3-dihydro-1H-inden-1-amine | |
| 45 | 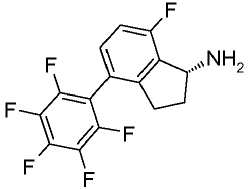 | |
| | (1R)-7-Fluoro-4-(pentafluorophenyl)-2,3-dihydro-1H-inden-1-amine | |
| 46 | 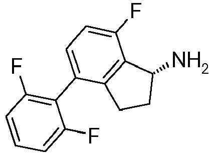 | |
| | (1R)-4-(2,6-Difluorophenyl)-7-fluoro-2,3-dihydro-1H-inden-1-amine | |
| 47 | 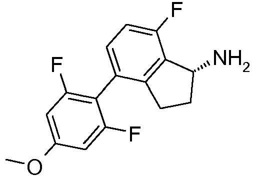 | |
| | (1R)-4-(2,6-Difluoro-4-methoxyphenyl)-7-fluoro-2,3-dihydro-1H-inden-1-amine | |
| 48 | 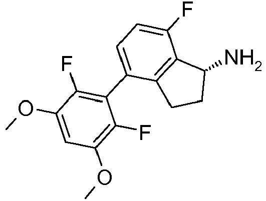 | |
| | (1R)-4-(2,6-Difluoro-3,5-dimethoxyphenyl)-7-fluoro-2,3-dihydro-1H-inden-1-amine | |
| 49 | 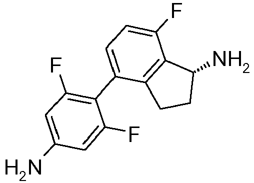 | |
| | (1 R)-4-(4-Amino-2,6-difluorophenyl)-7-fluoro-2,3-dihydro-1 H-inden-1-amine | |
| 50 | 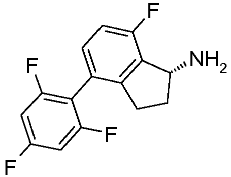 | |
| | (1R)-7-Fluoro-4-(2,4,6-trifluorophenyl)-2,3-dihydro-1H-inden-1-amine | |
| 51 | 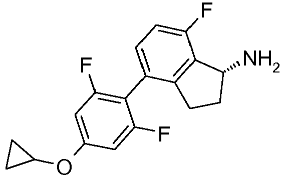 | |
| | (1R)-4-(4-Cyclopropoxy-2,6-difluorophenyl)-7-fluoro-2,3-dihydro-1H-inden-1-amine | |
| 52 | 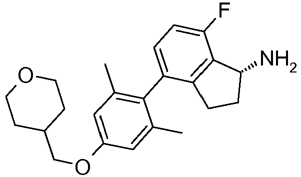 | |
| | (1R)-4-{2,6-Dimethyl-4-[(oxan-4-yl)methoxy]phenyl}-7-fluoro-2,3-dihydro-1H-inden-1-amine | |
| 53 | 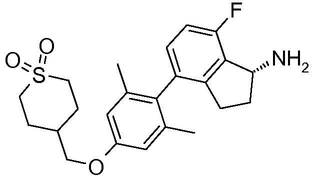 | |
| | 4-({4-[(1R)-1-Amino-7-fluoro-2,3-dihydro-1H-inden-4-yl]-3,5-dimethylphenoxy}methyl)-1λ⁶-thiane-1,1-dione | |
| 54 | 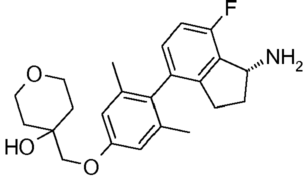 | |
| | 4-({4-[(1R)-1-Amino-7-fluoro-2,3-dihydro-1H-inden-4-yl]-3,5-dimethylphenoxy}methyl)oxan-4-ol | |
| 55 | 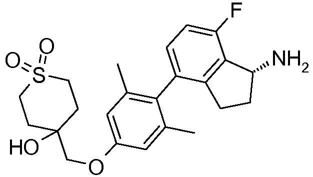 | |
| | 4-({4-[(1R)-1-Amino-7-fluoro-2,3-dihydro-1H-inden-4-yl]-3,5-dimethylphenoxy}methyl)-4-hydroxy-1λ⁶-thiane-1,1-dione | |
| 56 | 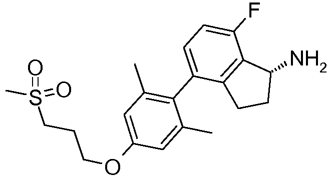 | |
| | (1R)-7-Fluoro-4-[4-(3-methanesulfonylpropoxy)-2,6-dimethylphenyl]-2,3-dihydro-1H-inden-1-amine | |
| 57 | 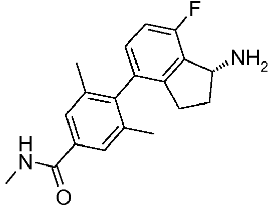 | |
| | 4-[(1R)-1-Amino-7-fluoro-2,3-dihydro-1H-inden-4-yl]-N,3,5-trimethylbenzamide | |
| 58 | 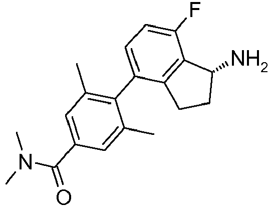 | |
| | 4-[(1R)-1-Amino-7-fluoro-2,3-dihydro-1H-inden-4-yl]-N,N,3,5-tetramethylbenzamide | |
| 59 | 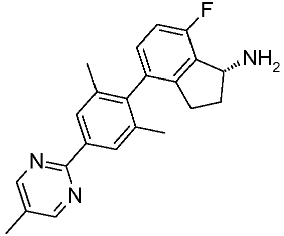 | |
| | (1R)-4-[2,6-Dimethyl-4-(5-methylpyrimidin-2-yl)phenyl]-7-fluoro-2,3-dihydro-1H-inden-1-amine | |
| 60 | 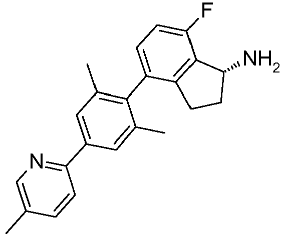 | |
| | (1R)-4-[2,6-Dimethyl-4-(5-methylpyridin-2-yl)phenyl]-7-fluoro-2,3-dihydro-1H-inden-1-amine | |
| 61 | 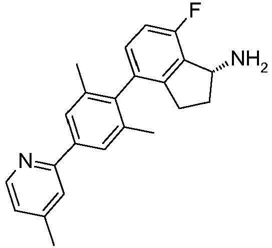 | |
| | (1R)-4-[2,6-Dimethyl-4-(4-methylpyridin-2-yl)phenyl]-7-fluoro-2,3-dihydro-1H-inden-1-amine | |

### Intermediate 62

### (1R)-4-[2,6-Dimethyl-4-(2-methyl-2H-1,2,3,4-tetrazol-5-yl)phenyl]-2,3-dihydro-1H-inden-1-amine

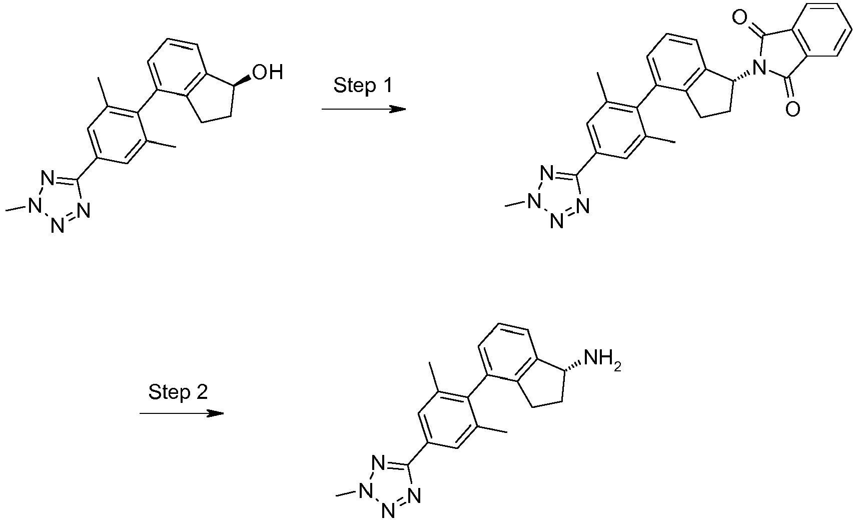

### Step 1: 2-[(1R)-4-[2,6-Dimethyl-4-(2-methyl-2H-1,2,3,4-tetrazol-5-yl)phenyl]-2,3-dihydro-1H-inden-1-yl]-2,3-dihydro-1H-isoindole-1 ,3-dione

Under an argon atmosphere (1S)-4-[2,6-dimethyl-4-(2-methyl-2H-1,2,3,4-tetrazol-5-yl)phenyl]-2,3-dihydro-1H-inden-1-ol (934 mg), 2,3-dihydro-1H-isoindole-1,3-dione (phthalimide, 515 mg) and PBu₃ (1 mL) are dissolved in tetrahydrofurane (20 mL) and cooled to 0°C. Di-tert.-butyl-azodicarboxylate (910 mg) is added portionwise and the mixture is stirred for 3 days while warming to room temperature. The mixture is then partitioned between saturated aqueous NaHCO₃ solution and ethylacetate. The organic phase is washed with brine and dried (MgSO₄). The solvents are evaporated in vacuo and the residue is chromatographed on silica gel (petroleum ether/ethyl acetate 70:30→50:50) to give the title compound. Mass spectrum (ESI⁺): m/z = 450 [M+H]⁺.

### Step 2: (1R)-4-[2,6-Dimethyl-4-(2-methyl-2H-1,2,3,4-tetrazol-5-yl)phenyl]-2,3-dihydro-1H-inden-1-amine

A mixture of 2-[(1R)-4-[2,6-dimethyl-4-(2-methyl-2H-1,2,3,4-tetrazol-5-yl)phenyl]-2,3-dihydro-1H-inden-1-yl]-2,3-dihydro-1H-isoindole-1,3-dione (838 mg) and hydrazine hydrate (100 µL) in methanol (12 mL) is stirred for 2 days at room temperature. Hydrazine hydrate (100 µL) is added and the mixture is stirred for 1 day at room temperature. Then the mixture is diluted with ethylacetate (70 mL), the precipitate is filtered off, the residue is concentrated in vacuo and purified by chromatographed on Al₂O₃ (dichloromethane/methanol 95:5→80:20) to give the title compound. Mass spectrum (ESI⁺): m/z = 320 [M+H]⁺.

The following intermediates may be obtained analogously to the procedure described for intermediate 62, starting from the corresponding indanol-derivative:

| Intermediate | Structure / Name | Mass spectrum (MS) |
|---|---|---|
| 63 | 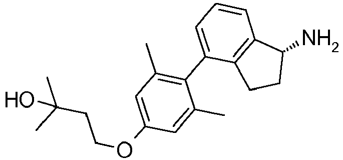 | MS (ESI⁺): m/z = 340 [M+H]⁺ |
| | 4-{4-[(1R)-1-Amino-2,3-dihydro-1H-inden-4-yl]-3,5-dimethylphenoxy}-2-methylbutan-2-ol | |
| 64 | 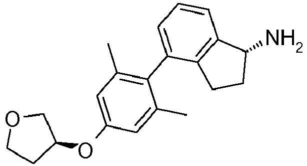 | |
| | (1R)-4-{2,6- Dimethyl-4-[(3S)-oxolan-3-yloxy] phenyl}-2,3-dihydro-1H-inden-1-amine | |
| 65 | 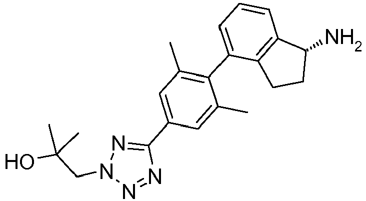 | |
| | 1-(5-{4-[(1R)-1-Amino-2,3-dihydro-1H-inden-4-yl]-3,5-dimethylphenyl}-2H-1,2,3,4-tetrazol-2-yl)-2-methylpropan-2-ol | |
| 66 | 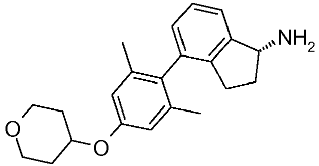 | |
| | (1R)-4-[2,6-Dimethyl-4-(oxan-4-yloxy)phenyl]-2,3-dihydro-1H-inden-1-amine | |
| 67 | 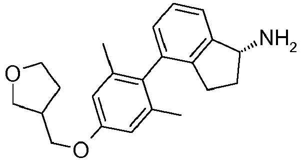 | |
| | (1R)-4-{2,6-Dimethyl-4-[(oxolan-3-yl)methoxy]phenyl}-2,3-dihydro-1H-inden-1-amine | |
| 68 | 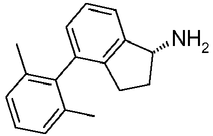 | |
| | (1R)-4-(2,6-Dimethylphenyl)-2,3-dihydro-1H-inden-1-amine | |
| 69 | 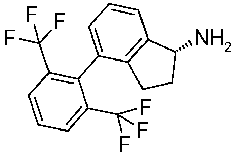 | |
| | (1R)-4-[2,6-Bis(trifluoromethyl)phenyl]-2,3-dihydro-1H-inden-1-amine | |
| 70 | 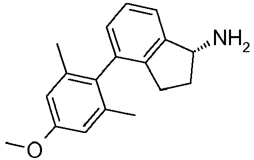 | |
| | (1R)-4-(4-Methoxy-2,6-dimethylphenyl)-2,3-dihydro-1H-inden-1-amine | |
| 71 | 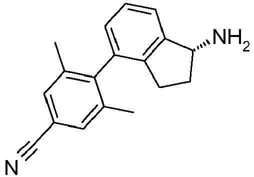 | |
| | 4-[(1R)-1-Amino-2,3-dihydro-1H-inden-4-yl]-3,5-dimethylbenzonitrile | |
| 72 | 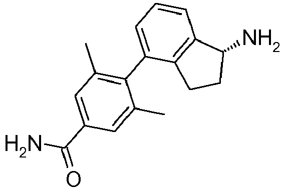 | |
| | 4-[(1R)-1-Amino-2,3-dihydro-1H-inden-4-yl]-3,5-dimethylbenzamide | |
| 73 | 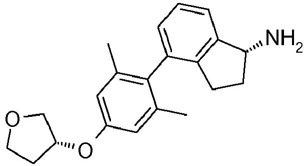 | |
| | (1R)-4-{2,6-Dimethyl-4-[(3R)-oxolan-3-yloxy]phenyl}-2,3-dihydro-1H-inden-1-amine | |
| 74 | 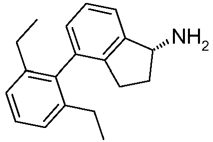 | |
| | (1R)-4-(2,6-Diethylphenyl)-2,3-dihydro-1H-inden-1-amine | |
| 75 | 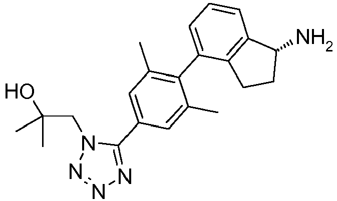 | |
| | 1-(5-{4-[(1R)-1-Amino-2,3-dihydro-1H-inden-4-yl]-3,5-dimethylphenyl}-1H-1,2,3,4-tetrazol-1-yl)-2-methylpropan-2-ol | |
| 76 | 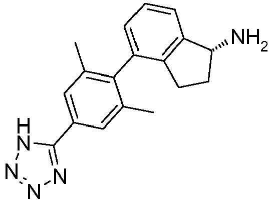 | |
| | (1R)-4-[2,6-Dimethyl-4-(1H-1,2,3,4-tetrazol-5-yl)phenyl]-2,3-dihydro-1H-inden-1-amine | |
| 77 | 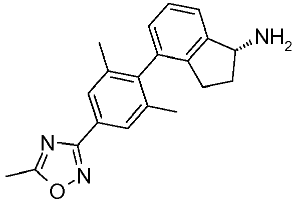 | |
| | (1R)-4-[2,6-Dimethyl-4-(5-methyl-1,2,4-oxadiazol-3-yl)phenyl]-2,3-dihydro-1H-inden-1-amine | |
| 78 | 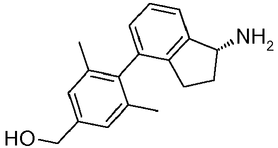 | |
| | {4-[(1R)-1-Amino-2,3-dihydro-1H-inden-4-yl]-3,5-dimethylphenyl}methanol | |
| 79 | 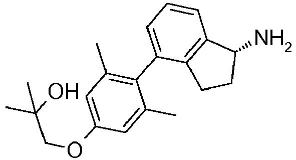 | |
| | 1-{4-[(1R)-1-Amino-2,3-dihydro-1H-inden-4-yl]-3,5-dimethylphenoxy}-2-methylpropan-2-ol | |
| 80 | 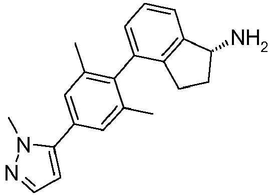 | |
| | (1R)-4-[2,6-Dimethyl-4-(1-methyl-1H-pyrazol-5-yl)phenyl]-2,3-dihydro-1H-inden-1-amine | |
| 81 | 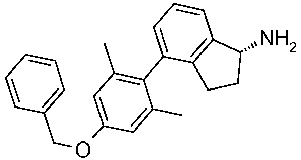 | |
| | (1R)-4-[4-(Benzyloxy)-2,6-dimethylphenyl]-2,3-dihydro-1H-inden-1-amine | |
| 82 | 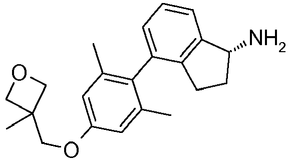 | |
| | (1R)-4-{2,6-Dimethyl-4-[(3-methyloxetan-3-yl)methoxy]phenyl}-2,3-dihydro-1H-inden-1-amine | |
| 83 | 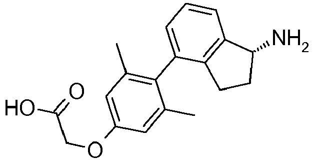 | |
| | 2-{4-[(1R)-1-Amino-2,3-dihydro-1H-inden-4-yl]-3,5-dimethylphenoxy}acetic acid | |
| 84 | 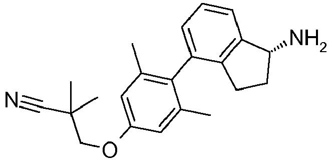 | |
| | 3-{4-[(1R)-1-Amino-2,3-dihydro-1H-inden-4-yl]-3,5-dimethylphenoxy}-2,2-dimethylpropanenitrile | |
| 85 | 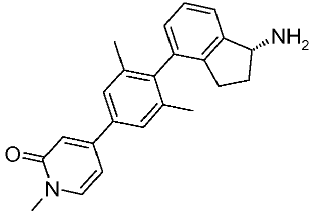 | |
| | 4-{4-[(1R)-1-Amino-2,3-dihydro-1H-inden-4-yl]-3,5-dimethylphenyl}-1-methyl-1,2-dihydropyridin-2-one | |
| 86 | 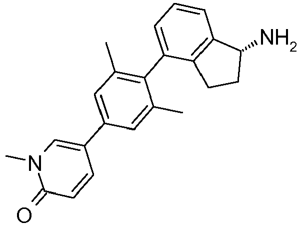 | |
| | 5-{4-[(1R)-1-Amino-2,3-dihydro-1H-inden-4-yl]-3,5-dimethylphenyl}-1-methyl-1,2-dihydropyridin-2-one | |
| 87 | 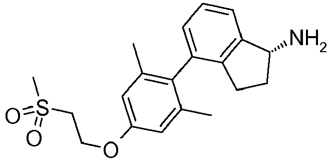 | |
| | (1R)-4-[4-(2-Methanesulfonylethoxy)-2,6-dimethylphenyl]-2,3-dihydro-1H-inden-1-amine | |
| 88 | 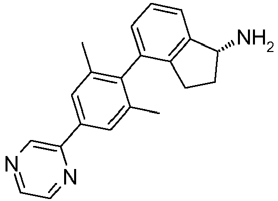 | |
| | (1R)-4-[2,6-Dimethyl-4-(pyrazin-2-yl)phenyl]-2,3-dihydro-1H-inden-1-amine | |
| 89 | 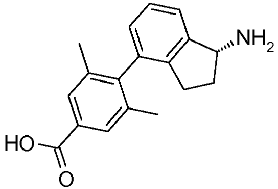 | |
| | 4-[(1R)-1-Amino-2,3-dihydro-1H-inden-4-yl]-3,5-dimethylbenzoic acid | |
| 90 | 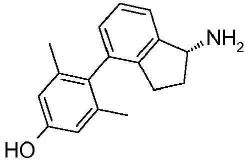 | |
| | 4-[(1R)-1-Amino-2,3-dihydro-1H-inden-4-yl]-3,5-dimethylphenol | |
| 91 | 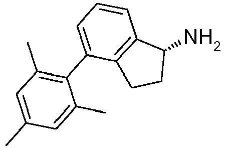 | |
| | (1R)-4-(2,4,6-Trimethylphenyl)-2,3-dihydro-1H-inden-1-amine | |
| 92 | 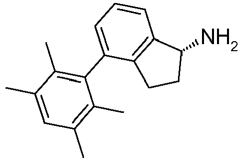 | |
| | (1R)-4-(2,3,5,6-Tetramethylphenyl)-2,3-dihydro-1H-inden-1-amine | |
| 93 | 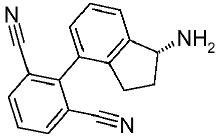 | |
| | 2-[(1R)-1-Amino-2,3-dihydro-1H-inden-4-yl]benzene-1,3-dicarbonitrile | |
| 94 | 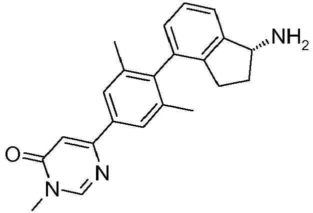 | |
| | 6-{4-[(1R)-1-Amino-2,3-dihydro-1H-inden-4-yl]-3,5-dimethylphenyl}-3-methyl-3,4-dihydropyrimidin-4-one | |
| 95 | 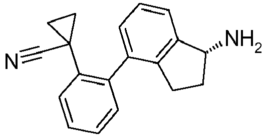 | |
| | 1-{2-[(1R)-1 -Amino-2,3-dihydro-1H-inden-4-yl]phenyl}cyclopropane-1-carbonitrile | |
| 96 | 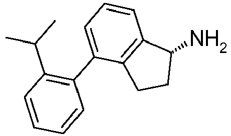 | |
| | (1R)-4-[2-(Propan-2-yl)phenyl]-2,3-dihydro-1H-inden-1-amine | |
| 97 | 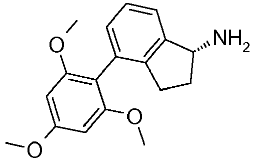 | |
| | (1R)-4-(2,4,6-Trimethoxyphenyl)-2,3-dihydro-1H-inden-1-amine | |
| 98 | 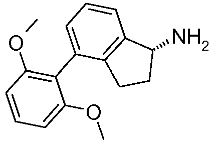 | |
| | (1R)-4-(2,6-Dimethoxyphenyl)-2,3-dihydro-1H-inden-1-amine | |
| 99 | 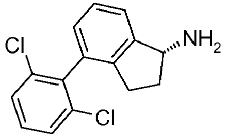 | |
| | (1R)-4-(2,6-Dichlorophenyl)-2,3-dihydro-1H-inden-1-amine | |
| 100 | 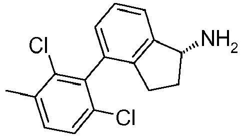 | |
| | (1R)-4-(2,6-Dichloro-3-methylphenyl)-2,3-dihydro-1H-inden-1-amine | |
| 101 | 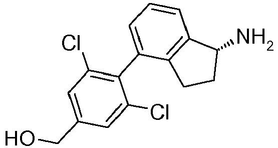 | |
| | {4-[(1R)-1-Amino-2,3-dihydro-1H-inden-4-yl]-3,5-dichlorophenyl}methanol | |
| 102 | 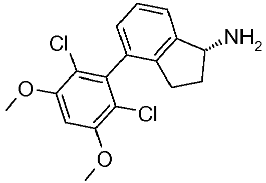 | |
| | (1R)-4-(2,6-Dichloro-3,5-dimethoxyphenyl)-2,3-dihydro-1H-inden-1-amine | |
| 103 | 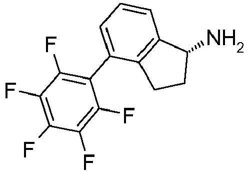 | |
| | (1R)-4-(Pentafluorophenyl)-2,3-dihydro-1H-inden-1-amine | |
| 104 | 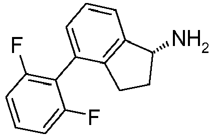 | |
| | (1R)-4-(2,6-Difluorophenyl)-2,3-dihydro-1H-inden-1-amine | |
| 105 | 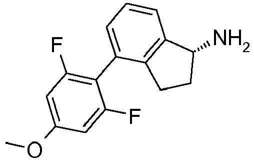 | |
| | (1R)-4-(2,6- Difluoro-4-methoxyphenyl)-2,3-dihydro-1H-inden-1-amine | |
| 106 | 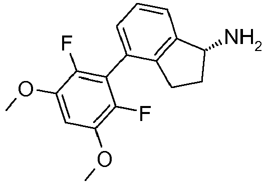 | |
| | (1R)-4-(2,6-Difluoro-3,5-dimethoxyphenyl)-2,3-dihydro-1H-inden-1-amine | |
| 107 | 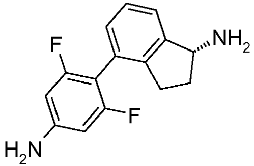 | |
| | (1R)-4-(4-Amino-2,6-difluorophenyl)-2,3-dihydro-1H-inden-1-amine | |
| 108 | 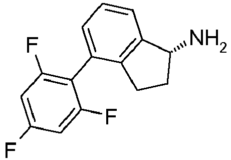 | |
| | (1R)-4-(2,4,6-Trifluorophenyl)-2,3-dihydro-1H-inden-1-amine | |
| 109 | 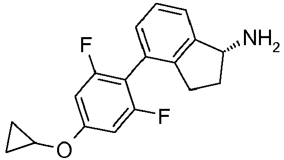 | |
| | (1R)-4-(4-Cyclopropoxy-2,6-difluorophenyl)-2,3-dihydro-1H-inden-1-amine | |
| 110 | 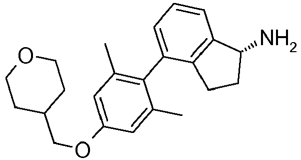 | |
| | (1R)-4-{2,6-Dimethyl-4-[(oxan-4-yl)methoxy]phenyl}-2,3-dihydro-1H-inden-1-amine | |
| 111 | 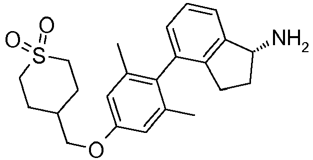 | |
| | 4-({4-[(1R)-1-Amino-2,3-dihydro-1H-inden-4-yl]-3,5-dimethylphenoxy}methyl)-1λ⁶-thiane-1,1-dione | |
| 112 | 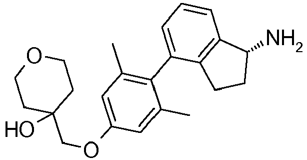 | |
| | 4-({4-[(1R)-1 -Amino-2,3-dihydro-1H-inden-4-yl]-3,5-dimethylphenoxy}methyl)oxan-4-ol | |
| 113 | 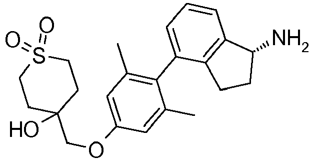 | |
| | 4-({4-[(1R)-1-Amino-2,3-dihydro-1H-inden-4-yl]-3,5 dimethylphenoxy}methyl)-4-hydroxy-1 λ⁶-thiane-1,1-dione | |
| 114 | 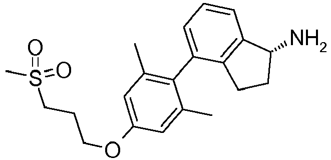 | |
| | (1R)-4-[4-(3-Methanesulfonylpropoxy)-2,6-dimethylphenyl]-2,3-dihydro-1H-inden-1-amine | |
| 115 | 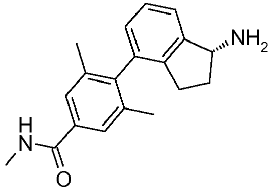 | |
| | 4-[(1R)-1-Amino-2,3-dihydro-1H-inden-4-yl]-N,3,5-trimethylbenzamide | |
| 116 | 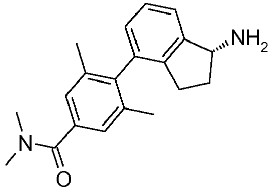 | |
| | 4-[(1R)-1-Amino-2,3-dihydro-1H-inden-4-yl]-N,N,3,5-tetramethylbenzamide | |
| 117 | 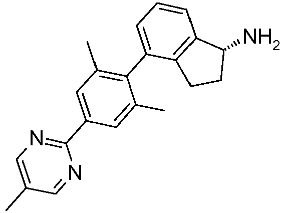 | |
| | (1R)-4-[2,6-Dimethyl-4-(5-methylpyrimidin-2-yl)phenyl]-2,3-dihydro-1H-inden-1-amine | |
| 118 | 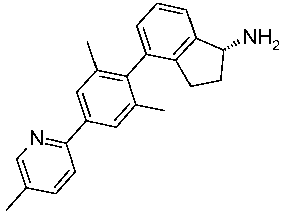 | |
| | (1R)-4-[2,6-Dimethyl-4-(5-methylpyridin-2-yl)phenyl]-2,3-dihydro-1H-inden-1-amine | |
| 119 | 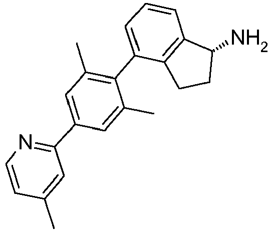 | |
| | (1R)-4-[2,6-Dimethyl-4-(4-methylpyridin-2-yl)phenyl]-2,3-dihydro-1H-inden-1-amine | |

### Intermediate 120

### Methyl 2-(6-{[(1R)-4-(tetramethyl-1,3,2-dioxaborolan-2-yl)-2,3-dihydro-1H-inden-1-yl]amino}-2H,3H-furo[3,2-b]pyridin-3-yl)acetate and tert-Butyl 2-(6-H{[(1R)-4-(tetramethyl-1,3,2-dioxaborolan-2-yl)-2,3-dihydro-1H-inden-1-yl]amino}-2H,3H-furo[3,2-b]pyridin-3-yl)acetate

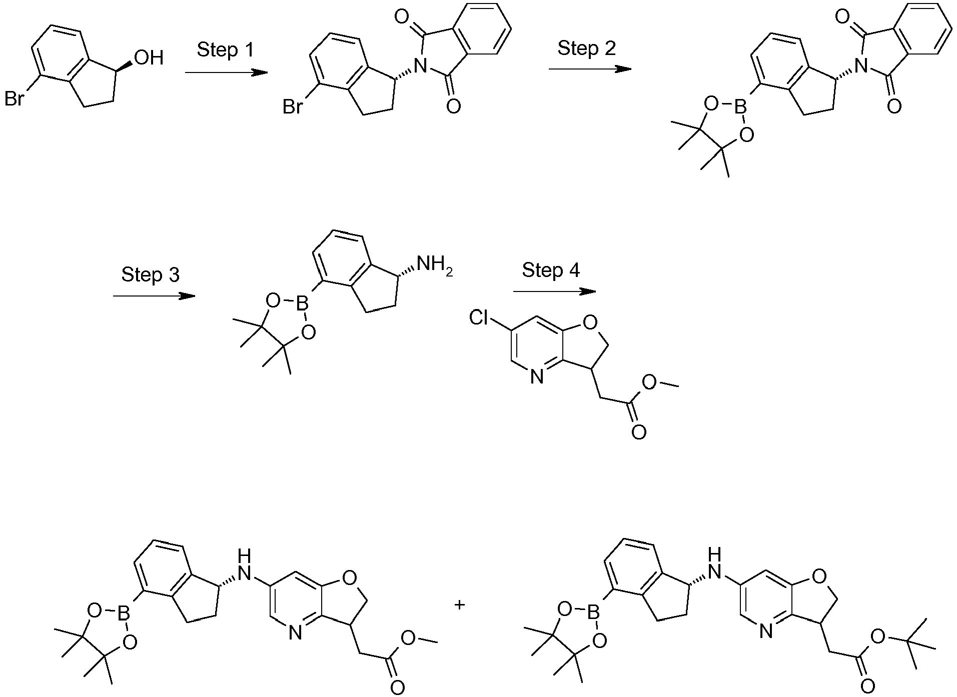

### Step 1: 2-[(1R)-4-Bromo-2,3-dihydro-1H-inden-1-yl]-2,3-dihydro-1H-isoindole-1,3-dione

To a solution of (1S)-4-bromo-2,3-dihydro-1H-inden-1-ol (0.5 g), phthalimide (0.35 g) and trophenylphosphine (0.62 g) in tetrahydrofurane (10 mL) is added dropwise under an argon atmosphere a solution of di-tert.-butyl-azodicarboxylate (0.54 g) in tetrahydrofurane (3 mL). Then the mixture is heated for 12 hours to 60°C. The mixture is diluted with ethylacetate, washed with saturated aqueous NaHCO₃ solution and dried (MgSO₄). The solvents are evaporated in vacuo and the residue is chromatographed on silica gel (petroleum ether/ethyl acetate 90:10→50:50) to give the title compound. Mass spectrum (ESI⁺): m/z = 342 [M+H]⁺.

### Step 2: 2-[(1R)-4-(Tetramethyl-1,3,2-dioxaborolan-2-yl)-2,3-dihydro-1H-inden-1-yl]-2,3-dihydro-1H-isoindole-1,3-dione

2-[(1R)-4-Bromo-2,3-dihydro-1H-inden-1-yl]-2,3-dihydro-1H-isoindole-1,3-dione (370 mg), bis(pinacolato)diboron (300 mg) and potassium acetate (170 mg) are dissolved in 1,4-dioxane (10 mL). The mixture is purged for 5 minutes with argon. 1,1'-bis(diphenylphosphino)ferrocenedichloropalladium(II) (40 mg) is added and the mixture is stirred for 4 hours at 100°C. Then the mixture is diluted with ethylacetate and washed successively with saturated aqueous NH₄Cl solution and brine. After drying (MgSO₄) the solvents are evaporated in vacuo and the residue is chromatographed on silica gel (petroleum ether/ethyl acetate 90:10→50:50) to give the title compound. Mass spectrum (ESI⁺): m/z = 390 [M+H]⁺.

### Step 3: (1R)-4-(Tetramethyl-1,3,2-dioxaborolan-2-yl)-2,3-dihydro-1H-inden-1-amine

Hydrazine hydrate (2.5 mL) is added to a solution of 2-[(1R)-4-(tetramethyl-1,3,2-dioxaborolan-2-yl)-2,3-dihydro-1H-inden-1-yl]-2,3-dihydro-1H-isoindole-1,3-dione (10 g) in methanol (50 mL) and then the mixture is stirred for 12 hours at room temperature. The mixture is diluted with ethylacetate and the precipitate is removed by filtration. The solvents are evaporated in vacuo and the residue is chromatographed on aluminium oxide (dichloromethane/methanol 99:1→90:10) to give the title compound. Mass spectrum (ESI⁺): m/z = 243 [M+H-NH₃]⁺.

### Step 4: Methyl 2-(6-{[(1R)-4-(tetramethyl-1,3,2-dioxaborolan-2-yl)-2,3-dihydro-1H-inden-1-yl]amino}-2H,3H-furo[3,2-b]pyridin-3-yl)acetate and tert-Butyl 2-(6-{[(1R)-4-(tetramethyl-1,3,2-dioxaborolan-2-yl)-2,3-dihydro-1H-inden-1-yl]amino}-2H,3H-furo[3,2-b]pyridin-3-yl)acetate

A microwave vial charged with a stir bar, (1R)-4-(tetramethyl-1,3,2-dioxaborolan-2-yl)-2,3-dihydro-1H-inden-1-amine (1 g), methyl 2-{6-chloro-2H,3H-furo[3,2-b]pyridin-3-yl}acetate (1.05 g), NaOtBu (930 mg) and 1,4-dioxane (15 mL) is purged with argon for 10 min. Chloro[2-(dicyclohexylphosphino)-3,6-dimethoxy-2',4',6'-triisopropyl-1,1'-biphenyl][2-(2-aminoethyl)phenyl]palladium(II)xMe-O-tBu (BrettPhos Pd G1 Methyl-t-butylether adduct, 155 mg) is added, the vial is sealed, and the mixture is stirred at 100 °C for 5 min. After cooling to room temperature, the mixture is diluted with ethylacetate, washed with saturated aqueous NH₄Cl solution and dried (MgSO₄). The solvents are evaporated in vacuo and the residue is chromatographed on silica gel (cyclohexane/ethyl acetate 99:1→70:30) to give the title compounds. Methyl 2-(6-{[(1R)-4-(tetramethyl-1,3,2-dioxaborolan-2-yl)-2,3-dihydro-1H-inden-1-yl]amino}-2H,3H-furo[3,2-b]pyridin-3-yl)acetate: Mass spectrum (ESI⁺): m/z = 451 [M+H]⁺.

tert-Butyl 2-(6-{[(1R)-4-(tetramethyl-1,3,2-dioxaborolan-2-yl)-2,3-dihydro-1H-inden-1-yl]amino}-2H,3H-furo[3,2-b]pyridin-3-yl)acetate: Mass spectrum (ESI⁺): m/z = 493 [M+H]⁺.

### Example 1

### 2-(6-{[(1R)-7-Fluoro-4-[4-(3-hydroxy-3-methylbutoxy)-2,6-dimethylphenyl]-2,3-dihydro-1H-inden-1-yl]amino}-2H,3H-furo[3,2-b]pyridin-3-yl)acetic acid

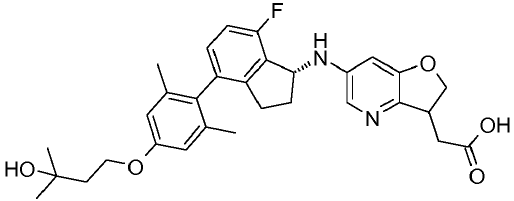

A microwave vial charged with a stir bar, 4-{4-[(1R)-1-amino-7-fluoro-2,3-dihydro-1H-inden-4-yl]-3,5-dimethylphenoxy}-2-methylbutan-2-ol (185 mg), methyl 2-{6-chloro-2H,3H-furo[3,2-b]pyridin-3-yl}acetate (140 mg), NaOtBu (300 mg) and 1,4-dioxane (5 mL) is purged with argon for 10 min. Chloro[2-(dicyclohexylphosphino)-3,6-dimethoxy-2',4',6'-triisopropyl-1,1'-biphenyl][2-(2-aminoethyl)phenyl]palladium(II)xMe-O-tBu (BrettPhos Pd G1 Methyl-t-butylether adduct, 40 mg) is added, the vial is sealed, and the mixture is stirred at 100 °C for 30 min. After cooling to room temperature, the mixture is concentrated and the residue is purified by HPLC (acetonitrile/water/trifluoroacetic acid) to give the title compound. LC (method 1): t_{R} = 0.91 min; Mass spectrum (ESI⁺): m/z = 535 [M+H]⁺.

### Example 2

### 2-[(3S)-6-{[(1R)-7-Fluoro-4-[4-(3-hydroxy-3-methylbutoxy)-2,6-dimethylphenyl]-2,3-dihydro-1H-inden-1-yl]amino}-2H,3H-furo[3,2-b]pyridin-3-yl]aceticacid

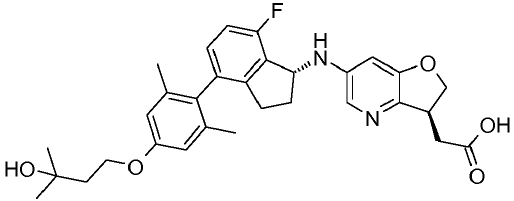

2-(6-{[(1R)-7-Fluoro-4-[4-(3-hydroxy-3-methylbutoxy)-2,6-dimethylphenyl]-2,3-dihydro-1H-inden-1-yl]amino}-2H,3H-furo[3,2-b]pyridin-3-yl)acetic acid is separated into the diastereomers by SFC on chiral phase (column: Phenomenex Inc. LUX® Cellulose 2, 5 µm, 250 mm x 10 mm; eluent: scCO₂/(ethanol 20 mM NH₃) 70:30, 60 mL/min, 40°C, backpressure 120 bar).

2-[(3S)-6-{[(1R)-7-Fluoro-4-[4-(3-hydroxy-3-methylbutoxy)-2,6-dimethylphenyl]-2,3-dihydro-1H-inden-1-yl]amino}-2H,3H-furo[3,2-b]pyridin-3-yl]acetic acid: LC (method 1b): t_{R} = 3.78 min.

Alternatively the title compound is prepared from 4-{4-[(1R)-1-amino-7-fluoro-2,3-dihydro-1H-inden-4-yl]-3,5-dimethylphenoxy}-2-methylbutan-2-ol and methyl 2-[(3S)-6-chloro-2H,3H-furo[3,2-b]pyridin-3-yl]acetate following a procedure analogous to that described for Example 1. LC (method 3): t_{R} = 0.79 min; Mass spectrum (ESI⁺): m/z = 535 [M+H]⁺.

### Example 3

### 2-[(3S)-6-{[(1R)-4-[2,6-Dimethyl-4-(2-methyl-2H-1,2,3,4-tetrazol-5-yl)phenyl]-2,3-dihydro-1H-inden-1-yl]amino}-2H,3H-furo[3,2-b]pyridin-3-yl]aceticacid

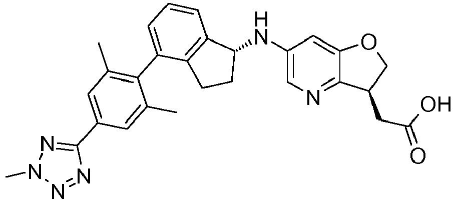

The title compound is prepared from (1R)-4-[2,6-dimethyl-4-(2-methyl-2H-1,2,3,4-tetrazol-5-yl)phenyl]-2,3-dihydro-1H-inden-1-amine and methyl 2-[(3S)-6-chloro-2H,3H-furo[3,2-b]pyridin-3-yl]acetate following a procedure analogous to that described for Example 1. LC (method 2): t_{R} = 0.91 min; Mass spectrum (ESI⁺): m/z = 497 [M+H]⁺.

### Example 4

### 2-[(3S)-6-{[(1R)-4-[4-(3-Hydroxy-3-methylbutoxy)-2,6-dimethylphenyl]-2,3-dihydro-1H-inden-1-yl]amino}-2H,3H-furo[3,2-b]pyridin-3-yl]acetic acid

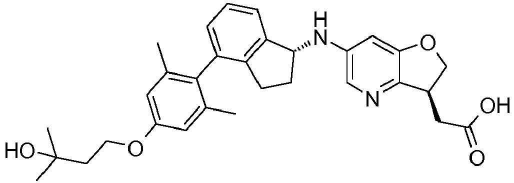

The title compound is prepared from 4-{4-[(1R)-1-amino-2,3-dihydro-1H-inden-4-yl]-3,5-dimethylphenoxy}-2-methylbutan-2-ol and methyl 2-[(3S)-6-chloro-2H,3H-furo[3,2-b]pyridin-3-yl]acetate following a procedure analogous to that described for Example 1. LC (method 2): t_{R} = 0.92 min; Mass spectrum (ESI⁺): m/z = 517 [M+H]⁺.

### Example 5

### 2-[(3S)-6-{[(1R)-4-[2,6-Dimethyl-4-(2-methyl-2H-1,2,3,4-tetrazol-5-yl)phenyl]-7-fluoro-2,3-dihydro-1H-inden-1-yl]amino}-2H,3H-furo[3,2-b]pyridin-3-yl]aceticacid

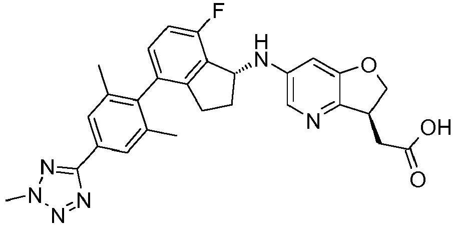

The title compound is prepared from (1R)-4-[2,6-dimethyl-4-(2-methyl-2H-1,2,3,4-tetrazol-5-yl)phenyl]-7-fluoro-2,3-dihydro-1H-inden-1-amine and methyl 2-[(3S)-6-chloro-2H,3H-furo[3,2-b]pyridin-3-yl]acetate following a procedure analogous to that described for Example 1. LC (method 2): t_{R} = 0.90 min; Mass spectrum (ESI⁺): m/z = 515 [M+H]⁺.

The following examples may be obtained analogously to the procedure described for example 1, starting from methyl 2-[(3S)-6-chloro-2H,3H-furo[3,2-b]pyridin-3-yl]acetate and the corresponding indanylamin-intermediate:

| Example | Used indanylaminintermediate | 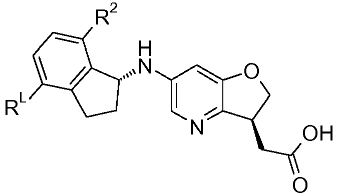 | |
|---|---|---|---|
| | | R^{L} | R² |
| 6 | 6 | 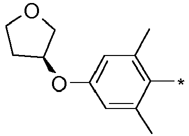 | F |
| 7 | 64 | | H |
| 8 | 7 | 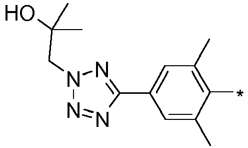 | F |
| 9 | 65 | | H |
| 10 | 8 | 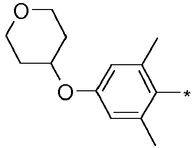 | F |
| 11 | 66 | | H |
| 12 | 9 | 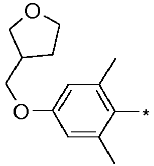 | F |
| 13 | 67 | | H |
| 14 | 10 | 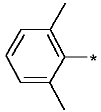 | F |
| 15 | 68 | | H |
| 16 | 11 | 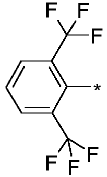 | F |
| 17 | 69 | | H |
| 18 | 12 | 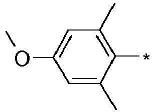 | F |
| 19 | 70 | | H |
| 20 | 13 | 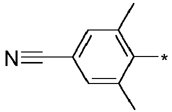 | F |
| 21 | 71 | | H |
| 22 | 14 | 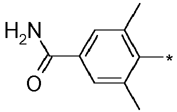 | F |
| 23 | 72 | | H |
| 24 | 15 | 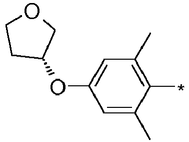 | F |
| 25 | 73 | | H |
| 26 | 16 | 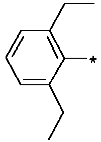 | F |
| 27 | 74 | | H |
| 28 | 17 | 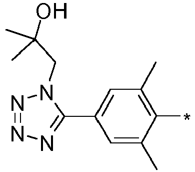 | F |
| 29 | 75 | | H |
| 30 | 18 | 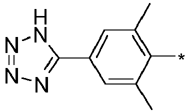 | F |
| 31 | 76 | | H |
| 32 | 19 | 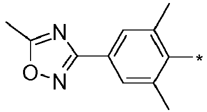 | F |
| 33 | 77 | | H |
| 34 | 20 | 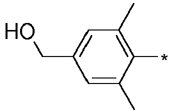 | F |
| 35 | 78 | | H |
| 36 | 21 | 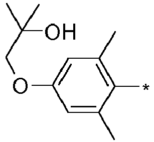 | F |
| 37 | 79 | | H |
| 38 | 22 | 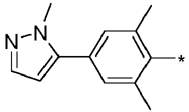 | F |
| 39 | 80 | | H |
| 40 | 23 | 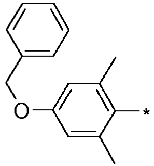 | F |
| 41 | 81 | | H |
| 42 | 24 | 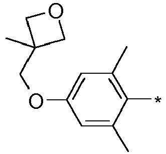 | F |
| 43 | 82 | | H |
| 44 | 25 | 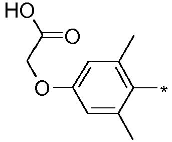 | F |
| 45 | 83 | | H |
| 46 | 26 | 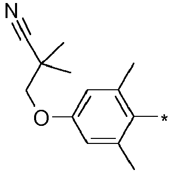 | F |
| 47 | 84 | | H |
| 48 | 27 | 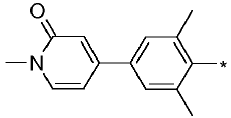 | F |
| 49 | 85 | | H |
| 50 | 28 | 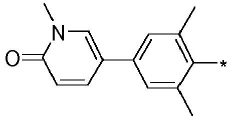 | F |
| 51 | 86 | | H |
| 52 | 29 | 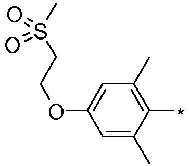 | F |
| 53 | 87 | | H |
| 54 | 30 | 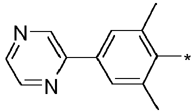 | F |
| 55 | 88 | | H |
| 56 | 31 | 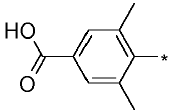 | F |
| 57 | 89 | | H |
| 58 | 32 | 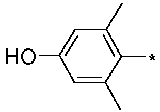 | F |
| 59 | 90 | | H |
| 60 | 33 | 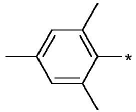 | F |
| 61 | 91 | | H |
| 62 | 34 | 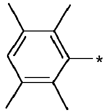 | F |
| 63 | 92 | | H |
| 64 | 35 | 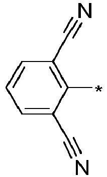 | F |
| 65 | 93 | | H |
| 66 | 36 | 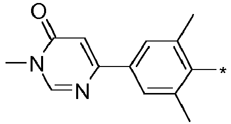 | F |
| 67 | 94 | | H |
| 68 | 37 | 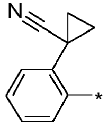 | F |
| 69 | 95 | | H |
| 70 | 38 | 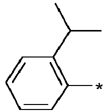 | F |
| 71 | 96 | | H |
| 72 | 39 | 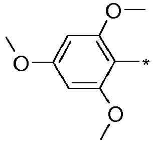 | F |
| 73 | 97 | | H |
| 74 | 40 | 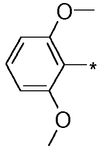 | F |
| 75 | 98 | | H |
| 76 | 41 | 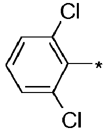 | F |
| 77 | 99 | | H |
| 78 | 42 | 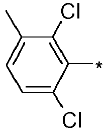 | F |
| 79 | 100 | | H |
| 80 | 43 | 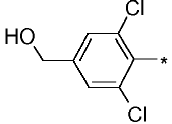 | F |
| 81 | 101 | | H |
| 82 | 44 | 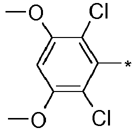 | F |
| 83 | 102 | | H |
| 84 | 45 | 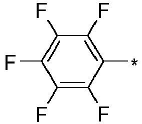 | F |
| 85 | 103 | | H |
| 86 | 46 | 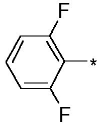 | F |
| 87 | 104 | | H |
| 88 | 47 | 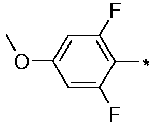 | F |
| 89 | 105 | | H |
| 90 | 48 | 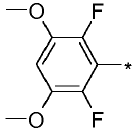 | F |
| 91 | 106 | | H |
| 92 | 49 | 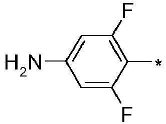 | F |
| 93 | 107 | | H |
| 94 | 50 | 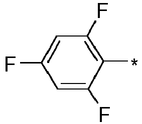 | F |
| 95 | 108 | | H |
| 96 | 51 | 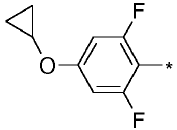 | F |
| 97 | 109 | | H |
| 98 | 52 | 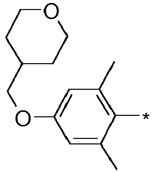 | F |
| 99 | 110 | | H |
| 100 | 53 | 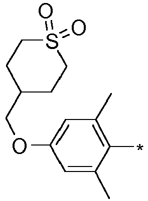 | F |
| 101 | 111 | | H |
| 102 | 54 | 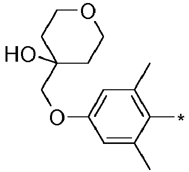 | F |
| 103 | 112 | | H |
| 104 | 55 | 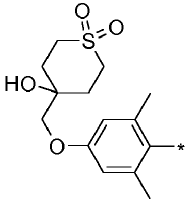 | F |
| 105 | 113 | | H |
| 106 | 56 | 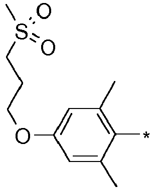 | F |
| 107 | 114 | | H |
| 108 | 57 | 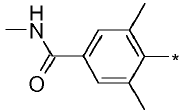 | F |
| 109 | 115 | | H |
| 110 | 58 | 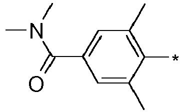 | F |
| 111 | 116 | | H |
| 112 | 59 | 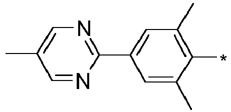 | F |
| 113 | 117 | | H |
| 114 | 60 | 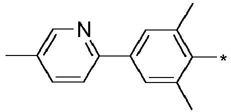 | F |
| 115 | 118 | | H |
| 116 | 61 | 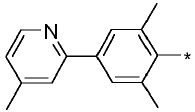 | F |
| 117 | 119 | | H |

### Example 118

### 2-(6-{[(1R)-4-[4-(2-Hydroxy-2-methylpropoxy)-2,6-dimethylphenyl]-2,3-dihydro-1H-inden-1-yl]amino}-2H,3H-furo[3,2-b]pyridin-3-yl)acetic acid

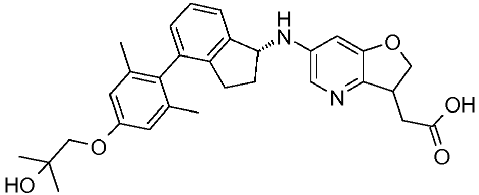

A microwave vial charged with a stir bar, methyl 2-(6-{[(1R)-4-(tetramethyl-1,3,2-dioxaborolan-2-yl)-2,3-dihydro-1H-inden-1-yl]amino}-2H,3H-furo[3,2-b]pyridin-3-yl)acetate (47 mg), 1-(4-bromo-3,5-dimethyl-phenoxy)-2-methyl-propan-2-ol (43 mg), K₃PO₄ (209 µL of a 1 M aqueous solution) and tetrahydrofurane (1 mL) is purged with argon for 10 min. Dichloro[1,3-bis(2,6di-3-pentylphenyl)-4,5-dichloro-imidazol-2-ylidene](3-chloropyridyl)palladium(II) (Pd-PEPPSI-IPent^{Cl}, 8 mg) is added, the vial is sealed, and the mixture is stirred at 140 °C for 20 min. After cooling to room temperature, the mixture is treated with NaOH (400 µL of a 4 M aqueous solution) for 12 hours. Then the mixture is acidified by addition of trifluoroacetic acid and chromatographed (HPLC on reversed phase, acetonitrile/water/trifluoroacetic acid) to give the corresponding carboxylic acid as trifluoroacetic acid salt. LC (method 2): t_{R} = 0.95 min; Mass spectrum (ESI⁺): m/z = 503 [M+H]⁺.

### Example 119

### 2-(6-{[(1R)-4-[2,6-Dimethyl-4-(oxan-4-yloxy)phenyl]-2,3-dihydro-1H-inden-1-yl]amino}-2H,3H-furo[3,2-b]pyridin-3-yl)acetic acid

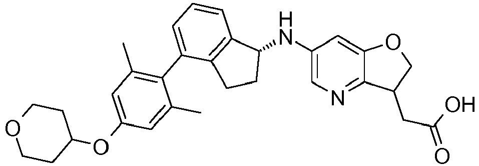

The title compound is prepared from methyl 2-(6-{[(1R)-4-(tetramethyl-1,3,2-dioxaborolan-2-yl)-2,3-dihydro-1H-inden-1-yl]amino}-2H,3H-furo[3,2-b]pyridin-3-yl)acetate and 4-(4-bromo-3,5-dimethylphenoxy)oxane following a procedure analogous to that described for Example 118. LC (method 2): t_{R} = 1.00 min; Mass spectrum (ESI⁺): m/z = 515 [M+H]⁺.

### Example 120

### 2-(6-{[(1R)-4-[2,6-Dimethyl-4-(1-methyl-2-oxo-1,2-dihydropyridin-4-yl)phenyl]-2,3-dihydro-1H-inden-1-yl]amino}-2H,3H-furo[3,2-b]pyridin-3-yl)acetic acid

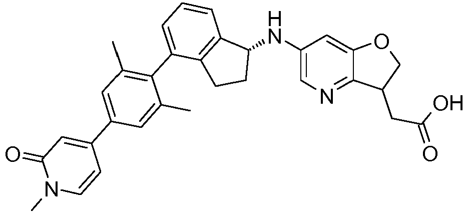

A microwave vial charged with a stir bar, methyl 2-(6-{[(1R)-4-(tetramethyl-1,3,2-dioxaborolan-2-yl)-2,3-dihydro-1H-inden-1-yl]amino}-2H,3H-furo[3,2-b]pyridin-3-yl)acetate (55 mg), 4-(4-bromo-3,5-dimethylphenyl)-1-methyl-1,2-dihydropyridin-2-one (46 mg), K₃PO₄ (209 µL of a 1 M aqueous solution) and tetrahydrofurane (1 mL) is purged with argon for 10 min. 1,1'-bis(di-*tert*-butylphosphino)ferrocene-palladium dichloride (1.4 mg) is added, the vial is sealed, and the mixture is stirred at 120 °C for 10 min. After cooling to room temperature, the mixture is treated with NaOH (400 µL of a 4 M aqueous solution) for 12 hours. Then the mixture is acidified by addition of trifluoroacetic acid and chromatographed (HPLC on reversed phase, acetonitrile/water/trifluoroacetic acid) to give the corresponding carboxylic acid as trifluoroacetic acid salt. LC (method 2): t_{R} = 0.91 min; Mass spectrum (ESI⁺): m/z = 522 [M+H]⁺.

### Example 121

### 2-(6-{[(1R)-4-[2,6-Dimethyl-4-(1-methyl-6-oxo-1,6-dihydropyridin-3-yl)phenyl]-2,3-dihydro-1H-inden-1-yl]amino}-2H,3H-furo[3,2-b]pyridin-3-yl)acetic acid

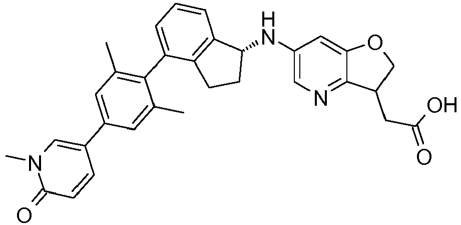

The title compound is prepared from methyl 2-(6-{[(1R)-4-(tetramethyl-1,3,2-dioxaborolan-2-yl)-2,3-dihydro-1H-inden-1-yl]amino}-2H,3H-furo[3,2-b]pyridin-3-yl)acetate and 5-(4-bromo-3,5-dimethylphenyl)-1-methyl-1,2-dihydropyridin-2-one following a procedure analogous to that described for Example 120. LC (method 2): t_{R} = 0.91 min; Mass spectrum (ESI⁺): m/z = 522 [M+H]⁺.

### Example 122

### 2-(6-{[(1R)-4-{4-[2-(2-Hydroxy-2-methylpropyl)-2H-1,2,3,4-tetrazol-5-yl]-2,6-dimethylphenyl}-2,3-dihydro-1H-inden-1-yl]amino}-2H,3H-furo[3,2-b]pyridin-3-yl)acetic acid

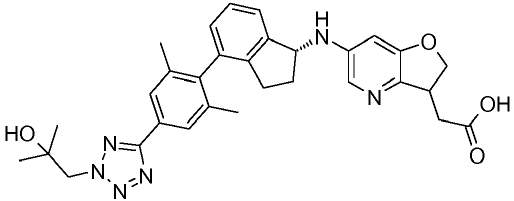

The title compound is prepared from methyl 2-(6-{[(1R)-4-(tetramethyl-1,3,2-dioxaborolan-2-yl)-2,3-dihydro-1H-inden-1-yl]amino}-2H,3H-furo[3,2-b]pyridin-3-yl)acetate and 1-[5-(4-bromo-3,5-dimethylphenyl)-2H-1,2,3,4-tetrazol-2-yl]-2-methylpropan-2-ol following a procedure analogous to that described for Example 120. LC (method 2): t_{R} = 0.95 min; Mass spectrum (ESI⁺): m/z = 555 [M+H]⁺.

### Example 123

### 2-(6-{[(1R)-4-[4-(2-Cyano-2,2-dimethylethoxy)-2,6-dimethylphenyl]-2,3-dihydro-1H-inden-1-yl]amino}-2H,3H-furo[3,2-b]pyridin-3-yl)acetic acid

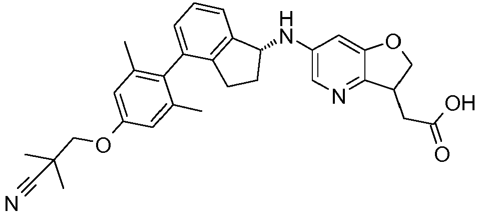

The title compound is prepared from tert-butyl 2-(6-{[(1R)-4-(tetramethyl-1,3,2-dioxaborolan-2-yl)-2,3-dihydro-1H-inden-1-yl]amino}-2H,3H-furo[3,2-b]pyridin-3-yl)acetate and 3-(4-bromo-3,5-dimethylphenoxy)-2,2-dimethylpropanenitrile following a procedure analogous to that described for Example 120. The mixture is heated to 140°C for 20 min. LC (method 2): t_{R} = 0.88 min; Mass spectrum (ESI⁺): m/z = 512 [M+H]⁺.

### Example 124

### 2-(6-{[(1R)-4-(2,6-Diethylphenyl)-2,3-dihydro-1H-inden-1-yl]amino}-2H,3H-furo[3,2-b]pyridin-3-yl)acetic acid

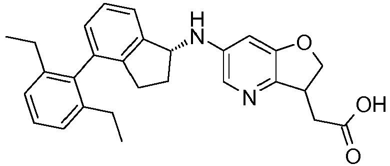

The title compound is prepared from tert-butyl 2-(6-{[(1R)-4-(tetramethyl-1,3,2-dioxaborolan-2-yl)-2,3-dihydro-1H-inden-1-yl]amino}-2H,3H-furo[3,2-b]pyridin-3-yl)acetate and 2-bromo-1,3-diethylbenzene following a procedure analogous to that described for Example 118. LC (method 2): t_{R} = 1.05 min; Mass spectrum (ESI⁺): m/z = 443 [M+H]⁺.

### Example 125

### 2-(6-{[(1R)-4-[2-(Trifluoromethyl)phenyl]-2,3-dihydro-1H-inden-1-yl]amino}-2H,3H-furo[3,2-b]pyridin-3-yl)acetic acid

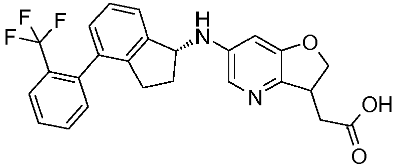

The title compound is prepared from tert-butyl 2-(6-{[(1R)-4-(tetramethyl-1,3,2-dioxaborolan-2-yl)-2,3-dihydro-1H-inden-1-yl]amino}-2H,3H-furo[3,2-b]pyridin-3-yl)acetate and 1-iodo-2-(trifluoromethyl)benzene following a procedure analogous to that described for Example 118. LC (method 2): t_{R} = 0.99 min; Mass spectrum (ESI⁺): m/z = 455 [M+H]⁺.

### Example 126

### 2-(6-{[(1R)-4-[4-(Dimethylcarbamoyl)-2,6-dimethylphenyl]-2,3-dihydro-1H-inden-1-yl]amino}-2H,3H-furo[3,2-b]pyridin-3-yl)acetic acid

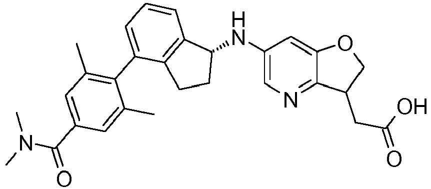

The title compound is prepared from tert-butyl 2-(6-{[(1R)-4-(tetramethyl-1,3,2-dioxaborolan-2-yl)-2,3-dihydro-1H-inden-1-yl]amino}-2H,3H-furo[3,2-b]pyridin-3-yl)acetate and 4-bromo-N,N,3,5-tetramethylbenzamide following a procedure analogous to that described for Example 118. LC (method 2): t_{R} = 0.90 min; Mass spectrum (ESI⁺): m/z = 486 [M+H]⁺.

### Example 127

### 2-(6-{[(1R)-4-(2,6-Difluoro-4-methoxyphenyl)-2,3-dihydro-1H-inden-1-yl]amino}-2H,3H-furo[3,2-b]pyridin-3-yl)acetic acid

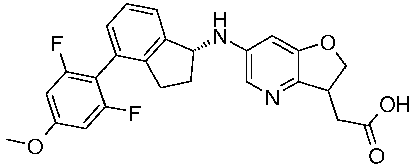

The title compound is prepared from tert-butyl 2-(6-{[(1R)-4-(tetramethyl-1,3,2-dioxaborolan-2-yl)-2,3-dihydro-1H-inden-1-yl]amino}-2H,3H-furo[3,2-b]pyridin-3-yl)acetate and 2-bromo-1,3-difluoro-5-methoxybenzene following a procedure analogous to that described for Example 118. LC (method 2): t_{R} = 0.96 min; Mass spectrum (ESI⁺): m/z = 453 [M+H]⁺.

### Example 128

### 2-(6-{[(1R)-4-(2,6-dichloro-3-methylphenyl)-2,3-dihydro-1H-inden-1-yl]amino}-2H,3H-furo[3,2-b]pyridin-3-yl)acetic acid

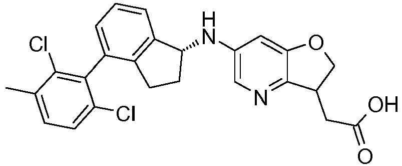

The title compound is prepared from tert-butyl 2-(6-{[(1R)-4-(tetramethyl-1,3,2-dioxaborolan-2-yl)-2,3-dihydro-1H-inden-1-yl]amino}-2H,3H-furo[3,2-b]pyridin-3-yl)acetate and 1,3-dichloro-2-iodo-4-methylbenzene following a procedure analogous to that described for Example 118. LC (method 2): t_{R} = 1.01 min; Mass spectrum (ESI⁺): m/z = 469 [M+H]⁺.

### Example 129

### 2-(6-{[(1R)-4-[2-(Propan-2-yl)phenyl]-2,3-dihydro-1H-inden-1-yl]amino}-2H,3H-furo[3,2-b]pyridin-3-yl)acetic acid

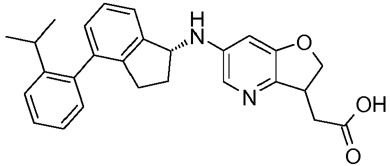

The title compound is prepared from tert-butyl 2-(6-{[(1R)-4-(tetramethyl-1,3,2-dioxaborolan-2-yl)-2,3-dihydro-1H-inden-1-yl]amino}-2H,3H-furo[3,2-b]pyridin-3-yl)acetate and 1-bromo-2-(propan-2-yl)benzene following a procedure analogous to that described for Example 118. The mixture is heated to 120°C for 20 min. LC (method 2): t_{R} = 1.02 min; Mass spectrum (ESI⁺): m/z = 429 [M+H]⁺.

### Example 130

### 2-(6-{[(1R)-4-(2,6-Dicyanophenyl)-2,3-dihydro-1H-inden-1-yl]amino}-2H,3H-furo[3,2-b]pyridin-3-yl)acetic acid

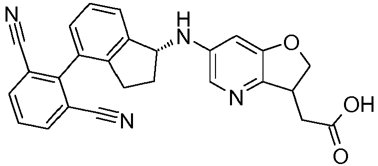

The title compound is prepared from tert-butyl 2-(6-{[(1R)-4-(tetramethyl-1,3,2-dioxaborolan-2-yl)-2,3-dihydro-1H-inden-1-yl]amino}-2H,3H-furo[3,2-b]pyridin-3-yl)acetate and 2-iodobenzene-1,3-dicarbonitrile following a procedure analogous to that described for Example 118. The mixture is heated to 120°C for 30 min. LC (method 3): t_{R} = 0.73 min; Mass spectrum (ESI⁺): m/z = 437 [M+H]⁺.

### Example 131

### 2-(6-{[(1R)-4-[2-(1-Cyanocyclopropyl)phenyl]-2,3-dihydro-1H-inden-1-yl]amino}-2H,3H-furo[3,2-b]pyridin-3-yl)acetic acid

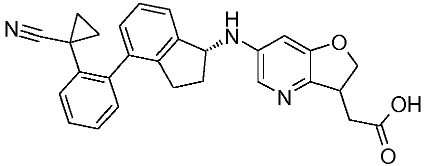

The title compound is prepared from tert-butyl 2-(6-{[(1R)-4-(tetramethyl-1,3,2-dioxaborolan-2-yl)-2,3-dihydro-1H-inden-1-yl]amino}-2H,3H-furo[3,2-b]pyridin-3-yl)acetate and 1-(2-bromophenyl)cyclopropane-1-carbonitrile following a procedure analogous to that described for Example 118. The mixture is heated to 120°C for 20 min. and to 140°C for 20 min. LC (method 2): t_{R} = 0.93 min; Mass spectrum (ESI⁺): m/z = 452 [M+H]⁺.

### Example 132

### 2-(6-{[(1R)-4-[2,6-Dimethyl-4-(5-methyl-1,2,4-oxadiazol-3-yl)phenyl]-2,3-dihydro-1H-inden-1-yl]amino}-2H,3H-furo[3,2-b]pyridin-3-yl)acetic acid

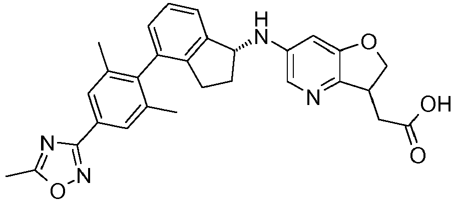

The title compound is prepared from tert-butyl 2-(6-{[(1R)-4-(tetramethyl-1,3,2-dioxaborolan-2-yl)-2,3-dihydro-1H-inden-1-yl]amino}-2H,3H-furo[3,2-b]pyridin-3-yl)acetate and 3-(4-bromo-3,5-dimethylphenyl)-5-methyl-1,2,4-oxadiazole following a procedure analogous to that described for Example 118. The mixture is heated to 120°C for 40 min. LC (method 2): t_{R} = 0.98 min; Mass spectrum (ESI⁺): m/z = 497 [M+H]⁺.

### Example 133

### 2-(6-{[(1R)-4-[2,6-Dimethyl-4-(1-methyl-6-oxo-1,6-dihydropyrimidin-4-yl)phenyl]-2,3-dihydro-1H-inden-1-yl]amino}-2H,3H-furo[3,2-b]pyridin-3-yl)acetic acid

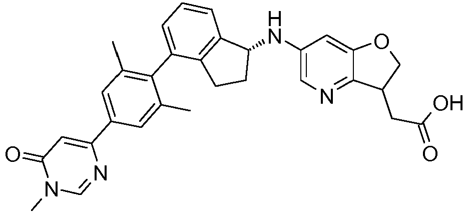

A microwave vial charged with a stir bar, tert-butyl 2-(6-{[(1R)-4-(tetramethyl-1,3,2-dioxaborolan-2-yl)-2,3-dihydro-1H-inden-1-yl]amino}-2H,3H-furo[3,2-b]pyridin-3-yl)acetate (50 mg), 6-(4-iodo-3,5-dimethylphenyl)-3-methyl-3,4-dihydropyrimidin-4-one (52 mg), Cs₂CO₃ (102 µL of a 2 M aqueous solution) and N,N-dimethylformamide (1 mL) is purged with argon for 10 min. 1,1'-bis(di-*tert-*butylphosphino)ferrocene-palladium dichloride (7 mg) is added, the vial is sealed, and the mixture is stirred at 90 °C for 2 hours. After cooling to room temperature, the mixture is diluted with N,N-dimethylformamide, filtered and chromatographed (HPLC on reversed phase, acetonitrile/water/NH₄OH) to give the tert.-butyl ester intermediate, which is dissolved in dichloromethane (5 mL). Hydrochloric acid (500 µL of a 4 M solution in 1,4-dioxane) is added and the mixture is stirred for 4 hours at room temperature. The solvents are evaporated in vacuo. The residue is suspended in acetonitrile and the solid is isolated by filtration to give the title compound as the hydrochloride. LC (method 2): t_{R} = 0.89 min; Mass spectrum (ESI⁺): m/z = 523 [M+H]⁺.

## Claims

1. A compound of formula I 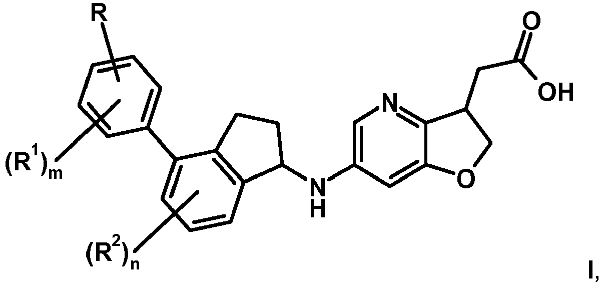 wherein
R is selected from a group consisting of
H, F, Cl, Br, I, C₁₋₆-alkyl, C₂₋₆-alkenyl, C₂₋₆-alkynyl, C₃₋₆-cycloalkyl, NC-, HNR^{N}-C(=O)-, C₁₋₄-alkyl-NR^{N}-C(=O)-, C₃₋₆-cycloalkyl-NR^{N}-C(=O)-, heterocyclyl-NR^{N}-C(=O)-, heteroaryl-NR^{N}-C(=O)-, HOOC-, C₁₋₄-alkyl-O-C(=O)-, O₂N-, HR^{N}N-, C₁₋₄-alkyl-R^{N}N-, C₁₋₄-alkyl-C(=O)NR^{N}-, C₃₋₆-cycloalkyl-C(=O)NR^{N}-, heterocyclyl-C(=O)NR^{N}-, heteroaryl-C(=O)NR^{N}-, C₁₋₄-alkyl-S(=O)₂NR^{N}-, C₃₋₆-cycloalkyl-S(=O)₂NR^{N}-, heterocyclyl-S(=O)₂NR^{N}-, heteroaryl-S(=O)₂NR^{N}-, HO-, C₁₋₆-alkyl-O-, HOOC-C₁₋₃-alkyl-O-, heterocyclyl-C₁₋₃-alkyl-O-, phenyl-C₁₋₃-alkyl-O-, C₃₋₆-cycloalkyl-O-, heterocyclyl-O-, heteroaryl-O-, C₁₋₄-alkyl-S-, C₃₋₆-cycloalkyl-S-, heterocyclyl-S-, C₁₋₄-alkyl-S(=O)-, C₃₋₆-cycloalkyl-S(=O)-, heterocyclyl-S(=O)-, C₁₋₄-alkyl-S(=O)₂-, C₃₋₆-cycloalkyl-S(=O)₂-, heterocyclyl-S(=O)₂-, phenyl-S(=O)₂-, heteroaryl-S(=O)₂-, HNR^{N}-S(=O)₂-, C₁₋₄-alkyl-NR^{N}-S(=O)₂-, heterocyclyl, phenyl, and heteroaryl,
wherein each alkyl, cycloalkyl, and heterocyclyl group or sub-group within the groups forming R is optionally substituted with 1 or more F atoms and optionally substituted with 1 to 3 groups independently selected from Cl, C₁₋₃-alkyl, NC-, (R^{N})₂N-, HO-, C₁₋₃-alkyl-O-, and C₁₋₃-alkyl-S(=O)₂-; and
wherein each phenyl and heteroaryl group or sub-group within the groups forming R is optionally substituted with 1 to 5 substituents independently selected from F, Cl, C₁₋₃-alkyl, HF₂C-, F₃C-, NC-, (R^{N})₂N-, HO-, C₁₋₃-alkyl-O-, F₃C-O-, and C₁₋₃-alkyl-S(=O)₂-;
wherein each heterocyclyl group or sub-group within the groups forming R is selected from
a cyclobutyl group wherein 1 CH₂ group is replaced by -NR^{N}- or -O-;
a C₅₋₆-cycloalkyl group wherein 1 CH₂ group is replaced by -C(=O)-, -NR^{N}-, -O- or -S(=O)₂- and/or 1 CH group is replaced by N;
a C₅₋₆-cycloalkyl group wherein 1 CH₂ group is replaced by -NR^{N}- or -O-, a second CH₂ group is replaced by -NR^{N}-, -C(=O)- or -S(=O)₂- and/or 1 CH group is replaced by N; and
a C₅₋₆-cycloalkyl group wherein 2 CH₂ groups are replaced by -NR^{N}- or 1 CH₂ group by -NR^{N}- and the other by -O- and a third CH₂ group is replaced by -C(=O)- or -S(=O)₂- and/or 1 CH group is replaced by N;
wherein each heteroaryl group or sub-group within the groups forming R is selected from
tetrazolyl and a 5- or 6-membered heteroaromatic ring which contains 1, 2, or 3 heteroatoms independently of each other selected from =N-, -NR^{N}-, -O-, and -S-, wherein in heteroaromatic groups containing a -HC=N- unit this group is optionally replaced by -NR^{N}-C(=O)-;
wherein in heteroaryl and heterocyclyl rings with one or more NH groups, each of said NH groups is replaced by NR^{N};
R¹ is selected from a group consisting of H, F, Cl, C₁₋₄-alkyl, C₃₋₆-cycloalkyl-, HO-C₁₋₄-alkyl, C₁₋₄-alkyl-O-C₁₋₄-alkyl, NC-, HO-, C₁₋₄-alkyl-O-, C₃₋₆-cycloalkyl-O-, C₁₋₄-alkyl-S-, C₁₋₄-alkyl-S(O)-, and C₁₋₄-alkyl-S(O)₂-,
wherein any alkyl and cycloalkyl group or sub-group within the groups forming R¹ is optionally substituted with 1 or more F atoms, and wherein multiple R¹ may be identical or different if m is 2, 3 or 4;
m is an integer selected from 1, 2, 3, and 4;
R² is H, F, Cl, C₁₋₄-alkyl, NC-, or C₁₋₄-alkyloxy,
wherein any alkyl group or sub-group within the groups forming R² is optionally substituted with 1 or more F atoms, and wherein multiple R² may be identical or different if n is 2 or 3;
n is an integer selected from 1, 2, and 3;
R^{N} is independently of each other selected from a group consisting of H, C₁₋₄-alkyl, HO-(C₁₋₄-alkyl)-H₂C-, C₁₋₃-alkyl-O-C₁₋₄-alkyl-, C₁₋₄-alkyl-C(=O)-, C₁₋₄-alkyl-NH-C(=O)-, C₁₋₄-alkyl-N(C₁₋₄-alkyl)-C(=O)-, C₁₋₄-alkyl-O-C(=O)-, and C₁₋₄-alkyl-S(=O)₂-,
wherein each alkyl group or sub-group within the groups forming R^{N} is optionally substituted with 1 or more F atoms;
wherein in any definition mentioned hereinbefore and if not specified otherwise, any alkyl group or sub-group may be straight-chained or branched,
or a salt thereof.

2. The compound according to claim 1, wherein
R is selected from the group consisting of H, F, Cl, C₁₋₆-alkyl, C₃₋₆-cycloalkyl, NC-, HNR^{N}-C(=O)-, C₁₋₄-alkyl-NR^{N}-C(=O)-, C₃₋₆-cycloalkyl-NR^{N}-C(=O)-, heterocyclyl-NR^{N}-C(=O)-, HOOC-, HR^{N}N-, C₁₋₄-alkyl-R^{N}N-, C₁₋₄-alkyl-C(=O)NR^{N}-, C₃₋₆-cycloalkyl-C(=O)NR^{N}-, heterocyclyl-C(=O)NR^{N}-, C₁₋₄-alkyl-S(=O)₂NR^{N}-, HO-, C₁₋₆-alkyl-O-, HOOC-C₁₋₂-alkyl-O-, heterocyclyl-C₁₋₂-alkyl-O-, phenyl-C₁₋₂-alkyl-O-, C₃₋₆-cycloalkyl-O-, heterocyclyl-O-, heteroaryl-O-, C₁₋₄-alkyl-S(=O)₂-, C₃₋₆-cycloalkyl-S(=O)₂-, heterocyclyl-S(=O)₂-, HNR^{N}-S(=O)₂-, C₁₋₄-alkyl-NR^{N}-S(=O)₂-, heterocyclyl, and heteroaryl,
wherein each alkyl, cycloalkyl, and heterocyclyl group or sub-group within the groups forming R is optionally substituted with 1 or more F atoms and optionally substituted with 1 to 2 groups independently selected from Cl, H₃C-, NC-, R^{N}HN-, HO-, H₃C-O-, and H₃C-S(=O)₂-;
wherein each heteroaryl group or sub-group within the groups forming R is optionally substituted with 1 to 3 substituents independently selected from F, Cl, H₃C-, F₃C-, NC-, (R^{N})₂N-, HO-, H₃C-O-, F₃C-O-, and H₃C-S(=O)₂-;
wherein each heterocyclyl group or sub-group within the groups forming R is selected from
a cyclobutyl group wherein 1 CH₂ group is replaced by -NR^{N}- or -O-;
a C₅₋₆-cycloalkyl group wherein 1 CH₂ group is replaced by -C(=O)-, -NR^{N}-, -O- or -S(=O)₂- and/or 1 CH group is replaced by N;
a C₅₋₆-cycloalkyl group wherein 1 CH₂ group is replaced by -NR^{N}- or -O-, a second CH₂ group is replaced by -NR^{N}-, -C(=O)- or -S(=O)₂- and/or 1 CH group is replaced by N;
wherein each heteroaryl group or sub-group within the groups forming R is selected from
tetrazolyl, a 5-membered heteroaromatic ring which contains 1, 2 or 3 heteroatoms independently of each other selected from =N-, -NH-, O and S, and a 6-membered heteroaromatic ring which contains 1 or 2 =N- atoms, wherein a -HC=N- unit is optionally replaced by -NH-C(=O)-;
and wherein in each heteroaryl and heterocyclyl group or sub-group containing one or more NH groups, said group(s) is replaced by NR^{N};
or a salt thereof.

3. The compound according to claim 1, wherein
R is selected from the group consisting of F, Cl, NC-, H₂NC(=O)-, H₃CHN-C(=O)-, (H₃C)₂N-C(=O)-, HOOC-, H₂N-, HO-;
C₁₋₃-alkyl optionally substituted with 1 or more F or optionally monosubstituted with HO-;
cyclopropyl optionally monosubstituted with NC-;
H₃C-O- optionally monosubstituted with C₁₋₄-alkyl, HOOC-, oxetanyl, tetrahydrofuranyl, tetrahydropyranyl, 1,1-dioxotetrahydrothiopyranyl, or phenyl, wherein said C₁₋₄-alkyl group is optionally monosubstituted with NC-, HO- or H₃C-S(=O)₂-, and wherein each of said oxetanyl, tetrahydrofuranyl, tetrahydropyranyl, 1,1-dioxotetrahydrothiopyranyl groups is optionally monosubstituted with H₃C- or HO-;
cyclopropyl-O-, tetrahydrofuranyl-O- and tetrahydropyranyl-O-; and
a heteroaryl group selected from pyrazolyl, [1,2,4]oxadiazolyl, tetrazolyl, pyridyl, pyridin-2-onyl, pyrazinyl, pyrimidinyl, and pyrimidin-4-onyl, wherein each of said heteroaryl groups is optionally monosubstituted with H₃C- and wherein each H-N group in said heteroaryl groups is optionally replaced with H₃C-N or (H₃C)₂C(OH)-H₂C-N;
or a salt thereof.

4. The compound according to claim 1, 2 or 3, wherein
R¹ is H, F, Cl, H₃C-, H₃C-H₂C-, F₃C-, NC-, or H₃CO-;
R² is H or F;
m is 2 and
n is 1;
or a salt thereof.

5. The compound according to claim 1, 2, 3 or 4, wherein R¹ is H₃C-; or a salt thereof.

6. The compound according to claim 1, wherein
R is selected from a group consisting of:
F, Cl, NC-, H₂NC(=O)-, H₃CHN-C(=O)-, (H₃C)₂N-C(=O)-, HOOC-, H₂N-, HO-;
C₁₋₃-alkyl optionally substituted with 1 or more F or optionally monosubstituted with HO-;
cyclopropyl optionally monosubstituted with NC-;
H₃C-O- optionally monosubstituted with C₁₋₄-alkyl, HOOC-, oxetanyl, tetrahydrofuranyl, tetrahydropyranyl, 1,1-dioxotetrahydrothiopyranyl, or phenyl,
wherein the C₁₋₄-alkyl group optionally attached to H₃C-alkyl-O- is optionally monosubstituted with NC-, HO- or H₃C-S(=O)₂-, and
wherein said oxetanyl, tetrahydrofuranyl, tetrahydropyranyl and 1,1-dioxotetrahydrothiopyranyl groups are optionally monosubstituted with H₃C- or HO-;
cyclopropyl-O-, tetrahydrofuranyl-O- and tetrahydropyranyl-O-;
a heteroaryl group selected from pyrazolyl, [1,2,4]oxadiazolyl, tetrazolyl, pyridyl, pyridin-2-onyl, pyrazinyl, pyrimidinyl, and pyrimidin-4-onyl,
wherein each of said heteroaryl groups is optionally monosubstituted with H₃C-, and
wherein each H-N group in said heteroaryl groups is optionally replaced with H₃C-N or (H₃C)₂C(OH)-H₂C-N;
R¹ is H, F, Cl, H₃C-, H₃C-H₂C -, F₃C-, NC-, or H₃C-O-;
m is 2;
R² is H or F; and
n is 1;
or a salt thereof.

7. The compound according to claim 1, wherein
R is selected from a group consisting of
F, Cl, H₃C-, H₃C-H₂C-, (H₃C)₂CH-, 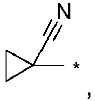 F₃C-, HOCH₂-, NC-, H₂N-C(=O)-, H₃C-NH-C(=O)-, (H₃C)₂N-C(=O)-, HOOC-, H₂N-, HO-, H₃C-O-, cyclopropyl-O-, 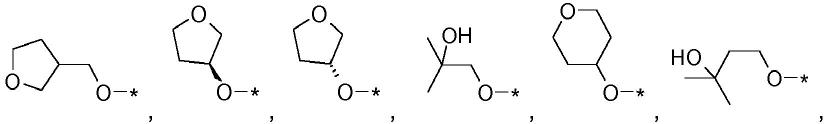 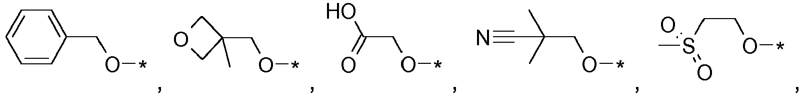 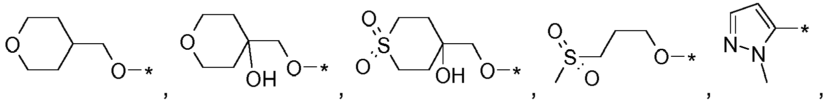 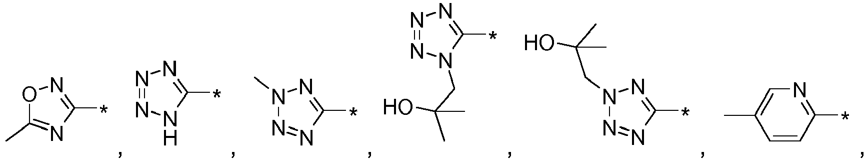 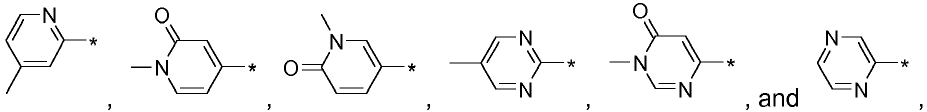 wherein the asterisk, -*, indicates the site/point of attachment;
R¹ is H₃C-;
m is 2;
R² is F; and
n is 1;
or a salt thereof.

8. The compound according to any one of the claims 1 to 7, with the structure and stereochemistry shown in formulae I.1, I.2, or I.3 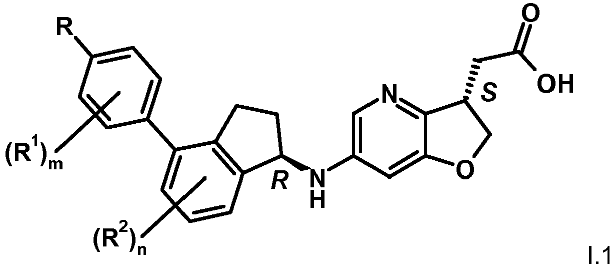 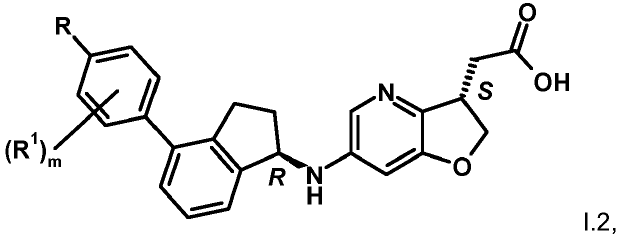 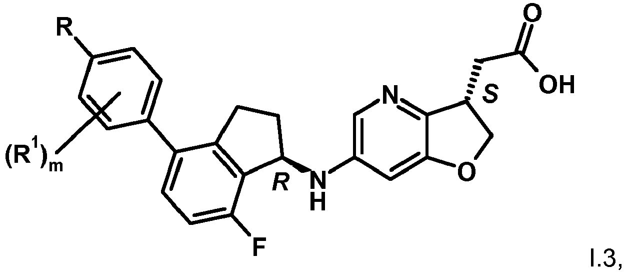 or a salt thereof.

9. A compound with one of the following structures 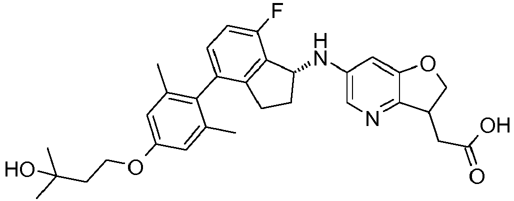 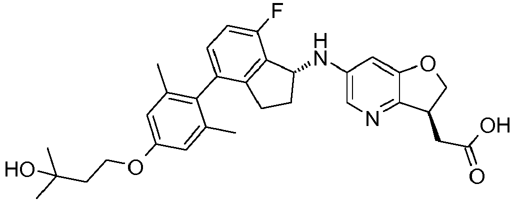 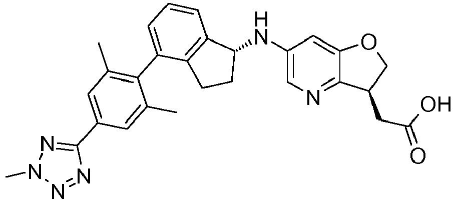 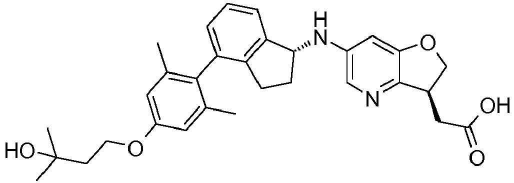 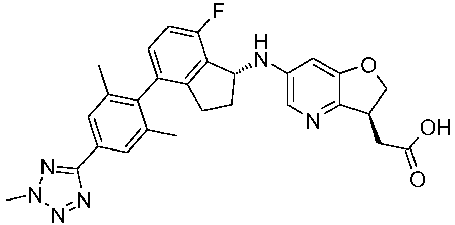 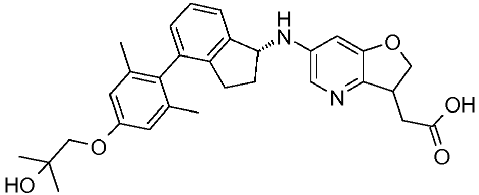 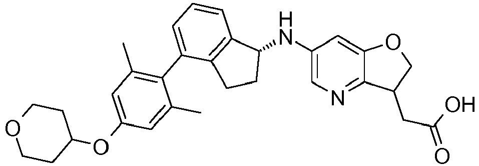 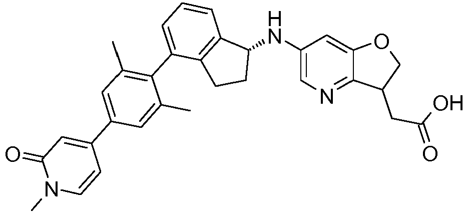 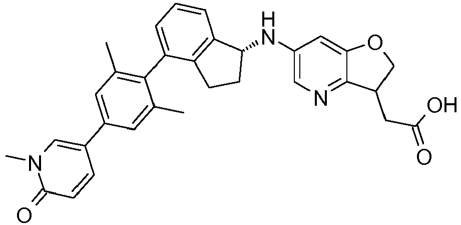 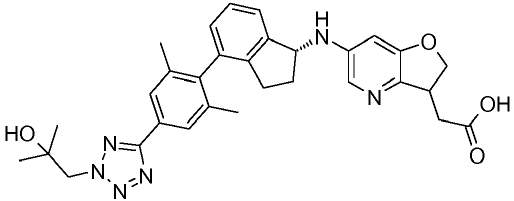 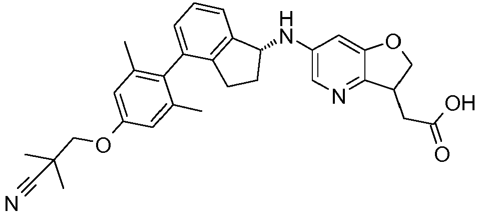 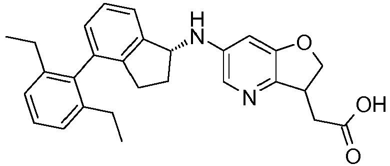 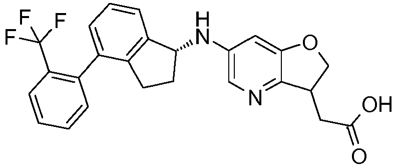 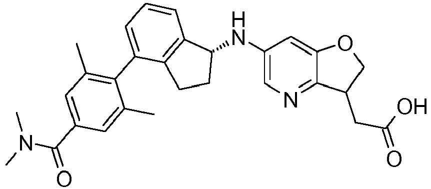 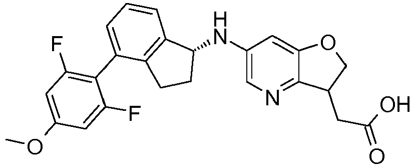 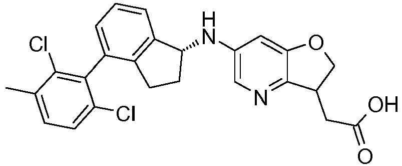 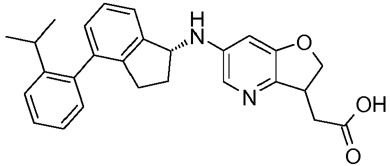 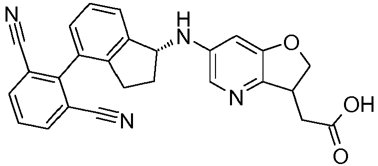 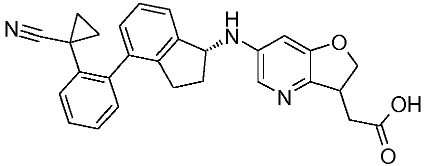 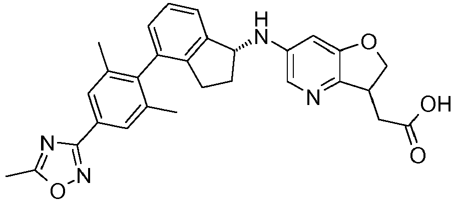 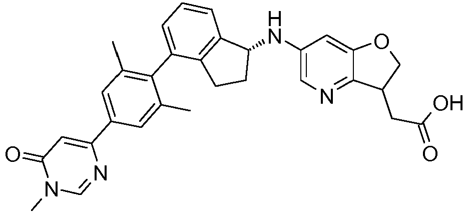 or a salt thereof.

10. A pharmaceutically acceptable salt of a compound according to one or more of the claims 1 to 9.

11. A pharmaceutical composition comprising one or more compounds according to one or more of the claims 1 to 9 or one or more pharmaceutically acceptable salts thereof, optionally together with one or more inert carriers and/or diluents.

12. A compound according to one or more of the claims 1 to 9 or a pharmaceutically acceptable salt thereof for use as a medicament.

13. A compound according to one or more of the claims 1 to 9 or a pharmaceutically acceptable salt thereof for use in the prophylaxis and/or therapy of metabolic diseases and conditions associated with the disease.

14. A compound for use according to claim 13, wherein the metabolic disease is diabetes, and the conditions associated with the disease are selected from the group consisting of insulin resistance, obesity, cardiovascular disease and dyslipidemia.

15. A compound for use according to claim 13, wherein the metabolic disease is type 2 diabetes mellitus.

16. A pharmaceutical composition comprising one or more compounds according to one or more of the claims 1 to 9 or one or more pharmaceutically acceptable salts thereof and one or more additional therapeutic agents, optionally together with one or more inert carriers and/or diluents.

17. A pharmaceutical composition according to claim 16 wherein the additional therapeutic agents are selected from the group consisting of antidiabetic agents, agents for the treatment of overweight and/or obesity and agents for the treatment of high blood pressure, heart failure and/or atherosclerosis.

## Patentansprüche

1. Verbindung der Formel I 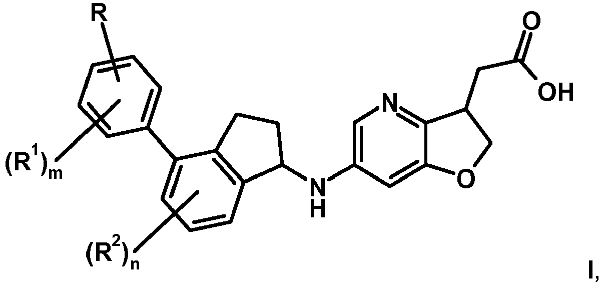 in der
R ausgewählt ist aus einer Gruppe, bestehend aus
H, F, Cl, Br, I, C₁₋₆-Alkyl, C₂₋₆-Alkenyl, C₂₋₆-Alkinyl, C₃₋₆-Cycloalkyl, NC-, HNR^{N}-C(=O)-, C₁₋₄-Alkyl-NR^{N}-C(=O)-, C₃₋₆-Cycloalkyl-NR^{N}-C(=O)-, Heterocyclyl-NR^{N}-C(=O)-, Heteroaryl-NR^{N}-C(=O)-, HOOC-, C₁₋₄-Alkyl-O-C(=O)-, O₂N-, HR^{N}N-, C₁₋₄-Alkyl-R^{N}N-, C₁₋₄-Alkyl-C(=O)NR^{N}-, C₃₋₆-Cycloalkyl-C(=O)NR^{N}-, Heterocyclyl-C(=O)NR^{N}-, Heteroaryl-C(=O)NR^{N}-, C₁₋₄-Alkyl-S(=O)₂NR^{N}-, C₃₋₆-Cycloalkyl-S(=O)₂NR^{N}-, Heterocyclyl-S(=O)₂NR^{N}-, Heteroaryl-S(=O)₂NR^{N}-, HO-, C₁₋₆-Alkyl-O-, HOOC-C₁₋₃-Alkyl-O-, Heterocyclyl-C₁₋₃-alkyl-O-, Phenyl-C₁₋₃-alkyl-O-, C₃₋₆-Cycloalkyl-O-, Heterocyclyl-O-, Heteroaryl-O-, C₁₋₄-Alkyl-S-, C₃₋₆-Cycloalkyl-S-, Heterocyclyl-S-, C₁₋₄-Alkyl-S(=O)-, C₃₋₆-Cycloalkyl-S(=O)-, Heterocyclyl-S(=O)-, C₁₋₄-Alkyl-S(=O)₂-, C₃₋₆-Cycloalkyl-S(=O)₂-, Heterocyclyl-S(=O)₂-, Phenyl-S(=O)₂-, Heteroaryl-S(=O)₂-, HNR^{N}-S(=O)₂-, C₁₋₄-Alkyl-NR^{N}-S(=O)₂-, Heterocyclyl, Phenyl und Heteroaryl,
wobei jede Alkyl-, Cycloalkyl- und Heterocyclylgruppe oder -untergruppe innerhalb der Gruppen, die R bilden, gegebenenfalls mit 1 oder mehreren F-Atomen substituiert ist und gegebenenfalls mit 1 bis 3 Gruppen, unabhängig ausgewählt aus Cl, C₁₋₃-Alkyl, NC-, (R^{N})₂N-, HO-, C₁₋₃-Alkyl-O- und C₁₋₃-Alkyl-S(=O)₂-, substituiert ist; und
wobei jede Phenyl- und Heteroarylgruppe oder -untergruppe innerhalb der Gruppen, die R bilden, gegebenenfalls mit 1 bis 5 Substituenten, unabhängig ausgewählt aus F, Cl, C₁₋₃-Alkyl, HF₂C-, F₃C-, NC-, (R^{N})₂N-, HO-, C₁₋₃-Alkyl-O-, F₃C-O- und C₁₋₃-Alkyl-S(=O)₂-, substituiert ist;
wobei jede Heterocyclylgruppe oder -untergruppe innerhalb der Gruppen, die R bilden, ausgewählt ist aus
einer Cyclobutylgruppe, in der 1 CH₂-Gruppe durch -NR^{N}- oder -O- ersetzt ist;
einer C₅₋₆-Cycloalkylgruppe, in der 1 CH₂-Gruppe durch -C(=O)-, -NR^{N}-, -O- oder -S(=O)₂- ersetzt ist und/oder 1 CH-Gruppe durch N ersetzt ist; einer C₅₋₆-Cycloalkylgruppe, in der 1 CH₂-Gruppe durch -NR^{N}- oder -O-ersetzt ist, eine zweite CH₂-Gruppe durch -NR^{N}-, -C(=O)- oder -S(=O)₂-ersetzt ist und/oder 1 CH-Gruppe durch N ersetzt ist; und
einer C₅₋₆-Cycloalkylgruppe, in der 2 CH₂-Gruppen durch -NR^{N}- ersetzt sind oder 1 CH₂-Gruppe durch -NR^{N}- und die andere durch -O- und eine dritte CH₂-Gruppe durch -C(=O)- oder -S(=O)₂- ersetzt sind und/oder 1 CH-Gruppe durch N ersetzt ist;
wobei jede Heteroarylgruppe oder -untergruppe innerhalb der Gruppen, die R bilden, ausgewählt ist aus
Tetrazolyl und einem 5- oder 6-gliedrigem heteroaromatischen Ring, der 1, 2 oder 3 Heteroatome, unabhängig voneinander ausgewählt aus =N-, -NR^{N}-, -O- und -S-, enthält, wobei in heteroaromatischen Gruppen, die eine -HC=N-Einheit enthalten, diese Gruppe gegebenenfalls durch -NR^{N}-C(=O)- ersetzt ist;
wobei in Heteroaryl- und Heterocyclylringen mit einer oder mehreren NH-Gruppen jede dieser NH-Gruppen durch NR^{N} ersetzt ist;
R¹ ausgewählt ist aus einer Gruppe, bestehend aus H, F, Cl, C₁₋₄-Alkyl, C₃₋₆-Cycloalkyl-, HO-C₁₋₄-Alkyl, C₁₋₄-Alkyl-O-C₁₋₄-alkyl, NC-, HO-, C₁₋₄-Alkyl-O-, C₃₋₆-Cycloalkyl-O-, C₁₋₄-Alkyl-S-, C₁₋₄-Alkyl-S(O)- und C₁₋₄-Alkyl-S(O)₂-,
wobei jede Alkyl- und Cycloalkylgruppe oder -untergruppe innerhalb der Gruppen, die R¹ bilden, gegebenenfalls mit 1 oder mehreren F-Atomen substituiert ist, und wobei mehrere R¹ gleich oder verschieden sein können, wenn m 2, 3 oder 4 ist;
m eine ganze Zahl, ausgewählt aus 1, 2, 3 und 4, ist;
R² H, F, Cl, C₁₋₄-Alkyl, NC- oder C₁₋₄-Alkyloxy bedeutet,
wobei jede Alkylgruppe oder -untergruppe innerhalb der Gruppen, die R² bilden, gegebenenfalls mit 1 oder mehreren F-Atomen substituiert ist, und wobei mehrere R² gleich oder verschieden sein können, wenn n 2 oder 3 ist;
n eine ganze Zahl, ausgewählt aus 1, 2 und 3, ist;
R^{N} unabhängig voneinander ausgewählt ist aus einer Gruppe, bestehend aus H, C₁₋₄-Alkyl, HO-(C₁₋₄-Alkyl)-H₂C-, C₁₋₃-Alkyl-O-C₁₋₄-alkyl-, C₁₋₄-Alkyl-C(=O)-, C₁₋₄-Alkyl-NH-C(=O)-, C₁₋₄-Alkyl-N(C₁₋₄-alkyl)-C(=O)-, C₁₋₄-Alkyl-O-C(=O)- und C₁₋₄-Alkyl-S(=O)₂-,
wobei jede Alkylgruppe oder -untergruppe innerhalb der Gruppen, die R^{N} bilden, gegebenenfalls mit 1 oder mehreren F-Atomen substituiert ist;
wobei in allen oben genannten Definitionen und sofern nicht anders angegeben, jede Alkylgruppe oder -untergruppe geradkettig oder verzweigt sein kann,
oder ein Salz hiervon.

2. Verbindung gemäß Anspruch 1, in der
R ausgewählt ist aus der Gruppe, bestehend aus H, F, Cl, C₁₋₆-Alkyl, C₃₋₆-Cycloalkyl, NC-, HNR^{N}-C(=O)-, C₁₋₄-Alkyl-NR^{N}-C(=O)-, C₃₋₆-Cycloalkyl-NR^{N}-C(=O)-, Heterocyclyl-NR^{N}-C(=O)-, HOOC-, HR^{N}N-, C₁₋₄-Alkyl-R^{N}N-, C₁₋₄-Alkyl-C(=O)NR^{N}-, C₃₋₆-Cycloalkyl-C(=O)NR^{N}-, Heterocyclyl-C(=O)NR^{N}-, C₁₋₄-Alkyl-S(=O)₂NR^{N}-, HO-, C₁₋₆-Alkyl-O-, HOOC-C₁₋₂-Alkyl-O-, Heterocyclyl-C₁₋₂-alkyl-O-, Phenyl-C₁₋₂-alkyl-O-, C₃₋₆-Cycloalkyl-O-, Heterocyclyl-O-, Heteroaryl-O-, C₁₋₄-Alkyl-S(=O)₂-, C₃₋₆-Cycloalkyl-S(=O)₂-, Heterocyclyl-S(=O)₂-, HNR^{N}-S(=O)₂-, C₁₋₄-Alkyl-NR^{N}-S(=O)₂-, Heterocyclyl und Heteroaryl,
wobei jede Alkyl-, Cycloalkyl- und Heterocyclylgruppe oder -untergruppe innerhalb der Gruppen, die R bilden, gegebenenfalls mit 1 oder mehreren F-Atomen substituiert ist und gegebenenfalls mit 1 bis 2 Gruppen, unabhängig ausgewählt aus Cl, H₃C-, NC-, R^{N}HN-, HO-, H₃C-O- und H₃C-S(=O)₂-, substituiert ist;
wobei jede Heteroarylgruppe oder -untergruppe innerhalb der Gruppen, die R bilden, gegebenenfalls mit 1 bis 3 Substituenten, unabhängig ausgewählt aus F, Cl, H₃C-, F₃C-, NC-, (R^{N})₂N-, HO-, H₃C-O-, F₃C-O- und H₃C-S(=O)₂-, substituiert ist;
wobei jede Heterocyclylgruppe oder -untergruppe innerhalb der Gruppen, die R bilden, ausgewählt ist aus
einer Cyclobutylgruppe, in der 1 CH₂-Gruppe durch -NR^{N}- oder -O- ersetzt ist;
einer C₅₋₆-Cycloalkylgruppe, in der 1 CH₂-Gruppe durch -C(=O)-, -NR^{N}-, -O- oder -S(=O)₂- ersetzt ist und/oder 1 CH-Gruppe durch N ersetzt ist;
einer C₅₋₆-Cycloalkylgruppe, in der 1 CH₂-Gruppe durch -NR^{N}- oder -O-ersetzt ist, eine zweite CH₂-Gruppe durch -NR^{N}-, -C(=O)- oder -S(=O)₂-ersetzt ist und/oder 1 CH-Gruppe durch N ersetzt ist;
wobei jede Heteroarylgruppe oder -untergruppe innerhalb der Gruppen, die R bilden, ausgewählt ist aus
Tetrazolyl, einem 5-gliedrigem heteroaromatischen Ring, der 1, 2 oder 3 Heteroatome, unabhängig voneinander ausgewählt aus =N-, -NH-, O und S, enthält und einem 6-gliedrigem heteroaromatischen Ring, der 1 oder 2 =N-Atome enthält, wobei eine -HC=N- Einheit gegebenenfalls durch -NH-C(=O)-ersetzt ist;
und wobei in allen Heteroaryl- und Heterocyclylgruppen oder -untergruppen, die eine oder mehrere NH-Gruppen enthalten, diese Gruppe(n) durch NR^{N} ersetzt ist/sind;
oder ein Salz hiervon.

3. Verbindung gemäß Anspruch 1, in der
R ausgewählt ist aus der Gruppe, bestehend aus F, Cl, NC-, H₂NC(=O)-, H₃CHN-C(=O)-, (H₃C)₂N-C(=O)-, HOOC-, H₂N-, HO-;
C₁₋₃-Alkyl, gegebenenfalls substituiert mit 1 oder mehreren F oder gegebenenfalls monosubstituiert mit HO-;
Cyclopropyl, gegebenenfalls monosubstituiert mit NC-;
H₃C-O-, gegebenenfalls monosubstituiert mit C₁₋₄-Alkyl, HOOC-, Oxetanyl, Tetrahydrofuranyl, Tetrahydropyranyl, 1,1-Dioxotetrahydrothiopyranyl oder Phenyl, wobei besagte C₁₋₄-Alkylgruppe gegebenenfalls mit NC-, HO- oder H₃C-S(=O)₂- monosubstituiert ist, und wobei jede der besagten Oxetanyl-, Tetrahydrofuranyl-, Tetrahydropyranyl-, 1,1-Dioxotetrahydrothiopyranylgruppen gegebenenfalls mit H₃C- oder HO- monosubstituiert ist;
Cyclopropyl-O-, Tetrahydrofuranyl-O- und Tetrahydropyranyl-O-; und
einer Heteroarylgruppe, ausgewählt aus Pyrazolyl, [1,2,4]Oxadiazolyl, Tetrazolyl, Pyridyl, Pyridin-2-onyl, Pyrazinyl, Pyrimidinyl und Pyrimidin-4-onyl, wobei jeder dieser Heteroarylgruppen gegebenenfalls mit H₃C- monosubstituiert ist und wobei jede H-N-Gruppe in diesen Heteroarylgruppen gegebenenfalls durch H₃C-N oder (H₃C)₂C(OH)-H₂C-N ersetzt ist;
oder ein Salz hiervon.

4. Verbindung gemäß Anspruch 1, 2 oder 3, in der
R¹ H, F, Cl, H₃C-, H₃C-H₂C-, F₃C-, NC- oder H₃CO- bedeutet;
R² H oder F bedeutet;
m 2 bedeutet und
n 1 bedeutet;
oder ein Salz hiervon.

5. Verbindung gemäß Anspruch 1, 2, 3 oder 4, in der R¹ H₃C- bedeutet; oder ein Salz hiervon.

6. Verbindung gemäß Anspruch 1, in der
R ausgewählt ist aus einer Gruppe, bestehend aus:
F, Cl, NC-, H₂NC(=O)-, H₃CHN-C(=O)-, (H₃C)₂N-C(=O)-, HOOC-, H₂N-, HO-;
C₁₋₃-Alkyl, gegebenenfalls substituiert mit 1 oder mehreren F oder gegebenenfalls monosubstituiert mit HO-;
Cyclopropyl, gegebenenfalls monosubstituiert mit NC-;
H₃C-O-, gegebenenfalls monosubstituiert mit C₁₋₄-Alkyl, HOOC-, Oxetanyl, Tetrahydrofuranyl, Tetrahydropyranyl, 1,1-Dioxotetrahydrothiopyranyl oder Phenyl,
wobei die C₁₋₄-Alkylgruppe, welche gegebenenfalls mit der H₃C-alkyl-O-verknüpft ist, gegebenenfalls monosubstituiert ist mit NC-, HO- oder H₃C-S(=O)₂-, und
wobei besagte Oxetanyl-, Tetrahydrofuranyl-, Tetrahydropyranyl- und 1,1-Dioxotetrahydrothiopyranylgruppen gegebenenfalls mit H₃C- oder HO- monosubstituiert sind;
Cyclopropyl-O-, Tetrahydrofuranyl-O- und Tetrahydropyranyl-O-;
einer Heteroarylgruppe, ausgewählt aus Pyrazolyl, [1,2,4]Oxadiazolyl, Tetrazolyl, Pyridyl, Pyridin-2-onyl, Pyrazinyl, Pyrimidinyl und Pyrimidin-4-onyl,
wobei jede der besagten Heteroarylgruppen gegebenenfalls mit H₃C-monosubstituiert ist, und
wobei jede H-N-Gruppe in besagten Heteroarylgruppen gegebenenfalls durch H₃C-N oder (H₃C)₂C(OH)-H₂C-N ersetzt ist;
R¹ H, F, Cl, H₃C-, H₃C-H₂C -, F₃C-, NC- oder H₃C-O- bedeutet;
m 2 bedeutet;
R² H oder F bedeutet; und
n 1 bedeutet;
oder ein Salz hiervon.

7. Verbindung gemäß Anspruch 1, in der
R ausgewählt ist aus einer Gruppe, bestehend aus
F, Cl, H₃C-, H₃C-H₂C-, (H₃C)₂CH-, 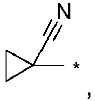 F₃C-, HOCH₂-, NC-, H₂N-C(=O)-, H₃C-NH-C(=O)-, (H₃C)₂N-C(=O)-, HOOC-, H₂N-, HO-, H₃C-O-, Cyclopropyl-O-, 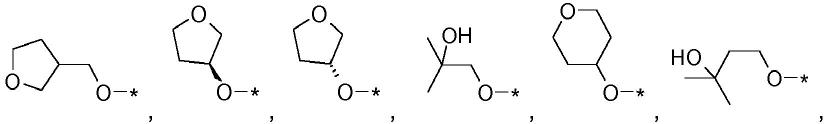 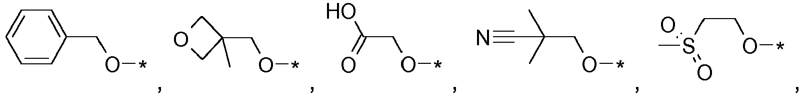 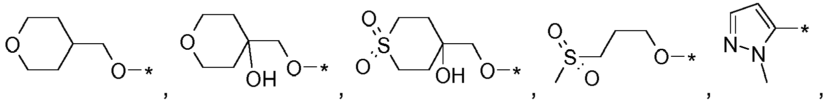 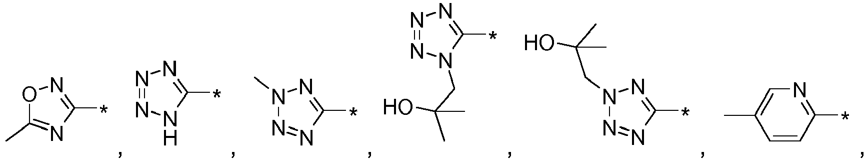 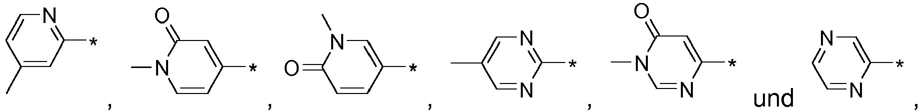 wobei das Sternchen, -*, den Anknüpfungspunkt kennzeichnet;
R¹ H₃C- bedeutet;
m 2 bedeutet;
R² F bedeutet; und
n 1 bedeutet;
oder ein Salz hiervon.

8. Verbindung gemäß irgendeinem der Ansprüche 1 bis 7, mit der in den Formeln I.1, I.2 oder I.3 gezeigten Struktur und Stereochemie 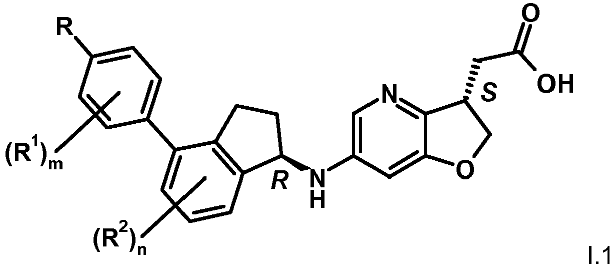 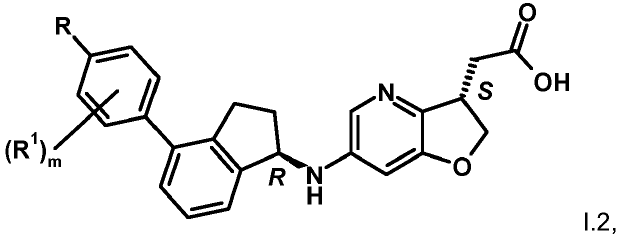 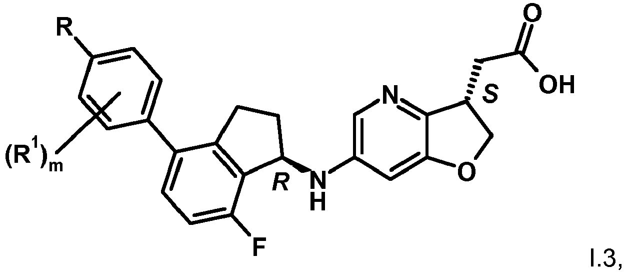 oder ein Salz hiervon.

9. Verbindung mit einer der folgenden Strukturen 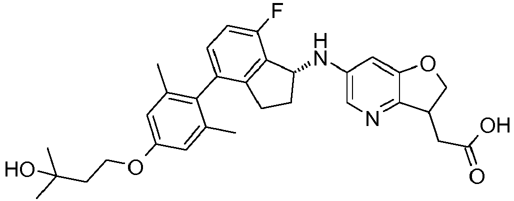 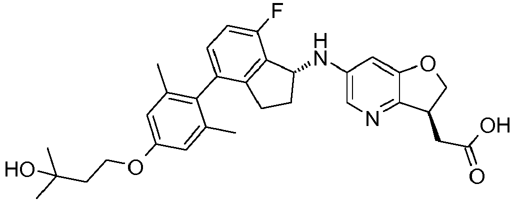 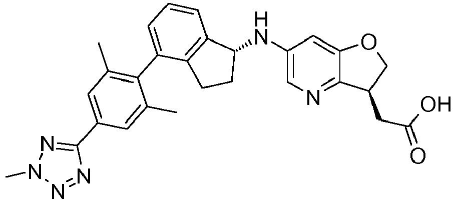 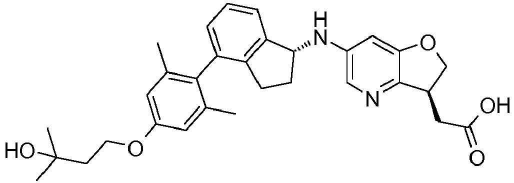 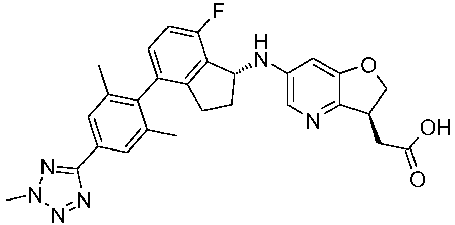 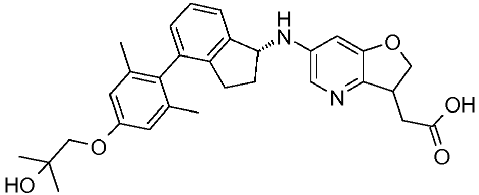 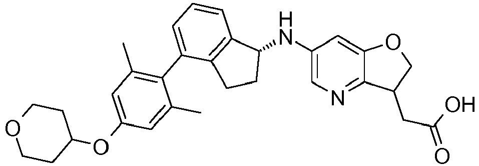 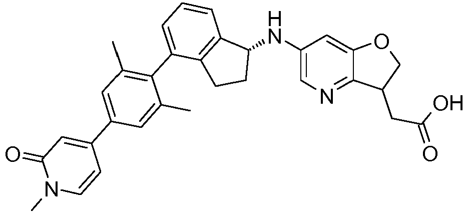 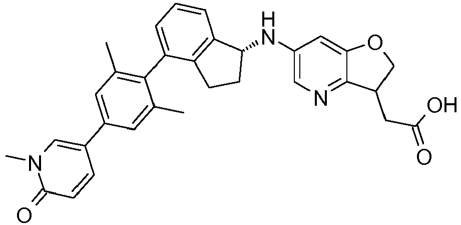 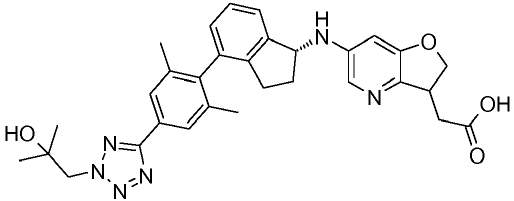 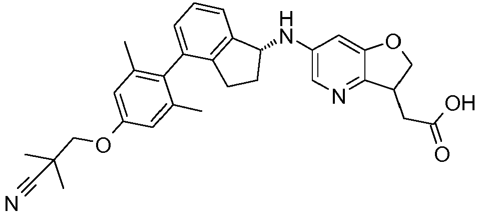 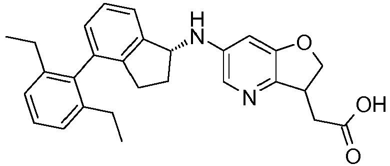 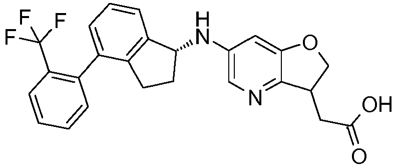 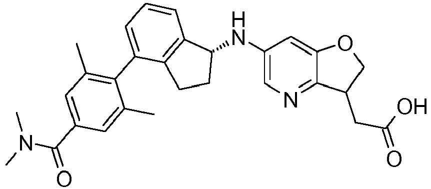 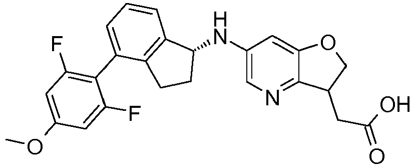 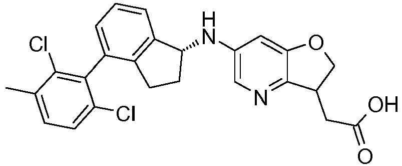 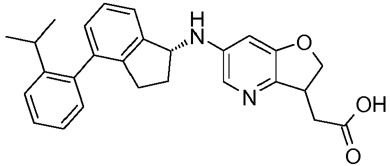 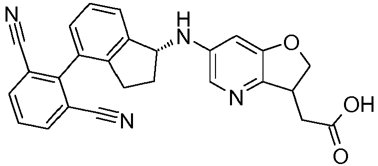 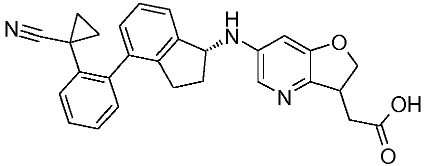 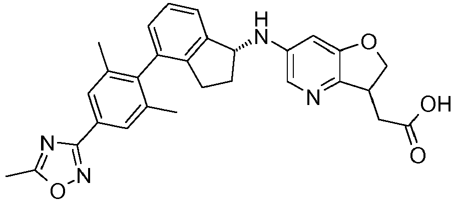 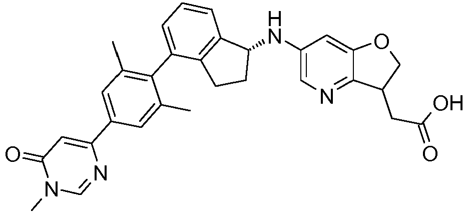 oder ein Salz hiervon.

10. Pharmazeutisch akzeptables bzw. annehmbares Salz einer Verbindung gemäß einem oder mehrerer der Ansprüche 1 bis 9.

11. Pharmazeutische Zusammensetzung, umfassend eine oder mehrere Verbindungen gemäß einem oder mehrerer der Ansprüche 1 bis 9 oder eines oder mehrere ihrer pharmazeutisch akzeptablen bzw. annehmbaren Salze, gegebenenfalls zusammen mit einem oder mehreren inerten Trägerstoffen und/oder Verdünnungsmitteln.

12. Verbindung gemäß einem oder mehrerer der Ansprüche 1 bis 9 oder eines ihrer pharmazeutisch akzeptablen bzw. annehmbaren Salze zur Verwendung als Arzneimittel.

13. Verbindung gemäß einem oder mehrerer der Ansprüche 1 bis 9 oder eines ihrer pharmazeutisch akzeptablen bzw. annehmbaren Salze zur Verwendung in der Prophylaxe und/oder Therapie von metabolischen Erkrankungen und Zuständen, die mit der Erkrankung in Verbindung stehen.

14. Verbindung zur Verwendung gemäß Anspruch 13, wobei die metabolische Erkrankung Diabetes ist und die Zustände, die mit der Erkrankung in Verbindung stehen, ausgewählt sind aus der Gruppe, bestehend aus Insulinresistenz, Adipositas, kardiovaskulären Erkrankungen und Dyslipidämie.

15. Verbindung zur Verwendung gemäß Anspruch 13, wobei die metabolische Erkrankung Typ 2 Diabetes mellitus ist.

16. Pharmazeutische Zusammensetzung, umfassend eine oder mehrere Verbindungen gemäß einem oder mehrerer der Ansprüche 1 bis 9 oder eines oder mehrere ihrer pharmazeutisch akzeptablen bzw. annehmbaren Salze und ein oder mehrere zusätzlichen therapeutische Mittel, gegebenenfalls zusammen mit einem oder mehreren inerten Trägerstoffen und/oder Verdünnungsmitteln.

17. Pharmazeutische Zusammensetzung gemäß Anspruch 16, wobei die zusätzlichen therapeutischen Mittel ausgewählt sind aus der Gruppe, bestehend aus antidiabetischen Mittel, Mitteln zur Behandlung von Übergewicht und/oder Adipositas und Mitteln zur Behandlung von hohem Blutdruck, Herzinsuffizienz und/oder Atherosklerose.

## Revendications

1. Composé de formule I 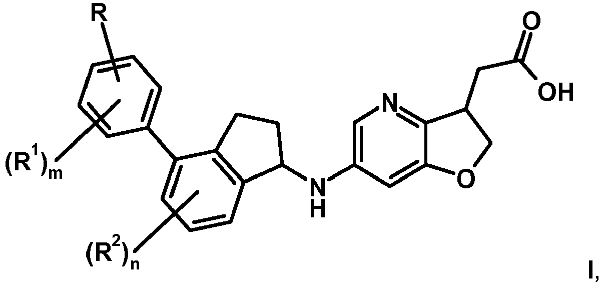 dans laquelle
R est sélectionné dans un groupe consistant en
H, F, Cl, Br, I, C₁₋₆-alkyle, C₂₋₆-alkényle, C₂₋₆-alkynyle, C₃₋₆-cycloalkyle, NC-, HNR^{N}-C(=O)-, C₁₋₄-alkyl-NR^{N}-C(=O)-, C₃₋₆-cycloalkyl-NR^{N}-C(=O)-, hétérocyclyl-NR^{N}-C(=O)-, hétéroaryl-NR^{N}-C(=O)-, HOOC-, C₁₋₄-alkyl-O-C(=O)-, O₂N-, HR^{N}N-, C₁₋₄-alkyl-R^{N}N-, C₁₋₄-alkyl-C(=O)NR^{N}-, C₃₋₆-cycloalkyl-C(=O)NR^{N}-, hétérocyclyl-C(=O)NR^{N}-, hétéroaryl-C(=O)NR^{N}-, C₁₋₄-alkyl-S(=O)₂NR^{N}-, C₃₋₆-cycloalkyl-S(=O)₂NR^{N}-, hétérocyclyl-S(=O)₂NR^{N}-, hétéroaryl-S(=O)₂NR^{N}-, HO-, C₁₋₆-alkyl-O-, HOOC-C₁₋₃-alkyl-O-, hétérocyclyl-C₁₋₃-alkyl-O-, phényl-C₁₋₃-alkyl-O-, C₃₋₆-cycloalkyl-O-, hétérocyclyl-O-, hétéroaryl-O-, C₁₋₄-alkyl-S-, C₃₋₆-cycloalkyl-S-, hétérocyclyl-S-, C₁₋₄-alkyl-S(=O)-, C₃₋₆-cycloalkyl-S(=O)-, hétérocyclyl-S(=O)-, C₁₋₄-alkyl-S(=O)₂-, C₃₋₆-cycloalkyl-S(=O)₂-, hétérocyclyl-S(=O)₂-, phényl-S(=O)₂-, hétéroaryl-S(=O)₂-, HNR^{N}-S(=O)₂-, C₁₋₄-alkyl-NR^{N}-S(=O)₂-, hétérocyclyle, phényle et hétéroaryle,
dans lequel tout groupe ou sous-groupe alkyle, cycloalkyle et hétérocyclyle au sein des groupes formant R est éventuellement substitué avec 1 ou plusieurs atomes de fluor F et est éventuellement substitué avec 1 à 3 groupes indépendamment sélectionnés parmi Cl, C₁₋₃-alkyle, NC-, (R^{N})₂N-, HO-, C₁₋₃-alkyl-O- et C₁₋₃-alkyl-S(=O)₂-; et
dans lequel tout groupe ou sous-groupe phényle et hétéroaryle au sein des groupes formant R est éventuellement substitué avec 1 à 5 substituants indépendamment sélectionnés parmi F, Cl, C₁₋₃-alkyle, HF₂C-, F₃C-, NC-, (R^{N})₂N-, HO-, C₁₋₃-alkyl-O-, F₃C-O- et C₁₋₃-alkyl-S(=O)₂-;
dans lequel tout groupe ou sous-groupe hétérocyclyle au sein des groupes formant R est sélectionné parmi
un groupe cyclobutyle, dans lequel 1 groupe CH₂ est remplacé par -NR^{N}- ou -O-;
un groupe C₅₋₆-cycloalkyle, dans lequel un 1 groupe CH₂ est remplacé par -C(=O)-, -NR^{N}-, -O- ou -S(=O)₂- et/ou 1 groupe CH est remplacé par N;
un groupe C₅₋₆-cycloalkyle, dans lequel 1 groupe CH₂ est remplacé par -NR^{N}- ou -O-, un deuxième groupe CH₂ est remplacé par -NR^{N}-, -C(=O)- ou -S(=O)₂- et/ou 1 groupe CH est remplacé par N; et
un groupe C₅₋₆-cycloalkyle, dans lequel 2 groupes CH₂ sont remplacés par -NR^{N}- ou 1 groupe CH₂ est remplacé par -NR^{N}- et l'autre par -O- et un troisième groupe CH₂ est remplacé par -C(=O)- ou -S(=O)₂- et/ou 1 groupe CH est remplacé par N;
dans lequel tout groupe ou sous-groupe hétéroaryle au sein des groupes formant R est sélectionné parmi
un groupe tétrazolyle et un cycle hétéroaromatique à 5 ou 6 chaînons qui contient 1, 2 ou 3 hétéroatomes indépendamment l'un de l'autre choisis parmi =N-, -NR^{N}-, -O- et -S-, dans lequel dans les groupes hétéroaromatiques qui contiennent une unité -HC=N-, ce groupe-ci est éventuellement remplacé par -NR^{N}-C(=O)-;
dans lequel dans les cycles hétéroaryle et hétérocyclyle avec un ou plusieurs groupes NH, chacun desdits groupes NH est remplacés par NR^{N};
R¹ est sélectionné dans un groupe consistant en H, F, Cl, C₁₋₄-alkyle, C₃₋₆-cycloalkyle-, HO-C₁₋₄-alkyle, C₁₋₄-alkyl-O-C₁₋₄-alkyle, NC-, HO-, C₁₋₄-alkyl-O-, C₃₋₆-cycloalkyl-O-, C₁₋₄-alkyl-S-, C₁₋₄-alkyl-S(O)- et C₁₋₄-alkyl-S(O)₂-,
dans lequel tout groupe ou sous-groupe alkyle et cycloalkyle au sein des groupes formant R¹ est éventuellement substitué avec 1 ou plusieurs atomes de fluor F, et dans lequel, si m est 2, 3 ou 4, plusieurs R¹ peuvent être identiques ou différents;
m est un nombre entier sélectionné parmi 1, 2, 3 et 4;
R² est H, F, Cl, C₁₋₄-alkyle, NC- ou C₁₋₄-alkyloxy,
dans lequel tout groupe ou sous-groupe alkyle au sein des groupes formant R² est éventuellement substitué avec 1 ou plusieurs atomes de fluor F, et dans lequel, si n est 2 ou 3, plusieurs R² peuvent être identiques ou différents;
n est un nombre entier sélectionné parmi 1, 2 et 3;
R^{N} sont indépendamment l'un de l'autre sélectionnés dans un groupe consistant en H, C₁₋₄-alkyle, HO-(C₁₋₄-alkyl)-H₂C-, C₁₋₃-alkyl-O-C₁₋₄-alkyle-, C₁₋₄-alkyl-C(=O)-, C₁₋₄-alkyl-NH-C(=O)-, C₁₋₄-alkyl-N(C₁₋₄-alkyl)-C(=O)-, C₁₋₄-alkyl-O-C(=O)- et C₁₋₄-alkyl-S(=O)₂-,
dans lequel tout groupe ou sous-groupe alkyle au sein des groupes formant R^{N} est éventuellement substitué avec 1 ou plusieurs atomes de fluor F;
dans lequel dans tous les définitions mentionnées ci-dessus et sauf mention particulière, tout groupe ou sous-groupe alkyle peut être linéaire ou ramnifié,
ou un sel de celui-ci.

2. Composé selon la revendication 1, dans laquelle
R est sélectionné dans le groupe consistant en H, F, Cl, C₁₋₆-alkyle, C₃₋₆-cycloalkyle, NC-, HNR^{N}-C(=O)-, C₁₋₄-alkyl-NR^{N}-C(=O)-, C₃₋₆-cycloalkyl-NR^{N}-C(=O)-, hétérocyclyl-NR^{N}-C(=O)-, HOOC-, HR^{N}N-, C₁₋₄-alkyl-R^{N}N-, C₁₋₄-alkyl-C(=O)NR^{N}-, C₃₋₆-cycloalkyl-C(=O)NR^{N}-, hétérocyclyl-C(=O)NR^{N}-, C₁₋₄-alkyl-S(=O)₂NR^{N}-, HO-, C₁₋₆-alkyl-O-, HOOC-C₁₋₂-alkyl-O-, hétérocyclyl-C₁₋₂-alkyl-O-, phényl-C₁₋₂-alkyl-O-, C₃₋₆-cycloalkyl-O-, hétérocyclyl-O-, hétéroaryl-O-, C₁₋₄-alkyl-S(=O)2-, C₃₋₆-cycloalkyl-S(=O)₂-, hétérocyclyl-S(=O)₂-, HNR^{N}-S(=O)₂-, C₁₋₄-alkyl-NR^{N}-S(=O)₂-, hétérocyclyle et hétéroaryle,
dans lequel tout groupe ou sous-groupe alkyle, cycloalkyle et hétérocyclyle au sein des groupes formant R est éventuellement substitué avec 1 ou plusieurs atomes de fluor F et est éventuellement substitué avec 1 à 2 groupes, indépendamment sélectionnés parmi Cl, H₃C-, NC-, R^{N}HN-, HO-, H₃C-O- et H₃C-S(=O)₂-;
dans lequel tout groupe ou sous-groupe hétéroaryle au sein des groupes formant R est éventuellement substitué avec 1 à 3 substituants indépendamment sélectionnés parmi F, Cl, H₃C-, F₃C-, NC-, (R^{N})₂N-, HO-, H₃C-O-, F₃C-O- et H₃C-S(=O)₂-;
dans lequel tout groupe ou sous-groupe hétérocyclyle au sein des groupes formant R est sélectionné parmi
un groupe cyclobutyle, dans lequel 1 groupe CH₂ est remplacé par -NR^{N}- ou -O-;
un groupe C₅₋₆-cycloalkyle, dans lequel 1 groupe CH₂ est remplacé par -C(=O)-, -NR^{N}-, -O- ou -S(=O)₂- et/ou 1 groupe CH est remplacé par N;
un groupe C₅₋₆-cycloalkyl, dans lequel 1 groupe CH₂ est remplacé par -NR^{N}-ou -O-, un deuxième groupe CH₂ est remplacé par -NR^{N}-, -C(=O)- ou -S(=O)₂- et/ou 1 groupe CH est remplacé par N;
dans lequel tout groupe ou sous-groupe hétéroaryle au sein des groupes formant R est sélectionné parmi
tétrazolyle, un cycle hétéroaromatique à 5 chaînons qui contient 1, 2 ou 3 hétéroatomes, indépendamment l'un de l'autre choisis parmi =N-, -NH-, O et S, et un cycle hétéroaromatique à 6 chaînons qui contient 1 ou 2 atomes d'azote =N-, dans leque une unité -HC=N- est éventuellement remplacé par -NH-C(=O)-;
et dans lequel dans tous les groupes ou sous-groupes hétéroaryle et hétérocyclyle contenant un ou plusieurs groupes NH, le(s)dit(s) groupe(s) NH est/sont remplacé(s) par NR^{N};
ou un sel de celui-ci.

3. Composé selon la revendication 1, dans laquelle
R est sélectionné dans le groupe consistant en F, Cl, NC-, H₂NC(=O)-, H₃CHN-C(=O)-, (H₃C)₂N-C(=O)-, HOOC-, H₂N-, HO-;
C₁₋₃-alkyle éventuellement substitué avec 1 ou plusieurs atomes de fluor F ou éventuellement monosubstitué avec HO-;
cyclopropyle éventuellement monosubstitué avec NC-;
H₃C-O- éventuellement monosubstitué avec C₁₋₄-alkyle, HOOC-, oxétanyle, tétrahydrofuranyle, tétrahydropyranyle, 1,1-dioxotétrahydrothiopyranyle ou phényle, dans lequel ledit groupe C₁₋₄-alkyle est éventuellement monosubstitué avec NC-, HO- ou H₃C-S(=O)₂-, et dans lequel chacun desdits groupes oxétanyle, tétrahydrofuranyle, tétrahydropyranyle, 1,1-dioxotétrahydrothiopyranyle est éventuellement monosubstitué avec H₃C- ou HO-;
cyclopropyl-O-, tétrahydrofuranyl-O- et tétrahydropyranyl-O-; et
un groupe hétéroaryle sélectionné parmi pyrazolyle, [1,2,4]oxadiazolyle, tétrazolyle, pyridyle, pyridin-2-onyle, pyrazinyle, pyrimidinyle et pyrimidin-4-onyle, dans lequel chacun desdits groupes hétéroaryle est éventuellement monosubstitué avec H₃C- et dans lequel tout groupe H-N au sein desdits groupes hétéroaryle est éventuellement remplacé par H₃C-N ou (H₃C)₂C(OH)-H₂C-N;
ou un sel de celui-ci.

4. Composé selon la revendication 1, 2 ou 3, dans laquelle
R¹ dénote H, F, Cl, H₃C-, H₃C-H₂C-, F₃C-, NC- ou H₃CO-;
R² dénote H ou F;
m vaut 2 et
n vaut 1;
ou un sel de celui-ci.

5. Composé selon la revendication 1, 2, 3 ou 4, dans laquelle R¹ dénote H₃C-; ou un sel de celui-ci.

6. Composé selon la revendication 1, dans laquelle
R est sélectionné dans un groupe consistant en:
F, Cl, NC-, H₂NC(=O)-, H₃CHN-C(=O)-, (H₃C)₂N-C(=O)-, HOOC-, H₂N-, HO-;
C₁₋₃-alkyle éventuellement substitué avec 1 ou plusieurs atomes des fluor F ou éventuellement monosubstitué avec HO-;
cyclopropyle éventuellement monosubstitué avec NC-;
H₃C-O- éventuellement monosubstitué avec C₁₋₄-alkyle, HOOC-, oxétanyle, tétrahydrofuranyle, tétrahydropyranyle, 1,1-dioxotétrahydrothiopyranyle ou phényle,
dans lequel le groupe C₁₋₄-alkyle éventuellement attaché au groupe H₃C-alkyl-O- est éventuellement monosubstitué avec NC-, HO- ou H₃C-S(=O)₂-, et
dans lequel lesdits groupes oxétanyle, tétrahydrofuranyle, tétrahydropyranyle et 1,1-dioxotétrahydrothiopyranyle sont éventuellement monosubstitué avec H₃C- ou HO-;
cyclopropyl-O-, tétrahydrofuranyl-O- et tétrahydropyranyl-O-;
un groupe hétéroaryle sélectionné parmi pyrazolyle, [1,2,4]oxadiazolyle, tétrazolyle, pyridyle, pyridin-2-onyle, pyrazinyle, pyrimidinyle et pyrimidin-4-onyle,
dans lequel chacun desdits groupes hétéroaryle est éventuellement monosubstitué avec H₃C-, et
dans lequel tout groupe H-N dans lequels desdits groupes hétéroaryle est éventuellement remplacé avec H₃C-N ou (H₃C)₂C(OH)-H₂C-N;
R¹ dénote H, F, Cl, H₃C-, H₃C-H₂C-, F₃C-, NC- ou H₃C-O-;
m vaut 2;
R² dénote H ou F; et
n vaut 1;
ou un sel de celui-ci.

7. Composé selon la revendication 1, dans laquelle
R est sélectionné dans un groupe consistant en
F, Cl, H₃C-, H₃C-H₂C-, (H₃C)₂CH-, 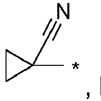 F₃C-, HOCH₂-, NC-, H₂N-C(=O)-, H₃C-NH-C(=O)-, (H₃C)₂N-C(=O)-, HOOC-, H₂N-, HO-, H₃C-O-, cyclopropyl-O-, 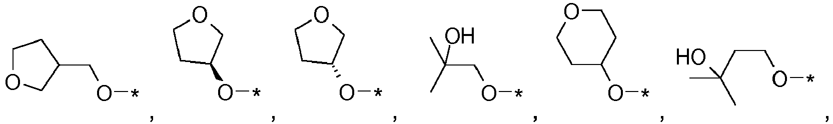 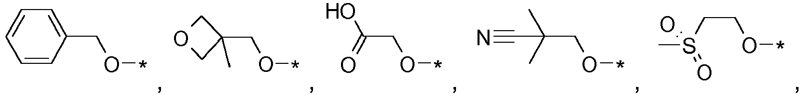 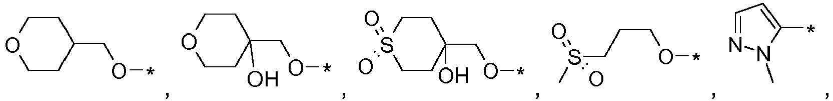 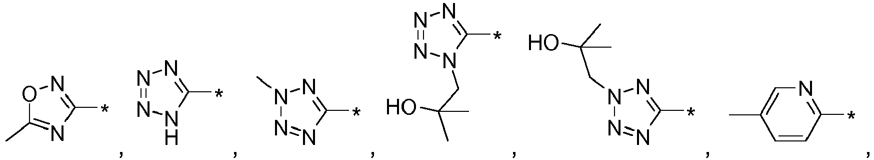 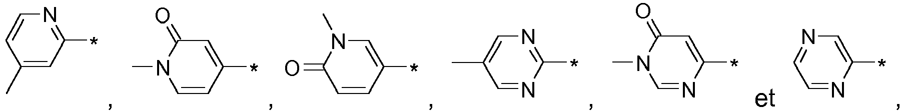 dans lequel l'astérisque, -*, indique le site/point d'attachement;
R¹ dénote H₃C-;
m vaut 2;
R² dénote F; et
n vaut 1;
ou un sel de celui-ci.

8. Composé selon l'une quelconque des revendications 1 à 7, avec la structure et la stéréochemie indiquées dans les formule I.1, I.2 ou I.3 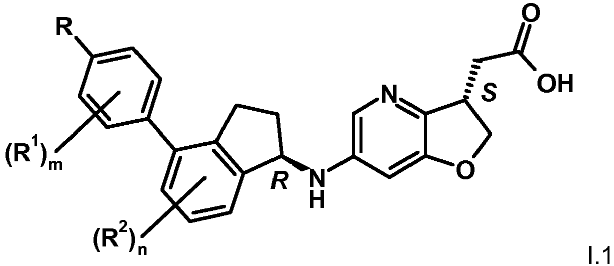 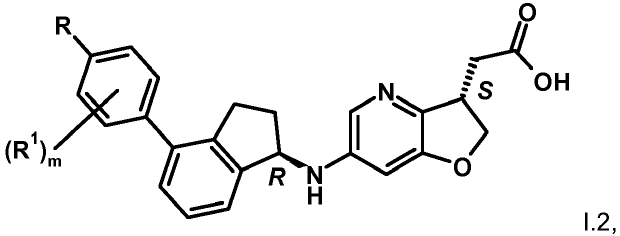 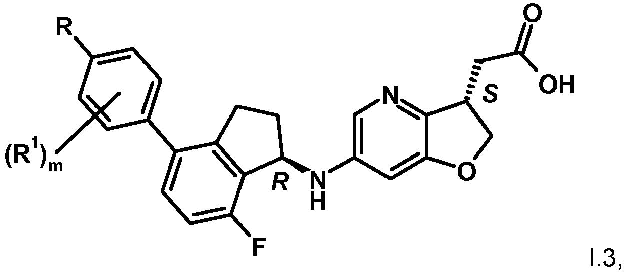 ou un sel de celui-ci.

9. Composé avec l'une des structures suivantes 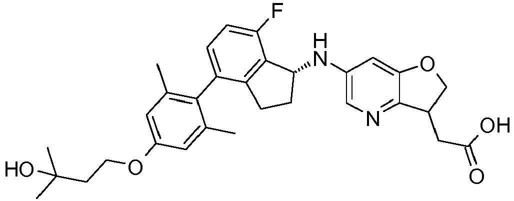 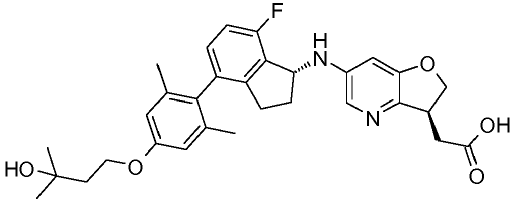 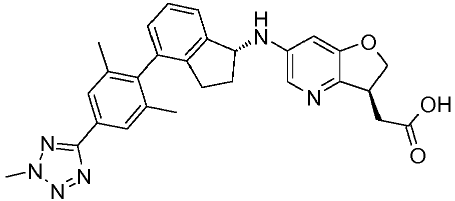 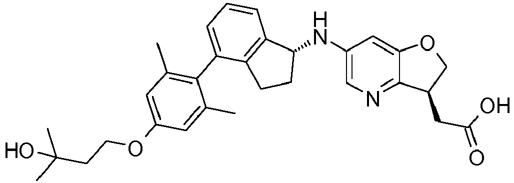 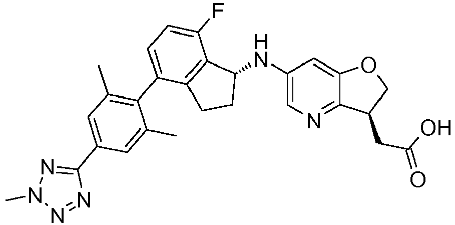 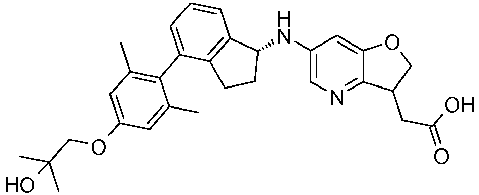 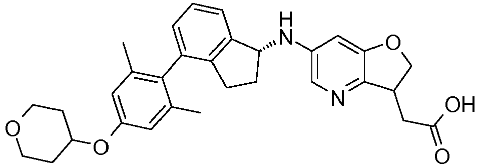 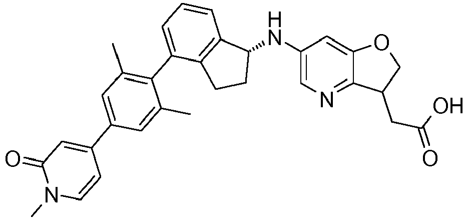 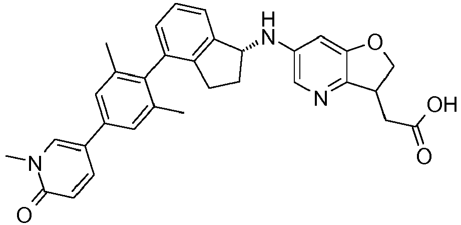 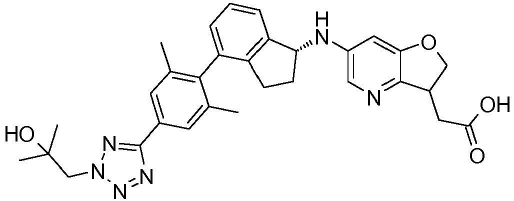 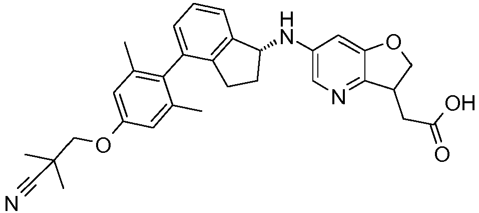 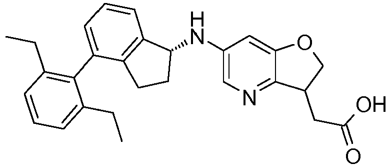 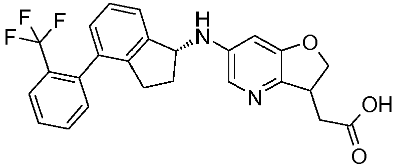 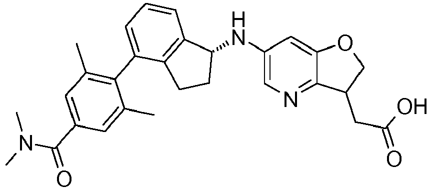 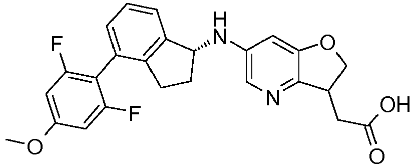 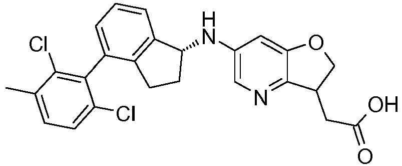 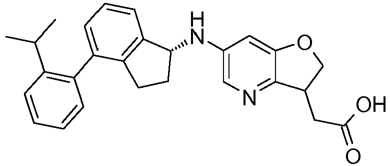 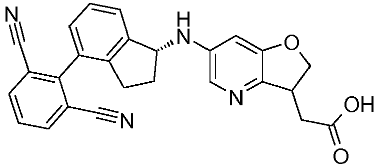 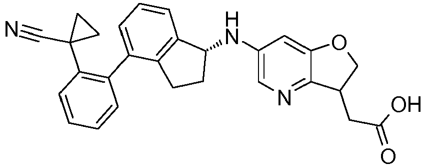 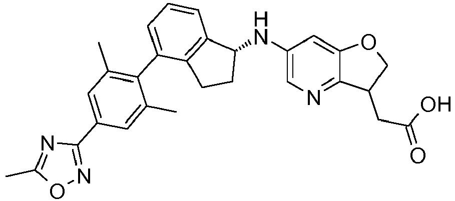 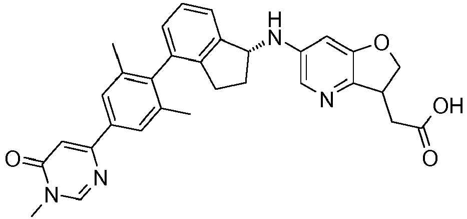 ou un sel de celui-ci.

10. Sel pharmaceutiquement acceptable d'un composé selon l'une ou plusieurs des revendications 1 à 9.

11. Composition pharmaceutique comprenant un ou plusieurs composés selon l'une ou plusieurs des revendications 1 à 9 ou un ou plusieurs de leurs sels pharmaceutiquement acceptables, éventuellement ensemble avec un ou plusieurs excipients et/ou diluants inertes.

12. Composé selon l'une ou plusieurs des revendications 1 à 9 ou un sel pharmaceutiquement acceptable de celui-ci pour son utilisation comme médicament.

13. Composé selon l'une ou plusieurs des revendications 1 à 9 ou un sel pharmaceutiquement acceptable de celui-ci pour son utilisation dans la prophylaxie et/ou la therapie des maladies métaboliques et des états physiques associés à la maladie.

14. Composé pour son utilisation selon la revendication 13, dans laquelle la maladie métabolique est le diabète, et les états physiques associés à la maladie sont sélectionnés dans le groupe consistant en la résistance d'insuline, l'obésité, la maladie cardiovasculaire et la dyslipidémie.

15. Composé pour son utilisation selon la revendication 13, dans laquelle la maladie métabolique est le diabète sucré de type 2.

16. Composition pharmaceutique comprenant un ou plusieurs composés selon l'une ou plusieurs des revendications 1 à 9 ou un ou plusieurs de leurs sels pharmaceutiquement acceptables et un ou plusieurs agents thérapeutiques supplémentaires, éventuellement ensemble avec un ou plusieurs excipients et/ou diluants inertes.

17. Composition pharmaceutique selon la revendication 16, dans laquelle les agents thérapeutiques supplémentaires sont sélectionnés dans le groupe consistant en les agents antidiabétiques, les agents pour le traitement du surpoids et/ou de l'obésité et les agents pour le traitement de l'hypertension, de l'insuffisance cardiaque et/ou de l'athérosclérose.
